# EUROPEAN PATENT SPECIFICATION

(11) **EP 1 730 311 B1**
(45) Date of publication and mention of the grant of the patent: **15.09.2010**
(21) Application number: 05729402.7
(22) Date of filing: 31.03.2005
(51) Int. Cl.: C12Q 1/68

(54) **A METHOD OF GENOTYPING BLOOD CELL ANTIGENS AND KIT SUITABLE FOR GENOTYPING BLOOD CELL ANTIGENS**
VERFAHREN ZUR GENOTYPISIERUNG VON ANTIGENEN VON BLUTZELLEN UND EIN KIT ZUR GENOTYPISIERUNG
UNE MÉTHODE DE GENOTYPING DES ANTIGÈNES DE CELLULES DE SANG ET UN KIT APPROPRIÉ AUX GENOTYPING DES ANTIGÈNES DE CELLULES DE SANG

(30) Priority: 01.04.2004 EP 04076046
(43) Date of publication of application: 13.12.2006
(73) Proprietor: Stichting Sanquin Bloedvoorziening, 1066 CX Amsterdam (NL)
(72) Inventor: BEIBOER, Sigrid, Herma, Wilma, NL-3192 NL Hoogvliet (NL); WIERINGA-JELSMA, Hendrika, NL-1318 GA Almere (NL); DEN DUNNEN, Johannes, Theodorus, NL-3069 LA Rotterdam (NL); DE HAAS, Maschenka, NL-1541 NP Koog aan de Zaan (NL)
(74) Representative: van Loon, C.J.J.
(86) International application number: PCT/NL2005/000236
(87) International publication number: WO 2005/095650

(56) References cited:
- EP-A- 1 180 549
- EP-A- 1 247 815
- US-A1- 2003 124 594
- ANTOINE C ET AL: "Long-term survival and transplantation of haemopoietic stem cells for immunodeficiencies: report of the European experience 1968-99" LANCET THE, LANCET LIMITED. LONDON, GB, vol. 361, no. 9357, 15 February 2003 (2003-02-15), pages 553-560, XP004778553 ISSN: 0140-6736

## Description

### FIELD OF THE INVENTION

This invention is in the field of genotyping of blood cell antigens, more particularly antigens on red blood cells (blood group antigens), blood platelets (platelet antigens) and leukocytes (leukocyte antigens).

The present invention provides a method of genotyping blood cell antigens and a kit suitable for genotyping blood cell antigens, and provides sets of primers and probes useful for genotyping blood cell antigens.

### BACKGROUND OF THE INVENTION

When blood, or a blood fraction, such as red blood cells (erythrocytes or red cells), platelets (thrombocytes) and white blood cells (leukocytes), derived from a donor are administered to another person (or more generally to another mammalian individual), serious adverse reactions may occur when the donor blood or blood fraction does not match properly with the blood of the recipient. Well known are transfusion reactions (agglutination) occurring when for example blood from a donor of blood group A is given to a person of blood group B (blood group antigens A and B belong to the AB0 system). When blood of a rhesus D (RhD) positive donor is given to a RhD negative patient there is a high chance that alloantibody formation occurs. RhD antibodies will lead to rapid destruction of RhD-positive red cells and to transfusion reactions. Furthermore, when a woman with red cell or platelet antibodies becomes pregnant, those antibodies can cross the placenta and can destruct the red cells or the platelets of the unborn child. This can lead to severe hemolysis resulting in anaemia, jaundice (after birth) and if not treated it can be fatal or lead to cerebral damage. Thus, to avoid transfusion reactions, the current blood transfusion policy is to transfuse only AB0 and RhD matched red cells. To avoid alloantibody formation with possible complications during pregnancy for women in childbearing age only AB0, RhD and K1 matched red cells are transfused. Possibly, in the future, also Rhc and RhE matched red cells will be given to women in childbearing age.

Various other blood group antigens (red cell antigens) exist, however, and these may also cause serious problems when non-matching donor blood is given to a recipient with alloantibodies. Typing of platelet-specific antigens is important for the diagnosis and therapy of the patients since different alloimmune thrombocytopenic syndromes can occur. If a woman has developed antiplatelet antigen antibodies (in most cases Human Platelet Antigen (HPA) type 1a antibodies) and mostly developed during a pregnancy, these antibodies can lead to fetal platelet destruction with an increased bleeding tendency in the unborn. In a number of cases this will lead to intracranial bleeding. To prevent bleeding, HPA-1a-negative platelets need to be transfused.

Platelets are usually transfused without previous typing of platelet antigens [human platelet antigens (HPA) in humans] of the donor and the recipient. Transfusions of non-matching blood or blood fractions may cause the generation of alloantibodies, especially in patients who need frequent or recurrent red cell or platelet (or leukocyte) transfusions. If multiple alloantibodies have formed, or if the alloantibodies are directed against high-frequency antigens, it can be a problem to find compatible red cells or platelets. According to a published study (Seltsam et al., 2003), transfusion support was unsatisfactory in about one-third of the hospitalized patients with antibodies to high-frequency antigens.

The classical method of testing for blood group antigens and antibodies is phenotyping by the hemagglutination test. The technique of this serological test is simple and inexpensive, but its costs and difficulties increase when multiple assays need to be done for complete typing and it requires availability of a large number of specific antisera. In The Netherlands alone, the total number of blood donors is in the order of magnitude of 500,000 people and it is estimated that the number of donors increases each year with about 60,000 new donors. For red cells, there are at least 29 blood group antigen systems (each with a number of different alleles). Furthermore, it will be relevant to type for high-frequency antigens and low-frequency antigens. The number of clinically relevant blood cell antigen systems is about 60. Complete phenotyping of all blood donors is therefore expensive, laborious, time consuming and not feasible due to lack of sufficient typing reagents.

The molecular basis for most blood cell antigen systems is known. Most blood group antigens, platelet antigens and neutrophil antigens are bi-allelic and are the result of a single nucleotide polymorphism (SNP). These SNPs may be used for genotyping. Innumerable DNA-based assays have been described in the scientific literature for the genotyping of blood groups and platelet antigens. These include PCR-RFLP, allele-specific PCR, sequence specific PCR as single or multiplex assays, real-time quantitative PCR, a single-nucleotide dye terminator extension method and high-throughput bead technology (reviewed by Reid, 2003). Semiautomated methods using a mass spectrophotometer or pyrosequencer may also be used for genotyping.

For example, Randen et al. (2003) recently disclosed a genotyping of the human platelet antigens 1, 2, 3, 4, 5 and Gov (recently called HPA-15, Metcalfe et al., 2003) by melting curve analysis using LightCycler technology. The biallelic systems HPA-1 through 5 and Gov are the ones most frequently involved in disease (Berry et al., 2000), making them important targets for genotyping. The LightCycler technology involves an amplification of relevant fragments of donor DNA by PCR using a specific primer pair for each of the above mentioned platelet antigens. By using fluorescent hybridization probes and melting curve analysis it was possible to achieve a simultaneous detection of both alleles of a platelet antigen in the same capillary, without a need of the laborious and time consuming gel electrophoresis of earlier genotyping methodology.

However, as with all other described blood cell antigen genotyping methods, also the LightCycler technology is not capable of performing the enormous task of a complete genotyping of blood donors, which would require methodology which is suitable for high-throughput screening. Typing each year 60,000 donors after two different donations for 60 blood group and platelet antigens would involve more than 7 million typing tests per year or about 140,000 typing tests per week. A suitable high-throughput method, which is rapid and reliable, is highly desirable for this task.

### Multiplex Polymerase Chain Reaction

The technique of Polymerase Chain Reaction (PCR) has developed much since it was introduced in the 1980's. Chamberlain et al. (1988) taught multiplex PCR as a general technique for the amplification of multiple loci in (genomic) DNA. Herein, instead of one primer pair for the amplification of one locus, more primer pairs are added in one reaction mixture. However, the development of such multiplex PCRs is limited by the complexity of the amplification reaction. Reaction components and cycling conditions must be adjusted for each extra pair of primers. Shuber et al. (1995) introduced 'chimeric' sequence-specific primers to circumvent this problem. These primers are complementary to the template DNA and contain an unrelated 20-nucleotide tag at the 5' end (universal sequence). Although the primers were designed in such a way that their predicted melting temperatures are similar to those of the other primers and have a calculated ΔG for primer duplexing below -10 kcal/mole, the concentration of the primers still needs to be adjusted to obtain similar yields of PCR product. To circumvent this problem and to also reduce primer-dimer formation further, Belgrader et al. (1996) applied only a limited amount of chimeric primer (2 pmol) and added an overload of universal primer after 15 PCR cycles. Thermal cycling proceeded then for another 25 cycles at a lower annealing temperature. Brownie et al. (1997) included the universal primer already in the PCR reaction mixture at the start. After 4 cycles of PCR the annealing temperature is raised from 60°C to 74°C and another 35 cycles of PCR is performed. Further, a nested PCR is taught by Heath et al. (2000) who applies in the second PCR two (complementary) universal primers, one of them carrying a fluorescent tag attached to the 5' end.

So far, multiplex PCR has only been used with relatively small sets of primer pairs for allele-specific amplification. Neither the feasibility of using a multiplex PCR in a method of genotyping a large number of blood cell antigens, nor the nature of the PCR conditions and the primer mixtures that are suitable for such blood cell antigen genotyping, nor a practical, rapid and reliable method for analyzing the products of the amplification to assign the clinically relevant blood cell antigen genotypes have been described in the prior art.

### DNA microarrays

Several approaches using microarray technology are known in the general field of genotyping. One of these approaches is the so-called mini-sequencing method, which includes an allele-specific extension either on the microarray or in solution (Pastinen et al., 1997; Fan et al., 2000). A difficulty in this approach is the occurrence of non-specific primer extension and further optimization of primers or the extension method itself is necessary (Pastinen et al., 2000; Lindroos et al., 2002). Moreover, this method requires laborious steps of enzymatic treatment and purification.

Another approach is called allele-specific oligonucleotide hybridization (ASO) on microarrays. This approach relies on the thermal stability of the target and short oligonucleotide probes for genotype determination (Hacia et al., 1996; Wang et al., 1998). The first light-generated oligonucleotide arrays were developed in 1991 (Fodor et al.). The method has been used for the determination of new SNPs or for resequencing. It has also been used for genotyping (Evans et al., 2002) by spotting the PCR amplification products derived from patient samples into an array and contacting the array with allele-specific oligos to discriminate the alleles of the patient. Many variations have been described, using different kinds of tools, like enzymes, nanoparticle probes, artificial nucleotides, thermal gradients, flow-through arrays, blocking oligonucleotides, for improving the sensitivity or specificity (Lu et al., 2002; Park et al., 2002; Prix et al., 2002; Kajiyama et al., 2003; Jobs et al., 2003; Van Beuningen et al., 2001; Iwasaki et al., 2002). Wen et al., 2000, made a comparison of the sensitivity and specificity of an oligonucleotide array analysis of TP53 mutations with conventional DNA sequence analysis. The oligonucleotide array used contained a plurality of probes per SNP. To immobilize the oligos to the matrix support, usually a glass slide, the probes may be provided with a spacer and an amine group (Guo et al., 2001; Wen et al., 2003). Various microarray formats have been described, for example slides with a 96-microarray format containing 250 spots per array to improve throughput (Huang et al., 2001). Flow-through systems have been described in order to reduce the hybridization time and thereby increase throughput (Cheek et al., 2001; Van Beuningen et al., 2001).

So far, DNA microarray methods have not been applied to genotyping of blood cell antigens and neither the feasibility of using a DNA microarray in a method of genotyping a large number of blood cell antigens, nor the nature of the oligonucleotide probes and microarray formats that are suitable for such blood cell antigen genotyping, nor a practical, rapid and reliable method for analyzing the hybridization results to assign the clinically relevant blood cell antigen genotypes have been described in the prior art.

### SUMMARY OF THE INVENTION

An object of the present invention is to provide methods and means allowing a practical, rapid and reliable genotyping of a large number of blood cell antigens.

Another object of the invention is to develop a high-throughput technique which allows genotyping of the whole existing donor cohort and/or the donor cohort increase for a number of blood cell antigens in the order of magnitude of 20 and ultimately even some 60 blood cell antigen systems, to thereby facilitate the selection of correct donor blood and improve the safety of blood transfusion.

Again another object of the invention is to achieve an essentially complete and reliable genotyping, at least covering the majority of clinically relevant blood cell antigen systems, using simple apparatus, in a short time, such as less than 30 hours, or less than 24 hours, preferably less than 6 hours and more preferably less than 2 hours or even less than 1 hour, on a single DNA sample subjected to one PCR reaction in a single reaction tube to simultaneously amplify and detectably label relevant DNA fragments.

These objects are achieved by the present invention which provides, in one aspect, a method of genotyping blood cell antigens comprising subjecting DNA from an individual of a mammalian species to a multiplex Polymerase Chain Reaction (PCR) to amplify and detectably label a region of the locus of at least two different blood cell antigens which contains the site of nucleotide polymorphism of said blood cell antigen and using the thus amplified and labeled DNA fragments to determine the genotype for each of said blood cell antigens, said multiplex PCR comprising the use of at least one pair of blood cell antigen-specific chimeric primers for each blood cell antigen to be genotyped and at least one detectably labeled universal primer, wherein said at least one universal primer has a unique sequence not occurring in the DNA of said mammalian species, and wherein each chimeric primer pair comprises a left chimeric primer and a right chimeric primer, each of them comprising a blood cell antigen-specific part at the 3' end and a universal part at the 5' end, wherein the base sequence of the universal part of the chimeric primers corresponds to the base sequence of said at least one universal primer, and wherein said blood cell antigen-specific parts of the chimeric primer pair enclose a region of the locus of the blood cell antigen which contains the site of nucleotide polymorphism of said blood cell antigen.

In the method of the invention, it is preferred that a pair of detectably labeled universal primers with a unique sequence not occurring in the DNA of said mammalian species is used and that for each chimeric primer pair the base sequence of the universal part of one member of the chimeric primer pair corresponds to the base sequence of one member of the universal primer pair and the base sequence of the universal part of the other member of the chimeric primer pair corresponds to the base sequence of the other member of the universal primer pair.

Further, it is strongly preferred in the present invention that the genotype for each of said blood cell antigens is determined by hybridizing the products of the multiplex PCR amplification, after denaturation, to blood cell antigen allele-specific oligonucleotide probes contained in a DNA array or presented in any other form, such as supported on beads, and analyzing the hybridization pattern.

In another aspect, the present invention provides a kit for genotyping blood cell antigens by a method as defined herein, comprising at least one pair of blood cell antigen-specific chimeric primers for each blood cell antigen to be genotyped and one pair of detectably labeled universal primers, both as defined herein.

This invention further provides a set of blood cell antigen-specific chimeric primer pairs useful in a multiplex PCR, and a set of blood cell antigen allele-specific oligonucleotide probes useful for genotyping blood cell antigens.

### BRIEF DESCRIPTION OF THE DRAWING

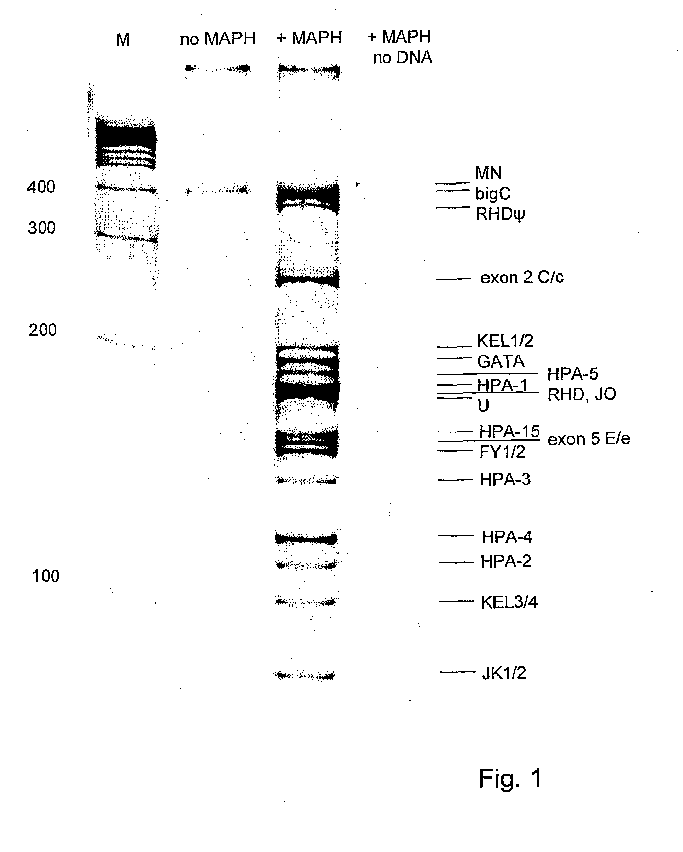

Figure 1 depicts the results of a multiplex PCR of 19 blood group antigens with or without the presence of universal MAPH primers. Very little amount of PCR product is amplified during the first amplification cycles (lane 2), but enough to be used as template by the universal MAPH primers in the following cycles. The MAPH primers perform the actual amplification (lane 3), so adjustments of primer concentrations are hardly necessary and similar yields of PCR product are obtained.

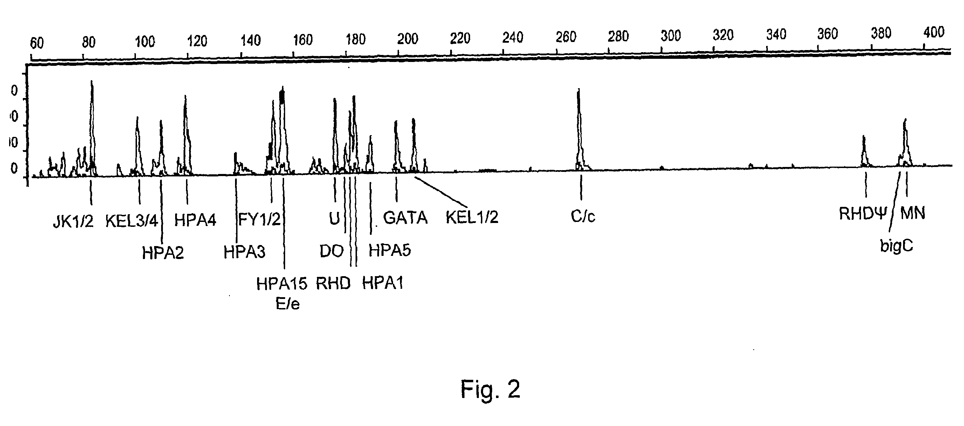

Figure 2 depicts the pattern obtained with ABI capillary sequencer from the multiplex PCR of 19 blood group antigens as described in this invention.

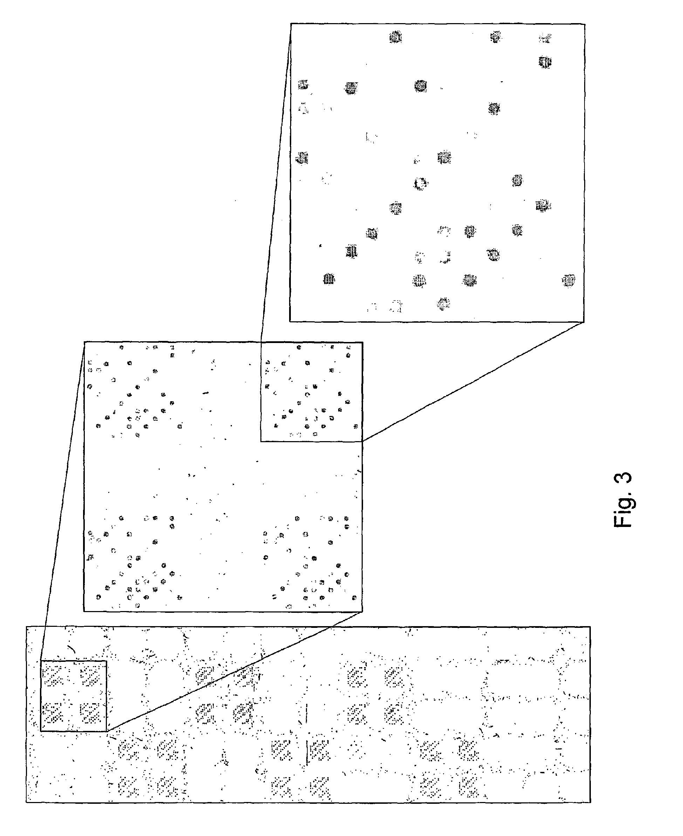

Figure 3 depicts the scan results of the hybridisation of 6 donor samples, each comprising the 6 human platelet antigen 1 through 5 and Gov PCR products. The PCR products of one sample were hybridised to two arrays, that is 4 blocks. The original scans have red spots and a black background. For better visualisation this is changed to grayscale and inverted.

### DETAILED DESCRIPTION OF THE INVENTION

The invention provides a method of genotyping blood cell antigens comprising subjecting DNA from an individual of a mammalian species to a multiplex Polymerase Chain Reaction (PCR) to amplify and detectably label a region of the locus of at least two different blood cell antigens which contains the site of nucleotide polymorphism of said blood cell antigen and using the thus amplified and labeled DNA fragments to determine the genotype for each of said blood cell antigens, said multiplex PCR comprising the use of at least one pair of blood cell antigen-specific chimeric primers for each blood cell antigen to be genotyped and at least one detectably labeled universal primer, wherein said at least one universal primer has a unique sequence not occurring in the DNA of said mammalian species, and wherein each chimeric primer pair comprises a left chimeric primer and a right chimeric primer, each of them comprising a blood cell antigen-specific part at the 3' end and a universal part at the 5' end, wherein the base sequence of the universal part of the chimeric primers corresponds to the base sequence of said at least one universal primer, and wherein said blood cell antigen-specific parts of the chimeric primer pair enclose a region of the locus of the blood cell antigen which contains the site of nucleotide polymorphism of said blood cell antigen.

More particularly, the invention provides a method of genotyping blood cell antigens comprising subjecting DNA from an individual of a mammalian species to a multiplex Polymerase Chain Reaction (PCR) to amplify and detectably label a region of the locus of at least two different blood cell antigens which contains the site of nucleotide polymorphism of said blood cell antigen and using the thus amplified and labeled DNA fragments to determine the genotype for each of said blood cell antigens, said multiplex PCR comprising the use of at least one pair of blood cell antigen-specific chimeric primers for each blood cell antigen to be genotyped and one pair of detectably labeled universal primers, wherein said pair of universal primers comprises a forward universal primer and a reverse universal primer each having a unique sequence not occurring in the DNA of said mammalian species, and wherein each chimeric primer pair comprises a left chimeric primer and a right chimeric primer, each of them comprising a blood cell antigen-specific part at the 3' end and a universal part at the 5' end, wherein the base sequence of the universal part of one of these chimeric primers corresponds to the base sequence of one of the universal primers and the base sequence of the universal part of the other chimeric primer corresponds to the base sequence of the other of the universal primers, and wherein said blood cell antigen-specific parts of the chimeric primer pair enclose a region of the locus of the blood cell antigen which contains the site of nucleotide polymorphism of said blood cell antigen.

The invention involves a particular sequence of steps that in combination allows to achieve the above mentioned objects. This sequence of steps comprises isolation of DNA from a sample which is to be subjected to the genotyping analysis of this invention, followed by one of the key aspects of the present invention, a multiplex PCR of gene fragments carrying the single nucleotide polymorphisms (SNPs) that are responsible for blood cell antigen differences. This multiplex PCR applies a mixture of different primers, including two or more pairs of chimeric primers and in addition including a labelled universal primer or a pair of labelled universal primers. Then the thus amplified and labelled DNA fragments are denatured (such as by heating) and are contacted with a set of probes, preferably contained in a microarray comprising a multitude of allele-specific probes, under conditions which support hybridisation between matching sequences. In the next step, the presence of labelled fragments is measured for each spot in the microarray, and finally the signals measured are interpreted to type the sample. The probes may be presented also in other formats, for example as probes supported on beads (glass beads, polystyrene beads, etc.), or probes attached to the wall of a suitable container, etc. An array format is however the most preferred embodiment.

### SPECIES

The invention allows the genotyping of blood cell antigens not only of humans, but of all animals with a blood cell antigen system. For all practical purposes, genotyping will relate to blood cell antigens of mammals, including for example farm animals and pets, wild-life animals and generally all animals of economic, emotional or wild life preservation value. Most preferably, however, the invention concerns the genotyping of human blood cell antigens.

### BLOOD CELL ANTIGENS

The blood cell antigens that may be genotyped by the present invention include typical blood group antigens, such as Kidd, Duffy, Kell and Rhesus. The blood group antigens of the AB0 system may be genotyped by the present invention as well, although it may be necessary, because of their extreme importance, to perform the existing and well-accepted serological test. Other blood group antigens that may be genotyped by the present invention comprise antigen variants and or gene variants leading to gene silencing of the blood group systems MNSs, Rhesus (Rh), Kell, Kidd, Duffy, Colton, Diego, Dombrock, Lutheran, Lewis, Cartwright, Landsteiner Wiener (LW), Cromer, Knops, Kx, Indian, Gerbich, Hh, Chido/Rodgers, GIL, I, JMH, OK, P-related, RAPH-MER2, Scianna, Xg, YT, etc.

In addition to the blood group antigens, also platelet and leukocyte (especially neutrophil) antigens may be genotyped by the method of this invention. The clinically most relevant platelet antigens, at least in the Western world, comprise HPA-1, HPA-2, HPA-3, HPA-4, HPA-5 and Gov (or HPA-15). Other platelet antigens that may be genotyped by the present invention, even though they are considered of lesser importance, comprise for example human platelet antigens 6 through 14 and 16. Examples of typical Human Neutrophil Antigens that may be genotyped by the method of the invention include HNA-1, 4 and 5. Others, such as HNA-2 and 3, can be genotyped as soon as their molecular basis has been elucidated.

It is conceived that the present invention is useful even when only a restricted number of blood cell antigens is genotyped. It may for some purposes be sufficient that only the red cell antigens are genotyped, or only the platelet antigens, or only a selected group of antigens which is relevant in a certain context. The minimum number of blood cell antigens to be genotyped by the present invention is two blood cell antigens, for example the blood cell antigen RhD and the RHD gene variant RhDΨ, or the blood cell antigen JK1/2 (Jk³/Jk^{b}) and the blood cell antigen FY1/2 (Fy^{a}/Fy^{b}). Genotyping of a larger number of blood cell antigens is clearly preferred, for example three, four, five, and especially six or more. For most purposes, however, a more or less complete genotyping, at least covering most of the clinically relevant blood cell antigens, is preferred. Most preferred, the invention includes at least six different chimeric primer pairs specific for HPA's 1 through 5 and HPA-15, and at least 10 or 11 or 12 chimeric primer pairs specific for red cell antigens, such as the various Kidd, Duffy, Kell and Rhesus antigens.

### THE DNA

The DNA subjected to the multiplex PCR will usually be genomic DNA of the individual whose blood cell antigens are to be genotyped

The donor DNA may be obtained from any suitable source, for example from EDTA, heparin or citrate treated blood samples or buffy coats, using isolation procedures and means well known to the skilled person, such as for example the commercially available Qiagen Blood DNA extraction kit, use of a salting-out method, such as by the standard protocol of Miller et al. (1988), or use of the Roche Magnapure.

### UNIVERSAL PRIMERS

The method of the invention uses at least one universal primer carrying a detectable label, preferably a pair of universal primers both carrying a detectable label. The sequence of the universal primer(s) corresponds with the sequence of the universal part at the 5' end of the chimeric primers. It is possible to use only one universal primer. In that case, all chimeric primers have an identical base sequence at their 5' end, i.e. the universal part at the 5' end of the chimeric primers corresponds with the sequence of said single universal primer. Practically, however, it was found preferable to use two different universal primers (i.e. a pair of universal primers). In that case, each pair of chimeric primers contains a primer with a universal part corresponding to one of the universal primers and a primer with a universal part corresponding to the other universal primer.

It is important that the universal primer(s) do not hybridize to the DNA which is to be genotyped. Assuming that the DNA to be genotyped is human genomic DNA, the universal primer(s) (and the corresponding parts of the chimeric primers) should not hybridize with human genomic DNA and therefore have a unique sequence not occurring in said DNA. Preferably, the sequence of the universal primer(s) differs significantly from any sequence occurring in the human genome. Further, it is strongly preferred that the universal primer(s) are designed such that there Tm value is similar to the Tm value of the blood cell antigen specific part of the chimeric primers. Preferably, the Tm value of the universal primer(s) is between 50 and 70°C, more preferably between 56 and 6S°C. It is also preferred that the universal primer(s) have a length of between 12 and 30 bases, more preferably between 15 and 25 or even between 16 and 20 bases.

Very good results were obtained with universal primers disclosed in White et al., 2002, with the sequences ggccgcgggaattcgatt (SEQ ID NO:1, forward MAPH), with a Tm of 67.55°C, and gccgcgaattcactagtg (SEQ ID NO:2, reverse MAPH) with a Tm of 57.94°C.

### DETECTABLE LABELS

The universal primer(s) carry a detectable label. The label may be any label suitable for the purpose, such as a radioactive atom or group, an enzyme, such as an enzyme catalyzing a measurable conversion of a substrate thereof, a dye, a fluorescent substance, a chemiluminescent substance, biotin, etc. Most preferably, the label is a fluorescent group, of which many are known to the person skilled in the art. Examples thereof are Cy3, Cy5, Fluorescein (FITC), Phycoerythrin (PE), Rhodamine, Hex, Tet, Fam, etc.

Most preferably, the invention uses the fluorescent group Cy5, which is a sulfoindocyanine dye (Mujumdar et al., 1993).

Usually, in particular when the label is a moiety attached to the oligonucleotide, it will be attached to the 5' end of the oligonucleotide sequence of the universal primer(s).

Because the universal primer(s) are labeled, while the chimeric primers are not labelled, the invention achieves very efficient labeling simultaneous with amplification, while the various chimeric primers can be used without previous labeling.

### CHIMERIC PRIMERS

The chimeric primers used in this invention have a universal part at their 5' end and a blood cell antigen-specific part at their 3' end. The oligonucleotide sequence of the universal part corresponds with the sequence of the universal primer(s) and requires no further elucidation here.

As to the blood cell antigen-specific part of the chimeric primers, it is most preferable that these parts have similar Tm values, both in comparison with blood cell antigen-specific parts of other chimeric primers in the mixture, and in comparison with the universal primers used. Thus, it is preferred that these blood cell antigen-specific parts of the chimeric primers have Tm values between 50 and 70°C, more preferably between 55 and 65°C and most preferably between 56 and 62°C. It is also preferred that the blood cell antigen-specific parts of the chimeric primers have a length of between 12 and 35 bases, more preferably between 15 and 30 or even between 16 and 25 bases.

It is further preferred that the blood cell antigen-specific parts of the chimeric primers are selected in such a way that the PCR amplification results in oligonucleotide products with a length of between 50 and 800, preferably between 80 and 500, most preferably between 100 and 400 or 300 nucleotides. A product length of from 100 to 200 nucleotides would be ideal.

Various software products are available that can be used to design suitable blood cell antigen-specific parts of the chimeric primers. On the basis of the publicly available DNA sequences of the blood cell antigen-encoding genes, the software product Primer3

(http://www.broad.mit.edu/cgi-bin/primer/primer3 www.cgi) allows to design primers. To check for any unspecific genomic DNA binding the CELERA database can be used and to check for any primer-dimer formation the Oligo6 software (Medprobe) can be used. It is preferred to select primers having a calculated ΔG below -10 kcal/mole.

A list of preferred chimeric primers includes the following:

| | | |
|---|---|---|
| HPA1-left | gccgcgaattcactagtgcttcaggtcacagcgaggt | SEQ ID NO:3 |
| HPA1-right | ggccgcgggaattcgattgctccaatgtacggggtaaa | SEQ ID NO:4 |
| | | |
| HPA2-left | gccgcgaattcactagtgtgaaaggcaatgagctgaag | SEQ ID NO:5 |
| HPA2-right | ggccgcgggaattcgattagccagactgagcttctcca | SEQ ID NO:6 |
| | | |
| HPA3-left | gccgcgaattcactagtggcctgaccactcctttgc | SEQ ID NO:7 |
| HPA3-right | ggccgcgggaattcgattggaagatctgtctgcgatcc | SEQ ID NO:8 |
| | | |
| HPA4-left | gccgcgaattcactagtgatccgcaggttactggtgag | SEQ ID NO:9 |
| HPA4-right | ggccgcgggaattcgattccatgaaggatgatctgtgg | SEQ ID NO: 10 |
| | | |
| HPA5-left | gccgcgaattcactagtgtccaaatgcaagttaaattaccag | SEQ ID NO:11 |
| HPA5-right | ggccgcgggaattcgattacagacgtgctcttggtaggt | SEQ ID NO:12 |
| | | |
| HPA15-left | gccgcgaattcactagtgtgtatcagttcttggttttgtgatg | SEQ ID NO:13 |
| HPA15-right | ggccgcgggaattcgattaaaaccagtagccacccaag | SEQ ID NO:14 |
| | | |
| JK1/2-left | gccgcgaattcactagtggtctttcagccccatttgag | SEQ ID NO:15 |
| JK1/2-right | ggccgcgggaattcgattgttgaaaccccagagtccaa | SEQ ID NO:16 |
| | | |
| FY1/2-left | gccgcgaattcactagtggaattcttcctatggtgtgaatga | SEQ ID NO:17 |
| FY1/2-right | ggccgcgggaattcgattaagaagggcagtgcagagtc | SEQ ID NO:18 |
| | | |
| GATAbox-left | gccgcgaattcactagtgggccctcattagtccttgg | SEQ ID NO:19 |
| GATAbox-right | ggccgcgggaattcgattgaaatgaggggcatagggata | SEQ ID NO:20 |
| | | |
| Fyx-left | gccgcgaattcactagtgtcatgcttttcagacctctcttc | SEQ ID NO:175 |
| Fyx-right | ggccgcgggaattcgattcaagacgggcaccacaat | SEQ ID NO:176 |
| | | |
| KEL1/2-left | gccgcgaattcactagtgaagggaaatggccatactga | SEQ ID NO:21 |
| KEL1/2-right | ggccgcgggaattcgattagctgtgtaagagccgatcc | SEQ ID NO:22 |
| | | |
| KEL3/4-left | gccgcgaattcactagtggcctcagaaactggaacagc | SEQ ID NO:23 |
| KEL3/4-right | ggccgcgggaattcgattagcaaggtgcaagaacactct | SEQ ID NO:24 |
| | | |
| KEL6/7-left | gccgcgaattcactagtggcagcaccaaccctatgttc | SEQ ID NO:177 |
| KEL6/7-right | ggccgcgggaattcgatttcaggcacaggtgagcttc | SEQ ID NO:178 |
| | | |
| RHCEex2for | gccgcgaattcactagtgcgtctgcttccccctcc | SEQ ID NO:25 |
| RHex2rev | ggccgcgggaattcgattctgaacagtgtgatgaccacc | SEQ ID NO:26 |
| | | |
| RHDex3-left | gccgcgaattcactagtgtcctggctctccctctct | SEQ ID NO:179 |
| RHCEex3-right | ggccgcgggaattcgatttttttcaaaaccccggaag | SEQ ID NO:180 |
| | | |
| RHCEex5-left | gccgcgaattcactagtgggatgttctggccaagtg | SEQ ID NO:27 |
| RHex5rev | ggccgcgggaattcgattggctgtcaccacactgactg | SEQ ID NO:28 |
| | | |
| RHDΨ-left | ggccgcgggaattcgattgtagtgagctggcccatca | SEQ ID NO:29 |
| RHDΨ-right | gccgcgaattcactagtgtgtctagtttcttaccggcaagt | SEQ ID NO:30 |
| | | |
| RHD-leftB | gccgcgaattcactagtgttataataacacttgtccacaggg | SEQ ID NO:31 |
| RHD-rightC | ggccgcgggaattcgattcggctccgacggtatc | SEQ ID NO:32 |
| | | |
| BigC-left | gccgcgaattcactagtgggccaccaccatttgaa | SEQ ID NO:33 |
| BigC-rightintron2 | ggccgcgggaattcgattccatgaacatgccacttcac | SEQ ID NO:34 |
| | | |
| RhDVI-left (fw) | ggccgcgggaattcgattctttgaattaagcacttcacaga | SEQ ID NO:181 |
| RhDVI-right (rev) | gccgcgaattcactagtggccagaatcacactcctgct | SEQ ID NO:182 |
| | | |
| MN-left | gccgcgaattcactagtgtgagggaatttgtcttttgca | SEQ ID NO:35 |
| MN-right | ggccgcgggaattcgattcagaggcaagaattcctcca | SEQ ID NO:36 |
| | | |
| Ss-left | gccgcgaattcactagtgtttttctttgcacatgtcttt | SEQ ID NO:183 |
| Ss-right | ggccgcgggaattcgatttctttgtctttacaatttcgtgtg | SEQ ID NO:184 |
| | | |
| U-left | gccgcgaattcactagtgcgctgatgttatctgtcttatttttc | SEQ ID NO:37 |
| U-right | ggccgcgggaattcgattgatcgttccaataataccagcc | SEQ ID NO:38 |
| | | |
| DO-left | ggccgcgggaattcgatttgatccctccctatgagctg | SEQ ID NO:185 |
| DO-right | gccgcgaattcactagtgttatatgtgctcaggttcccagt | SEQ ID NO:186 |
| | | |
| JO-left | gccgcgaattcactagtgcctggcttaaccaaggaaaa | SEQ ID NO:39 |
| JO-right | ggccgcgggaattcgatttcatactgctgtggagtcctg | SEQ ID NO:40 |
| Colton-left | gccgcgaattcactagtggccacgaccctctttgtct | SEQ ID NO:187 |
| Colton-right | ggccgcgggaattcgatttacatgagggcacggaagat | SEQ ID NO:188 |
| | | |
| Diego-left | gccgcgaattcactagtgacttattcacgggcatccag | SEQ ID NO:189 |
| Diego-right | ggccgcgggaattcgattaagctccacgttcctgaaga | SEQ ID NO:190 |
| | | |
| Wr-left | gccgcgaattcactagtgggcttcaaggtgtccaactc | SEQ ID NO:636 |
| Wr-right | ggccgcgggaattcgattaggatgaagaccagcagagc | SEQ ID NO:637 |
| | | |
| Yt-left | ggccgcgggaattcgattccttcgtgcctgtggtagat | SEQ ID NO:638 |
| Yt-right | gccgcgaattcactagtgttctgggacttctgggaatg | SEQ ID NO:639 |
| | | |
| Lu-left | gccgcgaattcactagtgggacccagagagagagagactg | SEQ ID NO:640 |
| Lu-right | ggccgcgggaattcgattgggagtccagctggtatgg | SEQ ID NO:641. |

Most preferred are the chimeric primers with SEQ ID NOS:3 to 40.

The above mentioned software products would allow the skilled person to design additional primers for other blood cell antigens. The present invention encompasses the use of a set of similar chimeric primers having the same blood cell antigen-specific part but a different universal part.

### PROPORTIONS OF THE PRIMERS

To achieve the aims of the invention, and in particular to avoid the occurrence of primer-dimers as much as possible, it is important that only a minimal proportion of the chimeric primers is used and that amplification is due to a large extent to elongation of the universal primers. Practically, it is preferred that the universal primers are used in a molar amount which is at least 10 times, more preferably at least 40 times the molar amount of each chimeric primer. Per amplification reaction 5 nM of each chimeric primer and 0.2 µM of each universal primer per chimeric primer pair is preferred. Per microliter reaction volume, an amount of 5 femtomol (5*10⁻¹⁵ mol) of each chimeric primer would be preferred, while about 0.2 pmol (0.2*10⁻¹² mol) of each universal primer is used per chimeric primer pair.

### MULTIPLEX PCR CONDITIONS

The multiplex PCR in the method of this invention uses a mixture of universal and chimeric primers which are present from the beginning of the reaction. The PCR as used in this invention does not distinguish between a separate first part in which only chimeric primers are extended, and a second part in which only universal primers are extended. The present invention applies an annealing or primer extension temperature in the later cycles of the PCR which is the same as the annealing or primer extension temperature used in the first few cycles of the PCR. The inventors found that a change of reaction conditions in order to switch from chimeric primer extension to universal primer extension is not required.

The DNA polymerase used in the multiplex PCR is preferably a heat-resistant DNA polymerase, such as Taq DNA polymerase. Other heat-resistant DNA polymerases are useful as well, and even heat-sensitive DNA polymerases, although this may require repeated addition of a fresh amount of DNA polymerase. Suitable DNA polymerases are commercially available, for example as a component of multiplex PCR kits, like the Qiagen multiplex kit. Such kits also contain other necessary components, such as the required dNTP's and a suitable buffer system.

Apparatus for carrying out PCR thermocycling is commercially available as well, for example the MWG AG Biotech PrimusHT thermal cycler.

The amplification of the invention usually starts with a heat treatment to activate the DNA polymerase, for example 15 min at 95°C. Subsequently, the thermal cycling begins. Each cycle includes a heat denaturation step, an annealing step (to bind primer by hybridization to its template) and a primer extension or elongation step. In the invention, it is preferred that the heat denaturation step involves heating at a temperature of 90 to 98°C, more preferably at about 94 or 95°C, and takes a time of from 15 to 60 seconds (shorter and especially longer times are allowable), more preferably about 30 seconds. In the invention, it is further preferred that the annealing step involves heating at a temperature of 54 to 60°C, more preferably at about 57°C, and takes a time of from 60 to 120 seconds (shorter and especially longer times are allowable), more preferably about 90 seconds. In the invention, it is further preferred that the primer extension step involves heating at a temperature of 68 to 76°C, more preferably at about 72°C, and takes a time of from 60 to 120 seconds (shorter and especially longer times are allowable), more preferably about 90 seconds. A final treatment at the temperature of the primer extension step for a much longer time, for example 10 minutes, may conclude the thermal cycling.

The total number of cycles may be chosen at will, depending on the available time and the precision sought. A total of 30 cycles may be enough, but 40 or 45 or 50 cycles or more is preferable.

### GENOTYPING

The result of the multiplex PCR is a mixture of labelled amplification products, in which ideally each product essentially comprises a region of the locus of different blood cell antigens which contains the site of nucleotide polymorphism of said blood cell antigen. In most cases of blood cell antigen polymorphisms that are presently known, the nucleotide polymorphism is a single nucleotide polymorphism (SNP), but polymorphism sites covering more than one nucleotide are encompassed as well.

To analyse the SNP's present in the mixture of labelled amplification products, various strategies are available, including sequencing of the products, but many of these are not suitable for rapid and reliable high-throughput genotyping. The present invention therefore preferably applies the special step of contacting the products of the multiplex PCR with a DNA array which contains blood cell antigen allele-specific oligonucleotide probes. Said contacting is done after denaturation, for example by heating, of the oligonucleotidic products of the amplification and under conditions suitable for hybridization of these products to corresponding probes in the array. Purification of the products of the amplification before contacting them with the probes is not necessary and will usually be omitted.

### PROBES

The probes are oligonucleotides including the site of the nucleotide polymorphism. Thereby, they are allele-specific. Although their length may vary from only 8 or 10 nucleotides up to several hundreds of nucleotides, it is preferred in this invention to use probes with a length of from 15 to 40 nucleotides, more preferably 16 or 17 to 29 or 30 nucleotides. Preferably, all probes have similar Tm values, in particular in a range of 55 to 75°C, more preferably in the range of 60 to 70°C or especially in the range of 60 to 65°C.

Although in principle it is possible to use only one probe for each allele of each blood cell antigen, the invention applies several probes for each allele of each blood cell antigen, thereby increasing the reliability of the method. According to this invention, the array contains for each allele of each blood cell antigen at least two, preferably at least five, different sense probes and at least two, preferably at least five, antisense probes, each of them covering the site of the nucleotide polymorphism but at varying positions. Preferably, these positions are in or near the centre of the oligos.

Various software products, as mentioned above for the blood cell antigen-specific parts of the primers, are available that can be used to design suitable blood cell antigen allele-specific probes (in particular the sofware product Primer3).

A list of preferred probes includes the following (SNPs are highlighted):

| HPA1-allele a: | | | |
|---|---|---|---|
| | HPA-1aa | tacaggccctgcctctggg | SEQ ID NO:41 |
| | HPA-1ab | aggccctgcctctgggct | SEQ ID NO:42 |
| | HPA-1ac | ccctgcctctgggctcacc | SEQ ID NO:43 |
| | HPA-1ad | tgcctctgggctcacctcg | SEQ ID NO:44 |
| | HPA-1ae | ctctgggctcacctcgctg | SEQ ID NO:45 |
| | HPA-1aa CR | cagcgaggtgagcccagag | SEQ ID NO:46 |
| | HPA-1ab CR | cgaggtgagcccagaggca | SEQ ID NO:47 |
| | HPA-1ac CR | ggtgagcccagaggcaggg | SEQ ID NO:48 |
| | HPA-1ad CR | agcccagaggcagggcct | SEQ ID NO:49 |
| | HPA-1ae CR | cccagaggcagggcctgta | SEQ ID NO:50 |

| HPA1-allele b: | | | |
|---|---|---|---|
| | HPA-1ba | tacaggccctgcctccggg | SEQ ID NO:51 |
| | HPA-1bb | aggccctgcctccgggct | SEQ ID NO:52 |
| | HPA-1bc | ccctgcctccgggctcac | SEQ ID NO:53 |
| | HPA-1bd | ctgcctccgggctcacct | SEQ ID NO:54 |
| | HPA-1be | ctccgggctcacctcgct | SEQ ID NO:55 |
| | HPA-1ba CR | agcgaggtgagcccggag | SEQ ID NO:56 |
| | HPA-1bb CR | aggtgagcccggaggcag | SEQ ID NO:57 |
| | HPA-1bc CR | gtgagcccggaggcaggg | SEQ ID NO:58 |
| | HPA-1bd CR | agcccggaggcagggcct | SEQ ID NO:59 |
| | HPA-1be CR | cccggaggcagggcctgta | SEQ ID NO:60 |

| HPA2-allele a: | | | |
|---|---|---|---|
| | HPA-2aa | ctgacgcccacacccaag | SEQ ID NO:61 |
| | HPA-2ab | ctcctgacgcccacaccc | SEQ ID NO:62 |
| | HPA-2ac | ggctcctgacgcccacac | SEQ ID NO:63 |
| | HPA-2ad | agggctcctgacgcccac | SEQ ID NO:64 |
| | HPA-2ae | ccagggctcctgacgccc | SEQ ID NO:65 |
| | HPA-2aa CR | cttgggtgtgggcgtcag | SEQ ID NO:66 |
| | HPA-2ab CR | gggtgtgggcgtcaggag | SEQ ID NO:67 |
| | HPA-2ac CR | gtgtgggcgtcaggagcc | SEQ ID NO:68 |
| | HPA-2ad CR | gtgggcgtcaggagccct | SEQ ID NO:69 |
| | HPA-2ae CR | gggcgtcaggagccctgg | SEQ ID NO:70 |

| HPA2-allele b: | | | |
|---|---|---|---|
| | HPA-2ba | cctgatgcccacacccaag | SEQ ID NO:71 |
| | HPA-2bb | ctcctgatgcccacaccca | SEQ ID NO:72 |
| | HPA-2bc | ggctcctgatgcccacacc | SEQ ID NO:73 |
| | HPA-2bd | agggctcctgatgcccaca | SEQ ID NO:74 |
| | HPA-2be | ccagggctcctgatgccc | SEQ ID NO:75 |
| | HPA-2ba CR | cttgggtgtgggcatcagg | SEQ ID NO:76 |
| | HPA-2bb CR | tgggtgtgggcatcaggag | SEQ ID NO:77 |
| | HPA-2bc CR | ggtgtgggcatcaggagcc | SEQ ID NO:78 |
| | HPA-2bd CR | tgtgggcatcaggagccct | SEQ ID NO:79 |
| | HPA-2be CR | gggcatcaggagccctgg | SEQ ID NO:80 |

| HPA3-allele a: | | | |
|---|---|---|---|
| | HPA-3 aa | ccatccccagcccctccc | SEQ ID NO:81 |
| | HPA-3ab | gcccatccccagcccctc | SEQ ID NO:82 |
| | HPA-3ac | ctgcccatccccagcccc | SEQ ID NO:83 |
| | HPA-3ad1 | gctgcccatccccagccc | SEQ ID NO:84 |
| | HPA-3 ad | ggctgcccatccccagcc | SEQ ID NO:85 |
| | HPA-3ad2 | gggctgcccatccccagc | SEQ ID NO:86 |
| | HPA-3ae | ggggctgcccatcccca | SEQ ID NO:87 |
| | HPA-3 aa CR | gggaggggctggggatgg | SEQ ID NO:88 |
| | HPA-3ab CR | gaggggctggggatgggc | SEQ ID NO:89 |
| | HPA-3ac CR | ggggctggggatgggcag | SEQ ID NO:90 |
| | HPA-3ad1 CR | gggctggggatgggcagc | SEQ ID NO:91 |
| | HPA-3ad CR | ggctggggatgggcagcc | SEQ ID NO:92 |
| | HPA-3ad2 CR | gctggggatgggcagccc | SEQ ID NO:93 |
| | HPA-3ae CR | tggggatgggcagcccc | SEQ ID NO:94 |

| HPA3-allele b: | | | |
|---|---|---|---|
| | HPA-3ba | ccagccccagcccctcc | SEQ ID NO:95 |
| | HPA-3bb | gcccagccccagcccct | SEQ ID NO:96 |
| | HPA-3bc | ctgcccagccccagccc | SEQ ID NO:97 |
| | HPA-3bd1 | gctgcccagccccagcc | SEQ ID NO:98 |
| | HPA-3bd | ggctgcccagccccagc | SEQ ID NO:99 |
| | HPA-3bd2 | gggctgcccagccccag | SEQ ID NO:100 |
| | HPA-3be | ggggctgcccagcccca | SEQ ID NO:101 |
| | HPA-3ba CR | ggaggggctggggctgg | SEQ ID NO:102 |
| | HPA-3bb CR | aggggctggggctgggc | SEQ ID NO:103 |
| | HPA-3bc CR | gggctggggctgggcag | SEQ ID NO:104 |
| | HPA-3bd1 CR | ggctggggctgggcagc | SEQ ID NO:105 |
| | HPA-3bd CR | gctggggctgggcagcc | SEQ ID NO:106 |
| | HPA-3bd2 CR | ctggggctgggcagccc | SEQ ID NO:107 |
| | HPA-3be CR | tggggctgggcagcccc | SEQ ID NO:108 |

| HPA4-allele a: | | | |
|---|---|---|---|
| | HPA-4aa | gccacccagatgcgaaag | SEQ ID NO:109 |
| | HPA-4ab | cacccagatgcgaaagct | SEQ ID NO:110 |
| | HPA-4ac | cccagatgcgaaagctca | SEQ ID NO:111 |
| | HPA-4ad | cagatgcgaaagctcacc | SEQ ID NO:112 |
| | HPA-4ae | gatgcgaaagctcaccag | SEQ ID NO:113 |
| | HPA-4aa CR | ctggtgagctttcgcatc | SEQ ID NO:114 |
| | HPA-4ab CR | ggtgagctttcgcatctg | SEQ ID NO:115 |
| | HPA-4ac CR | tgagctttcgcatctggg | SEQ ID NO:116 |
| | HPA-4ad CR | agctttcgcatctgggtg | SEQ ID NO:117 |
| | HPA-4ae CR | ctttcgcatctgggtggc | SEQ ID NO:118 |

| HPA4-allele b: | | | |
|---|---|---|---|
| | HPA-4ba | gccacccagatgcaaaag | SEQ ID NO:119 |
| | HPA-4bb | ccacccagatgcaaaagct | SEQ ID NO:120 |
| | HPA-4bc | acccagatgcaaaagctcac | SEQ ID NO:121 |
| | HPA-4bd | cagatgcaaaagctcacca | SEQ ID NO:122 |
| | HPA-4be | gatgcaaaagctcaccagtaa | SEQ ID NO:123 |
| | HPA-4ba CR | ttactggtgagcttttgcatc | SEQ ID NO:124 |
| | HPA-4bb CR | tggtgagcttttgcatctg | SEQ ID NO:125 |
| | HPA-4bc CR | gtgagcttttgcatctgggt | SEQ ID NO:126 |
| | HPA-4bd CR | agcttttgcatctgggtgg | SEQ ID NO:127 |
| | HPA-4be CR | cttttgcatctgggtggc | SEQ ID NO:128 |

| HPA5-allele a: | | | |
|---|---|---|---|
| | HPA-5aa | gagtctacctgtttactatcaaagagg | SEQ ID NO:129 |
| | HPA-5ab | agtctacctgtttactatcaaagaggta | SEQ ID NO:130 |
| | HPA-5ac | gtctacctgtttactatcaaagaggtaa | SEQ ID NO:131 |
| | HPA-5ad | ctacctgtttactatcaaagaggtaaaa | SEQ ID NO:132 |
| | HPA-5ae | acctgtttactatcaaagaggtaaaaa | SEQ ID NO:133 |
| | HPA-5aa CR | tttttacctctttgatagtaaacaggt | SEQ ID NO:134 |
| | HPA-5ab CR | ttttacctctttgatagtaaacaggtag | SEQ ID NO:135 |
| | HPA-5ac CR | ttacctctttgatagtaaacaggtagac | SEQ ID NO:136 |
| | HPA-5ad CR | tacctctttgatagtaaacaggtagact | SEQ ID NO:137 |
| | HPA-5ae CR | cctctttgatagtaaacaggtagactc | SEQ ID NO:138 |

| HPA5-allele b: | | | |
|---|---|---|---|
| | HPA-5ba | gagtctacctgtttactatcaaaaagg | SEQ ID NO:139 |
| | BPA-5bb | agtctacctgtttactatcaaaaaggta | SEQ ID NO:140 |
| | HPA-5bc | gtctacctgtttactatcaaaaaggtaa | SEQ ID NO:141 |
| | HPA-5ad | ctacctgtttactatcaaaaaggtaaaa | SEQ ID NO:142 |
| | HPA-5be | acctgtttactatcaaaaaggtaaaaa | SEQ ID NO:143 |
| | HPA-5ba CR | tttttacctttttgatagtaaacaggt | SEQ ID NO:144 |
| | HPA-5bb CR | ttttacctttttgatagtaaacaggtag | SEQ ID NO:145 |
| | HPA-5bc CR | ttacctttttgatagtaaacaggtagac | SEQ ID NO:146 |
| | HPA-5bd CR | tacctttttgatagtaaacaggtagact | SEQ ID NO:147 |
| | HPA-5be CR | cctttttgatagtaaacaggtagactc | SEQ ID NO:148 |

| HPA15-allele a: | | | |
|---|---|---|---|
| | Gov-aa | ttattatcttgacttcagttacaggattt | SEQ ID NO:149 |
| | Gov-ab | tcttgacttcagttacaggatttacc | SEQ ID NO:150 |
| | Gov-ac | tgacttcagttacaggatttaccaa | SEQ ID NO:151 |
| | Gov-ad | cttcagttacaggatttaccaagaat | SEQ ID NO:152 |
| | Gov-ae | cagttacaggatttaccaagaatttg | SEQ ID NO:153 |
| | Gov-aa CR | caaattcttggtaaatcctgtaactg | SEQ ID NO:154 |
| | Gov-ab CR | attcttggtaaatcctgtaactgaag | SEQ ID NO:155 |
| | Gov-ac CR | tggtaaatcctgtaactgaagtcaa | SEQ ID NO:156 |
| | Gov-ad CR | ggtaaatcctgtaactgaagtcaaga | SEQ ID NO:157 |
| | Gov-ae CR | aaatcctgtaactgaagtcaagataataa | SEQ ID NO:158 |

| HPA15-allele b: | | | |
|---|---|---|---|
| | Gov-ba | tatcttgacttcagttccaggatt | SEQ ID NO:159 |
| | Gov-bb | cttgacttcagttccaggatttac | SEQ ID NO:160 |
| | Gov-bc | gacttcagttccaggatttacca | SEQ ID NO:161 |
| | Gov-bd | ttcagttccaggatttaccaag | SEQ ID NO:162 |
| | Gov-be | cagttccaggatttaccaagaatt | SEQ ID NO:163 |
| | Gov-ba CR | aattcttggtaaatcctggaactg | SEQ ID NO:164 |
| | Gov-bb CR | cttggtaaatcctggaactgaa | SEQ ID NO:165 |
| | Gov-bc CR | ggtaaatcctggaactgaagtca | SEQ ID NO:166 |
| | Gov-bd CR | gtaaatcctggaactgaagtcaag | SEQ ID NO:167 |
| | Gov-be CR | aatcctggaactgaagtcaagata. | SEQ ID NO:168 |

| Colton-a-allele: | | | |
|---|---|---|---|
| | Co.a.1 | aacaaccagacggcggt | SEQ ID NO:191 |
| | Co.a.2 | accagacggcggtccag | SEQ ID NO:192 |
| | Co.a.3 | cagacggcggtccagga | SEQ ID NO:193 |
| | Co.a.4 | gacggcggtccaggacaa | SEQ ID NO:194 |
| | Co.a.5 | cggcggtccaggacaac | SEQ ID NO:195 |
| | Co.a.1.cr | gttgtcctggaccgccgt | SEQ ID NO:196 |
| | Co.a.2.cr | tcctggaccgccgtctg | SEQ ID NO:197 |
| | Co.a.3.cr | ctggaccgccgtctggt | SEQ ID NO:198 |
| | Co.a.4.cr | ggaccgccgtctggttg | SEQ ID NO:199 |
| | Co.a.5.cr | accgccgtctggttgtt | SEQ ID NO:200 |

| Colton-b-allele: | | | |
|---|---|---|---|
| | Co.b.1 | ggaacaaccagacggtggt | SEQ ID NO:201 |
| | Co.b.2 | acaaccagacggtggtccag | SEQ ID NO:202 |
| | Co.b.3 | accagacggtggtccagga | SEQ ID NO:203 |
| | Co.b.4 | gacggtggtccaggacaacg | SEQ ID NO:204 |
| | Co.b.5 | cggtggtccaggacaacg | SEQ ID NO:205 |
| | Co.b.1.cr | cgttgtcctggaccaccgt | SEQ ID NO:206 |
| | Co.b.2.cr | ttgtcctggaccaccgtctg | SEQ ID NO:207 |
| | Co.b.3.cr | ctggaccaccgtctggttgt | SEQ ID NO:208 |
| | Co.b.4.cr | ggaccaccgtctggttgttc | SEQ ID NO:209 |
| | Co.b.5.cr | accaccgtctggttgttcc | SEQ ID NO:210 |

| Diego-a-allele: | | | |
|---|---|---|---|
| | Di.a.1 | gtgaagtccacgccggc | SEQ ID NO:211 |
| | Di.a.2 | gaagtccacgccggcct | SEQ ID NO:212 |
| | Di.a.3 | agtccacgccggcctcc | SEQ ID NO:213 |
| | Di.a.4 | tccacgccggcctccct | SEQ ID NO:214 |
| | Di.a.5 | acgccggcctccctggcc | SEQ ID NO:215 |
| | Di.a.1.cr | gccagggaggccggcgt | SEQ ID NO:216 |
| | Di.a.2.cr | agggaggccggcgtgga | SEQ ID NO:217 |
| | Di.a.3.cr | ggaggccggcgtggact | SEQ ID NO:218 |
| | Di.a.4.cr | aggccggcgtggacttc | SEQ ID NO:219 |
| | Di.a.5.cr | ggccggcgtggacttca | SEQ ID NO:220 |

| Diego-b-allele: | | | |
|---|---|---|---|
| | Di.b.1 | ggtgaagtccacgctggc | SEQ ID NO:221 |
| | Di.b.2 | gtgaagtccacgctggcct | SEQ ID NO:222 |
| | Di.b.3 | tgaagtccacgctggcctcc | SEQ ID NO:223 |
| | Di.b.4 | gaagtccacgctggcctccct | SEQ ID NO:224 |
| | Di.b.5 | acgctggcctccctggccc | SEQ ID NO:225 |
| | Di.b.1.cr | ggccagggaggccagcgt | SEQ ID NO:226 |
| | Di.b.2.cr | cagggaggccagcgtgga | SEQ ID NO:227 |
| | Di.b.3.cr | agggaggccagcgtggact | SEQ ID NO:228 |
| | Di.b.4.cr | ggaggccagcgtggacttc | SEQ ID NO:229 |
| | Di.b.5.cr | ggccagcgtggacttcacc | SEQ ID NO:230 |

| Diego Wr-a-allele: | | | |
|---|---|---|---|
| | Wr.a.1 | tgggcttgcgttccaagt | SEQ ID NO:231 |
| | Wr.a.2 | ggcttgcgttccaagtttc | SEQ ID NO:232 |
| | Wr.a.3 | ttgcgttccaagtttccca | SEQ ID NO:233 |
| | Wr.a.4 | cgttccaagtttcccatct | SEQ ID NO:234 |
| | Wr.a.5 | tccaagtttcccatctgga | SEQ ID NO:235 |
| | Wr.a.1 CR | tccagatgggaaacttgga | SEQ ID NO:236 |
| | Wr.a.2 CR | agatgggaaacttggaacg | SEQ ID NO:237 |
| | Wr.a.3 CR | tgggaaacttggaacgcaa | SEQ ID NO:238 |
| | Wr.a.4 CR | gaaacttggaacgcaagcc | SEQ ID NO:239 |
| | Wr.a.5 CR | acttggaacgcaagccca | SEQ ID NO:240 |

| Diego Wr-b-allele:2 | | | |
|---|---|---|---|
| | Wr.b.1 | gggcttgcgttccgagtt | SEQ ID NO:241 |
| | Wr.b.2 | gcttgcgttccgagtttc | SEQ ID NO:242 |
| | Wr.b.3 | ttgcgttccgagtttccc | SEQ ID NO:243 |
| | Wr.b.4 | cgttccgagtttcccatc | SEQ ID NO:244 |
| | Wr.b.5 | tccgagtttcccatctgg | SEQ ID NO:245 |
| | Wr.b.1 CR | ccagatgggaaactcgga | SEQ ID NO:246 |
| | Wr.b.2 CR | gatgggaaactcggaacg | SEQ ID NO:247 |
| | Wr.b.3 CR | gggaaactcggaacgcaa | SEQ ID NO:248 |
| | Wr.b.4 CR | gaaactcggaacgcaagc | SEQ ID NO:249 |
| | Wr.b.5 CR | aactcggaacgcaagccc | SEQ ID NO:250 |

| Dombrock-a-allele: | | | |
|---|---|---|---|
| | Do.a.1 | taccacccaagaggaaact | SEQ ID NO:251 |
| | Do.a.2 | ccacccaagaggaaactgg | SEQ ID NO:252 |
| | Do.a.3 | acccaagaggaaactggttg | SEQ ID NO:253 |
| | Do.a.4 | caagaggaaactggttgca | SEQ ID NO:254 |
| | Do.a.5 | aggaaactggttgcagttga | SEQ ID NO:255 |
| | Do a 6 cr | ctcaactgcaaccagtttcc | SEQ ID NO:256 |
| | Do a 7 cr | caactgcaaccagtttcctc | SEQ ID NO:257 |
| | Do a 8 cr | tgcaaccagtttcctcttgg | SEQ ID NO:258 |
| | Do a 9 cr | accagtttcctcttgggtgg | SEQ ID NO:259 |
| | Do a 10 cr | cagtttcctcttgggtggta | SEQ ID NO:260 |

| Dombrock-b-allele: | | | |
|---|---|---|---|
| | Do.b.1 | taccacccaagaggagact | SEQ ID NO:261 |
| | Do.b.2 | ccacccaagaggagactgg | SEQ ID NO:262 |
| | Do.b.3 | acccaagaggagactggttg | SEQ ID NO:263 |
| | Do.b.4 | caagaggagactggttgca | SEQ ID NO:264 |
| | Do.b.5 | aggagactggttgcagttga | SEQ ID NO:265 |
| | Do b 6 cr | ctcaactgcaaccagtctcc | SEQ ID NO:266 |
| | Do b 7 cr | caactgcaaccagtctcctc | SEQ ID NO:267 |
| | Do b 8 cr | tgcaaccagtctcctcttgg | SEQ ID NO:268 |
| | Do b 9 cr | accagtctcctcttgggtgg | SEQ ID NO:269 |
| | Do b 10 cr | cagtctcctcttgggtggt | SEQ ID NO:270 |

| Dombrock-Joseph(a) positive allele: | | | |
|---|---|---|---|
| | Jo.a.pos.1 | ccccagaacatgactaccac | SEQ ID NO:271 |
| | Jo.a.pos.2 | ccagaacatgactaccacaca | SEQ ID NO:272 |
| | Jo.a.pos.3 | agaacatgactaccacacacgc | SEQ ID NO:273 |
| | Jo.a.pos.4 | catgactaccacacacgctgt | SEQ ID NO:274 |
| | Jo.a.pos.5 | actaccacacacgctgtgg | SEQ ID NO:275 |
| | Jo.a.pos.1.cr | gccacagcgtgtgtggtagt | SEQ ID NO:276 |
| | Jo.a.pos.2.cr | acagcgtgtgtggtagtcatg | SEQ ID NO:277 |
| | Jo.a.pos.3.cr | agcgtgtgtggtagtcatgtt | SEQ ID NO:278 |
| | Jo.a.pos.4.cr | cgtgtgtggtagtcatgttctg | SEQ ID NO:279 |
| | Jo.a.pos.5.cr | gtggtagtcatgttctgggg | SEQ ID NO:280 |

| Dombrock-Joseph(a) negative allele: | | | |
|---|---|---|---|
| | Jo.a.neg.1 | ctaccccagaacatgactatcac | SEQ ID NO:281 |
| | Jo.a.neg.2 | cccagaacatgactatcacaca | SEQ ID NO:282 |
| | Jo.a.neg.3 | cagaacatgactatcacacacgc | SEQ ID NO:283 |
| | Jo.a.neg.4 | catgactatcacacacgctgtg | SEQ ID NO:284 |
| | Jo.a.neg.5 | actatcacacacgctgtggc | SEQ ID NO:285 |
| | Jo.a.neg.1.cr | aatagccacagcgtgtgtgatagt | SEQ ID NO:286 |
| | Jo.a.neg.2.cr | cacagcgtgtgtgatagtcatg | SEQ ID NO:287 |
| | Jo.a.neg.3.cr | cagcgtgtgtgatagtcatgtt | SEQ ID NO:288 |
| | Jo.a.neg.4.cr | cgtgtgtgatagtcatgttctgg | SEQ ID NO:289 |
| | Jo.a.neg.5.cr | gtgatagtcatgttctggggtag | SEQ ID NO:290 |

| Duffy-a-allele: | | | |
|---|---|---|---|
| | Fy.A.1 | ccagatggagactatggtgcc | SEQ ID NO:291 |
| | Fy.A.2 | atggagactatggtgccaac | SEQ ID NO:292 |
| | Fy.A.3 | ggagactatggtgccaacctg | SEQ ID NO:293 |
| | Fy.A.4 | gactatggtgccaacctgga | SEQ ID NO:294 |
| | Fy.A.5 | tatggtgccaacctggaag | SEQ ID NO:295 |
| | Fy.A.1.cr | cttccaggttggcaccata | SEQ ID NO:296 |
| | Fy.A.2.cr | tccaggttggcaccatagtc | SEQ ID NO:297 |
| | Fy.A.3.cr | aggttggcaccatagtctcc | SEQ ID NO:298 |
| | Fy.A.4.cr | gttggcaccatagtctccat | SEQ ID NO:299 |
| | Fy.A.5.cr | gcaccatagtctccatctgg | SEQ ID NO:300 |

| Duffy-b-allele: | | | |
|---|---|---|---|
| | Fy.B.1 | cccagatggagactatgatgcc | SEQ ID NO:301 |
| | Fy.B.2 | gatggagactatgatgccaac | SEQ ID NO:302 |
| | Fy.B.3 | tggagactatgatgccaacctg | SEQ ID NO:303 |
| | Fy.B.4 | gactatgatgccaacctggaa | SEQ ID NO:304 |
| | Fy.B.5 | tatgatgccaacctggaagc | SEQ ID NO:305 |
| | Fy.B.1.cr | gcttccaggttggcatcata | SEQ ID NO:306 |
| | Fy.B.2.cr | ttccaggttggcatcatagtc | SEQ ID NO:307 |
| | Fy.B.3.cr | caggttggcatcatagtctcc | SEQ ID NO:308 |
| | Fy.B.4.cr | gttggcatcatagtctccatc | SEQ ID NO:309 |
| | Fy.B.5.cr | gcatcatagtctccatctggg | SEQ ID NO:310 |

| Duffy GATAbox-normal-allele: | | | |
|---|---|---|---|
| | Fy.GATA.normal.1 | agtccttggctcttatcttg | SEQ ID NO:311 |
| | Fy.GATA.normal.2 | agtccttggctcttatcttgga | SEQ ID NO:312 |
| | Fy.GATA.normal.3 | cttggctcttatcttggaagc | SEQ ID NO:313 |
| | Fy.GATA.normal.4 | gctcttatcttggaagcacagg | SEQ ID NO:314 |
| | Fy.GATA.normal.5 | cttatcttggaagcacaggcgc | SEQ ID NO:315 |
| | Fy.GATA.normal.1.cr | gcctgtgcttccaagataag | SEQ ID NO:316 |
| | Fy.GATA.normal.2.cr | tgtgcttccaagataagagc | SEQ ID NO:317 |
| | Fy.GATA.normal.3.cr | tgcttccaagataagagcca | SEQ ID NO:318 |
| | Fy.GATA.normal.4.cr | ttccaagataagagccaagga | SEQ ID NO:319 |
| | Fy.GATA.normal.5.cr | caagataagagccaaggact | SEQ ID NO:320 |

| Duffy GATAbox-mutation-allele: | | | |
|---|---|---|---|
| | Fy.GATA.mut.1 | tccttggctcttaccttg | SEQ ID NO:321 |
| | Fy.GATA.mut.2 | tccttggctcttaccttgga | SEQ ID NO:322 |
| | Fy.GATA.mut.3 | tggctcttaccttggaagc | SEQ ID NO:323 |
| | Fy.GATA.mut.4 | gctcttaccttggaagcacag | SEQ ID NO:324 |
| | Fy.GATA.mut.5 | cttaccttggaagcacaggcg | SEQ ID NO:325 |
| | Fy.GATA.mut.1.cr | cctgtgcttccaaggtaag | SEQ ID NO:326 |
| | Fy.GATA.mut.2.cr | gtgcttccaaggtaagagc | SEQ ID NO:327 |
| | Fy.GATA.mut.3.cr | gcttccaaggtaagagcca | SEQ ID NO:328 |
| | Fy.GATA.mut.4.cr | ttccaaggtaagagccaagg | SEQ ID NO:329 |
| | Fy.GATA.mut.5.cr | caaggtaagagccaagga | SEQ ID NO:330 |

| Duffy Fyx-normal-allele: | | | |
|---|---|---|---|
| | Fy.X.(b).1 | ttttcagacctctcttccgct | SEQ ID NO:331 |
| | Fy.X.(b).2 | agacctctcttccgctggc | SEQ ID NO:332 |
| | Fy.X.(b).3 | ctctcttccgctggcagc | SEQ ID NO:333 |
| | Fy.X.(b).4 | ctcttccgctggcagctc | SEQ ID NO:334 |
| | Fy.X.(b).5 | cttccgctggcagctctg | SEQ ID NO:335 |
| | Fy.X.(b).1.cr | cagagctgccagcggaa | SEQ ID NO:336 |
| | Fy.X.(b).2.cr | agctgccagcggaagag | SEQ ID NO:337 |
| | Fy.X.(b).3.cr | ctgccagcggaagagagg | SEQ ID NO:338 |
| | Fy.X.(b).4.cr | gccagcggaagagaggtc | SEQ ID NO:339 |
| | Fy.X.(b).5.cr | cagcggaagagaggtctg | SEQ ID NO:340 |

| Duffy Fyx-mutation-allele: | | | |
|---|---|---|---|
| | Fy.X.1 | gcttttcagacctctcttctgct | SEQ ID NO:341 |
| | Fy.X.2 | tcagacctctcttctgctggc | SEQ ID NO:342 |
| | Fy.X.3 | acctctcttctgctggcagc | SEQ ID NO:343 |
| | Fy.X.4 | ctcttctgctggcagctctg | SEQ ID NO:344 |
| | Fy.X.5 | cttctgctggcagctctgc | SEQ ID NO:345 |
| | Fy.X.1.cr | gcagagctgccagcagaa | SEQ ID NO:346 |
| | Fy.X.2.cr | gagctgccagcagaagagag | SEQ ID NO:347 |
| | Fy.X.3.cr | agctgccagcagaagagagg | SEQ ID NO:348 |
| | Fy.X.4.cr | gccagcagaagagaggtctg | SEQ ID NO:349 |
| | Fy.X.5.cr | cagcagaagagaggtctgaaa | SEQ ID NO:350 |

| Kidd-allele-a: | | | |
|---|---|---|---|
| | Jk.a.1 | cagccccatttgaggaca | SEQ ID NO:351 |
| | Jk.a.2 | gccccatttgaggacatcta | SEQ ID NO:352 |
| | Jk.a.3 | ccatttgaggacatctactttg | SEQ ID NO:353 |
| | Jk.a.4 | atttgaggacatctactttgga | SEQ ID NO:354 |
| | Jk.a.5 | gaggacatctactttggactct | SEQ ID NO:355 |
| | Jk.a.1.cr | cagagtccaaagtagatgtcctc | SEQ ID NO:356 |
| | Jk.a.2.cr | agtccaaagtagatgtcctcaaa | SEQ ID NO:357 |
| | Jk.a.3.cr | aaagtagatgtcctcaaatggg | SEQ ID NO:358 |
| | Jk.a.4.cr | tagatgtcctcaaatggggc | SEQ ID NO:359 |
| | Jk.a.5.cr | atgtcctcaaatggggctg | SEQ ID NO:360 |

| Kidd-allele-b: | | | |
|---|---|---|---|
| | Jk.b.1 | tcagccccatttgagaaca | SEQ ID NO:361 |
| | Jk.b.2 | gccccatttgagaacatcta | SEQ ID NO:362 |
| | Jk.b.3 | cccatttgagaacatctactttg | SEQ ID NO:363 |
| | Jk.b.4 | atttgagaacatctactttggac | SEQ ID NO:364 |
| | Jk.b.5 | gagaacatctactttggactctg | SEQ ID NO:365 |
| | Jk.b.1.cr | ccagagtccaaagtagatgttctc | SEQ ID NO:366 |
| | Jk.b.2.cr | agagtccaaagtagatgttctcaaa | SEQ ID NO:367 |
| | Jk.b.3.cr | ccaaagtagatgttctcaaatgg | SEQ ID NO:368 |
| | Jk.b.4.cr | agtagatgttctcaaatggggc | SEQ ID NO:369 |
| | Jk.b.5.cr | atgttctcaaatggggctga | SEQ ID NO:370 |

| Kell-K1-allele: | | | |
|---|---|---|---|
| | KEL.1.1 | tccttaaactttaaccgaatgct | SEQ ID NO:371 |
| | KEL.1.2 | ttaaactttaaccgaatgctgaga | SEQ ID NO:372 |
| | KEL.1.3 | aactttaaccgaatgctgagactt | SEQ ID NO:373 |
| | KEL.1.4 | aaccgaatgctgagacttctg | SEQ ID NO:374 |
| | KEL.1.5 | cgaatgctgagacttctgatgag | SEQ ID NO:375 |
| | KEL 1.6 CR | actcatcagaagtctcagcattc | SEQ ID NO:376 |
| | KEL 1.7 CR | tcagaagtctcagcattcggt | SEQ ID NO:377 |
| | KEL 1.8 CR | aagtctcagcattcggttaaag | SEQ ID NO:378 |
| | KEL 1.9 CR | tctcagcattcggttaaagtttaa | SEQ ID NO:379 |
| | KEL 1.10 CR | agcattcggttaaagtttaagga | SEQ ID NO:380 |

| Kell-K2-allele: | | | |
|---|---|---|---|
| | KEL.2.1 | ccttaaactttaaccgaacgct | SEQ ID NO:381 |
| | KEL.2.2 | aactttaaccgaacgctgaga | SEQ ID NO:382 |
| | KEL.2.3 | ctttaaccgaacgctgagactt | SEQ ID NO:383 |
| | KEL.2.4 | aaccgaacgctgagacttct | SEQ ID NO:384 |
| | KEL.2.5 | cgaacgctgagacttctgatg | SEQ ID NO:385 |
| | KEL 2.6 CR | tcatcagaagtctcagcgttc | SEQ ID NO:386 |
| | KEL 2.7 CR | cagaagtctcagcgttcggt | SEQ ID NO:387 |
| | KEL 2.8 CR | agtctcagcgttcggttaaag | SEQ ID NO:388 |
| | KEL 2.9 CR | tctcagcgttcggttaaagtt | SEQ ID NO:389 |
| | KEL 2.10 CR | agcgttcggttaaagtttaagg | SEQ ID NO:390 |

| Kell-K3-allele: | | | |
|---|---|---|---|
| | KEL.3.1 | aatctccatcacttcatggct | SEQ ID NO:391 |
| | KEL.3.2 | tccatcacttcatggctgtt | SEQ ID NO:392 |
| | KEL.3.3 | atcacttcatggctgttcca | SEQ ID NO:393 |
| | KEL.3.4 | acttcatggctgttccagtt | SEQ ID NO:394 |
| | KEL.3.5 | tcatggctgttccagtttc | SEQ ID NO:395 |
| | KEL.3.1.cr | agaaactggaacagccatgaa | SEQ ID NO:396 |
| | KEL.3.2.cr | aactggaacagccatgaagtg | SEQ ID NO:397 |
| | KEL.3.3.cr | tggaacagccatgaagtgatg | SEQ ID NO:398 |
| | KEL.3.4.cr | acagccatgaagtgatggag | SEQ ID NO:399 |
| | KEL.3.5.cr | gccatgaagtgatggagatt | SEQ ID NO:400 |

| Kell-K4-allele: | | | |
|---|---|---|---|
| | KEL.4.1 | tctccatcacttcacggct | SEQ ID NO:401 |
| | KEL.4.2 | ccatcacttcacggctgtt | SEQ ID NO:402 |
| | KEL.4.3 | cacttcacggctgttcca | SEQ ID NO:403 |
| | KEL.4.4 | acttcacggctgttccag | SEQ ID NO:404 |
| | KEL.4.5 | tcacggctgttccagttt | SEQ ID NO:405 |
| | KEL.4.1.cr | aaactggaacagccgtgaa | SEQ ID NO:406 |
| | KEL.4.2.cr | ctggaacagccgtgaagtg | SEQ ID NO:407 |
| | KEL.4.3.cr | ggaacagccgtgaagtgatg | SEQ ID NO:408 |
| | KEL.4.4.cr | acagccgtgaagtgatgg | SEQ ID NO:409 |
| | KEL.4.S.cr | gccgtgaagtgatggaga | SEQ ID NO:410 |

| Kell-K6-allele: | | | |
|---|---|---|---|
| | KEL.6.1 | tactgcctgggggctgccccgcc | SEQ ID NO:411 |
| | KEL.6.2 | tgggggctgccccgcctgt | SEQ ID NO:412 |
| | KEL.6.3 | gctgccccgcctgtgac | SEQ ID NO:413 |
| | KEL.6.4 | ctgccccgcctgtgacaa | SEQ ID NO:414 |
| | KEL.6.5 | gccccgcctgtgacaac | SEQ ID NO:415 |
| | KEL.6.1.cr | gttgtcacaggcggggc | SEQ ID NO:416 |
| | KEL.6.2.cr | ttgtcacaggcggggcag | SEQ ID NO:417 |
| | KEL.6.3.cr | tcacaggcggggcagccc | SEQ ID NO:418 |
| | KEL.6.4.cr | acaggcggggcagccccca | SEQ ID NO:419 |
| | KEL.6.5.cr | aggcggggcagccccca | SEQ ID NO:420 |

| Kell-K7-allele: | | | |
|---|---|---|---|
| | KEL.7.1 | actgcctgggggctgcctcgcc | SEQ ID NO:421 |
| | KEL.7.2 | cctgggggctgcctcgcctgt | SEQ ID NO:422 |
| | KEL.7.3 | ggctgcctcgcctgtgac | SEQ ID NO:423 |
| | KEL.7.4 | ctgcctcgcctgtgacaacc | SEQ ID NO:424 |
| | KEL.7.5 | gcctcgcctgtgacaacc | SEQ ID NO:425 |
| | KEL.7.1.cr | ggttgtcacaggcgaggc | SEQ ID NO:426 |
| | KEL.7.2.cr | ggttgtcacaggcgaggcag | SEQ ID NO:427 |
| | KEL.7.3.cr | ttgtcacaggcgaggcagccc | SEQ ID NO:428 |
| | KEL.7.4.cr | acaggcgaggcagcccccagg | SEQ ID NO:429 |
| | KEL.7.5.cr | aggcgaggcagcccccagg | SEQ ID NO:430 |

| Lutheran a-allele: | | | |
|---|---|---|---|
| | Lu.a.1 | ggagctcgcccccgcct | SEQ ID NO:431 |
| | Lu.a.2 | gctcgcccccgcctagc | SEQ ID NO:432 |
| | Lu.a.3 | cgcccccgcctagcctc | SEQ ID NO:433 |
| | Lu.a.4 | cccccgcctagcctcgg | SEQ ID NO:434 |
| | Lu.a.5 | cccgcctagcctcggct | SEQ ID NO:435 |
| | Lu.a.1 CR | agccgaggctaggcggg | SEQ ID NO:436 |
| | Lu.a.2 CR | ccgaggctaggcggggg | SEQ ID NO:437 |
| | Lu.a.3 CR | gaggctaggcgggggcg | SEQ ID NO:438 |
| | Lu.a.4 CR | gctaggcgggggcgagc | SEQ ID NO:439 |
| | Lu.a.5 CR | aggcgggggcgagctcc | SEQ ID NO:440 |

| Lutheran b-allele: | | | |
|---|---|---|---|
| | Lu.b.1 | gggagctcgcccccacct | SEQ ID NO:441 |
| | Lu.b.2 | gagctcgcccccacctagc | SEQ ID NO:442 |
| | Lu.b.3 | ctcgcccccacctagcctc | SEQ ID NO:443 |
| | Lu.b.4 | cccccacctagcctcggct | SEQ ID NO:444 |
| | Lu.b.5 | cccacctagcctcggctga | SEQ ID NO:445 |
| | Lu.b.1 CR | tcagccgaggctaggtggg | SEQ ID NO:446 |
| | Lu.b.2 CR | agccgaggctaggtggggg | SEQ ID NO:447 |
| | Lu.b.3 CR | gaggctaggtgggggcgag | SEQ ID NO:448 |
| | Lu.b.4 CR | gctaggtgggggcgagctc | SEQ ID NO:449 |
| | Lu.b.5 CR | aggtgggggcgagctccc | SEQ ID NO:450 |

| MNS M-allele: | | | |
|---|---|---|---|
| | M.1 | gtgagcatatcagcatcaag | SEQ ID NO:451 |
| | M.2 | atcagcatcaagtaccactgg | SEQ ID NO:452 |
| | M.3 | catcaagtaccactggtgtg | SEQ ID NO:453 |
| | M.4 | taccactggtgtggcaatgc | SEQ ID NO:454 |
| | M.5 | ctggtgtggcaatgcaca | SEQ ID NO:455 |
| | M.1.cr | tgtgcattgccacaccagt | SEQ ID NO:456 |
| | M.2.cr | gcattgccacaccagtggta | SEQ ID NO:457 |
| | M.3.cr | ccacaccagtggtacttgatg | SEQ ID NO:458 |
| | M.4.cr | accagtggtacttgatgct | SEQ ID NO:459 |
| | M.5.cr | cttgatgctgatatgctcac | SEQ ID NO:460 |

| MNS N-allele: | | | |
|---|---|---|---|
| | N.1 | tgtgagcatatcagcattaag | SEQ ID NO:461 |
| | N.2 | atcagcattaagtaccactgagg | SEQ ID NO:462 |
| | N.3 | cattaagtaccactgaggtgg | SEQ ID NO:463 |
| | N.4 | accactgaggtggcaatgc | SEQ ID NO:464 |
| | N.5 | ctgaggtggcaatgcacact | SEQ ID NO:465 |
| | N.1.cr | gtgtgcattgccacctcagt | SEQ ID NO:466 |
| | N.2.cr | gcattgccacctcagtggta | SEQ ID NO:467 |
| | N.3.cr | ccacctcagtggtacttaatgc | SEQ ID NO:468 |
| | N.4.cr | cctcagtggtacttaatgct | SEQ ID NO:469 |
| | N.5.cr | cttaatgctgatatgctcaca | SEQ ID NO:470 |

| MNS S-allele: | | | |
|---|---|---|---|
| | big.S.1 | tttgctttataggagaaatggga | SEQ ID NO:471 |
| | big.S.2 | ctttataggagaaatgggaca | SEQ ID NO:472 |
| | big.S.3 | ttataggagaaatgggacaacttg | SEQ ID NO:473 |
| | big.S.4 | gagaaatgggacaacttgtcc | SEQ ID NO:474 |
| | big.S.5 | aaatgggacaacttgtccatc | SEQ ID NO:475 |
| | big.S.1.cr | gatggacaagttgtcccattt | SEQ ID NO:476 |
| | big.S.2.cr | gacaagttgtcccatttctcc | SEQ ID NO:477 |
| | big.S.3.cr | aagttgtcccatttctcctata | SEQ ID NO:478 |
| | bigS.4.cr | tgtcccatttctcctataaagca | SEQ ID NO:479 |
| | big.S.5.cr | cccatttctcctataaagcaaaa | SEQ ID NO:480 |

| MNS s-allele: | | | |
|---|---|---|---|
| | little.s.1 | tgctttataggagaaacggga | SEQ ID NO:481 |
| | little.s.2 | tttataggagaaacgggaca | SEQ ID NO:482 |
| | little.s.3 | ggagaaacgggacaacttg | SEQ ID NO:483 |
| | little.s.4 | gagaaacgggacaacttgtc | SEQ ID NO:484 |
| | little.s.5 | aaacgggacaacttgtccat | SEQ ID NO:485 |
| | little.s.1.cr | tggacaagttgtcccgttt | SEQ ID NO:486 |
| | little.s.2.cr | acaagttgtcccgtttctcc | SEQ ID NO:487 |
| | little.s.3.cr | agttgtcccgtttctcctata | SEQ ID NO:488 |
| | little.s.4.cr | tgtcccgtttctcctataaagc | SEQ ID NO:489 |
| | little.s.5.cr | cccgtttctcctataaagca | SEQ ID NO:490 |

| MNS U-positive-allele: | | | |
|---|---|---|---|
| | U.pos.1 | ttgctgctctctttagctcc | SEQ ID NO:491 |
| | U.pos.2 | ctctctttagctcctgtagtgat | SEQ ID NO:492 |
| | U.pos.3 | agctcctgtagtgataatactca | SEQ ID NO:493 |
| | U.pos.4 | gtagtgataatactcattatttttg | SEQ ID NO:494 |
| | U.pos.5 | taatactcattatttttggggtg | SEQ ID NO:495 |
| | U.pos.1.cr | caccccaaaaataatgagtatta | SEQ ID NO:496 |
| | U.pos.2.cr | caaaaataatgagtattatcactaca | SEQ ID NO:497 |
| | U.pos.3.cr | agtattatcactacaggagctaaa | SEQ ID NO:498 |
| | U.pos.4.cr | atcactacaggagctaaagag | SEQ ID NO:499 |
| | U.pos.5.cr | gagctaaagagagcagcaaa | SEQ ID NO:500 |

| MNS U-negative-allele: | | | |
|---|---|---|---|
| | U.neg.1 | ttttgctgctctctttatctcc | SEQ ID NO:501 |
| | U.neg.2 | gctctctttatctcctgtagagat | SEQ ID NO:502 |
| | U.neg.3 | tatctcctgtagagataacactca | SEQ ID NO:503 |
| | U.neg.4 | gtagagataacactcattattttt | SEQ ID NO:504 |
| | U.neg.5 | taacactcattatttttggggt | SEQ ID NO:505 |
| | U.neg.1.cr | accccaaaaataatgagtgtta | SEQ ID NO:506 |
| | U.neg.2.cr | aaaaataatgagtgttatctctaca | SEQ ID NO:507 |
| | U.neg.3.cr | agtgttatctctacaggagataaa | SEQ ID NO:508 |
| | U.neg.4.cr | atctctacaggagataaagagag | SEQ ID NO:509 |
| | U.neg.5.cr | gagataaagagagcagcaaaatta | SEQ ID NO:510 |

| Rhesus C-allele(307T): | | | |
|---|---|---|---|
| | Rh.big.C.1 | tgagccagttcccttctgg | SEQ ID NO:511 |
| | Rh.big.C.2 | gagccagttcccttctgg | SEQ ID NO:512 |
| | Rh.big.C.3 | ctgagccagttcccttctg | SEQ ID NO:513 |
| | Rh.big.C.4 | ccttctgggaaggtggtc | SEQ ID NO:514 |
| | Rh.big.C.5 | ccttctgggaaggtggtca | SEQ ID NO:515 |
| | Rh.big.C.1.cr | tgaccaccttcccagaagg | SEQ ID NO:516 |
| | Rh.big.C.2.cr | gaccaccttcccagaagg | SEQ ID NO:517 |
| | Rh.big.C.3.cr | ccagaagggaactggctc | SEQ ID NO:518 |
| | Rh.big.C.4.cr | ccagaagggaactggctca | SEQ ID NO:519 |
| | Rh.big.C.5.cr | cagaagggaactggctcag | SEQ ID NO:520 |

| Rhesus c-allele(307C): | | | |
|---|---|---|---|
| | Rh.little.c.1 | gagccagttccctcctgg | SEQ ID NO:521 |
| | Rh.little.c.2 | agccagttccctcctgg | SEQ ID NO:522 |
| | Rh.little.c.3 | tgagccagttccctcctg | SEQ ID NO:523 |
| | Rh.little.c.4 | cctcctgggaaggtggt | SEQ ID NO:524 |
| | Rh.little.c.5 | cctcctgggaaggtggtc | SEQ ID NO:525 |
| | Rh.little.c.1.cr | gaccaccttcccaggagg | SEQ ID NO:526 |
| | Rh.little.c.2.cr | accaccttcccaggagg | SEQ ID NO:527 |
| | Rh.little.c.3.cr | ccaggagggaactggct | SEQ ID NO:528 |
| | Rh.little.c.4.cr | ccaggagggaactggctc | SEQ ID NO:529 |
| | Rh.little.c.5.cr | caggagggaactggctca | SEQ ID NO:530 |

| Rhesus BigC-intron2-speciitc-insert-allele: | | | |
|---|---|---|---|
| | RhC.intron.2.1 | agggtgccctttgtcacttc | SEQ ID NO:531 |
| | RhC.intron.2.2 | gccctttgtcacttcccagt | SEQ ID NO:532 |
| | RhC.intron.2.3 | cctttgtcacttcccagtgg | SEQ ID NO:533 |
| | RhC.intron.2.4 | ttgtcacttcccagtggtacaa | SEQ ID NO:534 |
| | RhC.intron.2.5 | tcacttcccagtggtacaatca | SEQ ID NO:535 |
| | RhC.intron.2.1.cr | gaagtgacaaagggcaccct | SEQ ID NO:536 |
| | RhC.intron.2.2.cr | actgggaagtgacaaagggc | SEQ ID NO:537 |
| | RhC.intron.2.3.cr | ccactgggaagtgacaaagg | SEQ ID NO:538 |
| | RhC.intron.2.4.cr | tgtaccactgggaagtgacaaa | SEQ ID NO:539 |
| | RhC.intron.2.5.cr | tgattgtaccactgggaagtga | SEQ ID NO:540 |

### Rhesus BigC-intron2-specific-insert-negative-allele:

The setup of the PCR is chosen in such a way that a PCR product will be formed only, when the bigC-intron2-specific-insert is present. The antitags (used for background subtraction) are used to calculate the ratio for genotyping.

| Rhesus E-allele: | | | |
|---|---|---|---|
| | Rh.big.E.1 | gccaagtgtcaactctcctct | SEQ ID NO:541 |
| | Rh.big.E.2 | caagtgtcaactctcctctgct | SEQ ID NO:542 |
| | Rh.big.E.3 | gtgtcaactctcctctgctgag | SEQ ID NO:543 |
| | Rh.big.E.4 | caactctcctctgctgagaagtc | SEQ ID NO:544 |
| | Rh.big.E.5 | tctcctctgctgagaagtcc | SEQ ID NO:545 |
| | Rh.big.E.1.cr | ggacttctcagcagaggagag | SEQ ID NO:546 |
| | Rh.big.E.2.cr | cttctcagcagaggagagttga | SEQ ID NO:547 |
| | Rh.big.E.3.cr | ctcagcagaggagagttgacac | SEQ ID NO:548 |
| | Rh.big.E.4.cr | gcagaggagagttgacacttg | SEQ ID NO:549 |
| | Rh.big.E.5.cr | gaggagagttgacacttggc | SEQ ID NO:550 |

| Rhesus e-allele: | | | |
|---|---|---|---|
| | Rh.little.e.1 | gccaagtgtcaactctgctct | SEQ ID NO:551 |
| | Rh.little.e.2 | caagtgtcaactctgctctgct | SEQ ID NO:552 |
| | Rh.little.e.3 | tgtcaactctgctctgctgag | SEQ ID NO:553 |
| | Rh.little.e.4 | caactctgctctgctgagaag | SEQ ID NO:554 |
| | Rh.little.e.5 | tctgctctgctgagaagtcc | SEQ ID NO:555 |
| | Rh.little.e.1.cr | ggacttctcagcagagcagag | SEQ ID NO:556 |
| | Rh.little.e.2.cr | ttctcagcagagcagagttga | SEQ ID NO:557 |
| | Rh.little.e.3.cr | tcagcagagcagagttgacac | SEQ ID NO:558 |
| | Rh.little.e.4.cr | gcagagcagagttgacacttg | SEQ ID NO:559 |
| | Rh.little.e.5.cr | gagcagagttgacacttggc | SEQ ID NO:560 |

| Rhesus RHD-allele: | | | |
|---|---|---|---|
| | RhD 1 | ttccccacagctccatcat | SEQ ID NO:561 |
| | RhD 2 | acagctccatcatgggctac | SEQ ID NO:562 |
| | RhD 3 | tccatcatgggctacaacttc | SEQ ID NO:563 |
| | RhD 4 | tcatgggctacaacttcagct | SEQ ID NO:564 |
| | RhD 5 | gggctacaacttcagcttgct | SEQ ID NO:565 |
| | RhD cr 1 | agcaagctgaagttgtagccc | SEQ ID NO:566 |
| | RhD cr 2 | agctgaagttgtagcccatga | SEQ ID NO:567 |
| | RhD cr 3 | gaagttgtagcccatgatgg | SEQ ID NO:568 |
| | RhD cr 4 | gcccatgatggagctgt | SEQ ID NO:569 |
| | RhD cr 5 | tgatggagctgtggggaa | SEQ ID NO:570 |

### Rhesus RHD-negative-allele:

The setup of the PCR is chosen in such a way that a PCR product will be formed only, when the RHD gene is present. The antitags (used for background subtraction) are used to calculate the ratio for genotyping.

| Rhesus r's-allele: | | |
|---|---|---|
| r's.T.1 | ggaaggtcaacttggtgca | SEQ ID NO:571 |
| r's.T.2 | ggaaggtcaacttggtgcagt | SEQ ID NO:572 |
| r's.T.3 | caacttggtgcagttggtg | SEQ ID NO:573 |
| r's.T.4 | acttggtgcagttggtggt | SEQ ID NO:574 |
| r's.T.5 | ttggtgcagttggtggtgat | SEQ ID NO:575 |
| r's.T.1.cr | catcaccaccaactgcacca | SEQ ID NO:576 |
| r's.T.2.cr | caccaccaactgcaccaagt | SEQ ID NO:577 |
| r's.T.3.cr | accaactgcaccaagttgac | SEQ ID NO:578 |
| r's.T.4.cr | actgcaccaagttgaccttcc | SEQ ID NO:579 |
| r's.T.5.cr | tgcaccaagttgaccttcc | SEQ ID NO:580 |

### Rhesus r's-negative-allele:

The setup of the PCR is chosen in such a way that a PCR product will be formed only, when the r's gene is present. The antitags (used for background subtraction) are used to calculate the ratio for genotyping.

### Rhesus DVI-allele:

The setup of the PCR is chosen in such a way that a PCR product will be formed only, when a normal RHD gene is present. The antitags (used for background subtraction) are used to calculate the ratio for genotyping.

| DVI-probes for presence normal RHD allele: | | | |
|---|---|---|---|
| | Rh.DVI.1 | atttcaaccctcttggcctt | SEQ ID NO:581 |
| | Rh.DVI.2 | aaccctcttggcctttgttt | SEQ ID NO:582 |
| | Rh.DVI.3 | tcttggcctttgtttccttg | SEQ ID NO:583 |
| | Rh.DVI.4 | ggtatcagcttgagagctcg | SEQ ID NO:584 |
| | Rh.DVI.5 | atcagcttgagagctcggag | SEQ ID NO:585 |
| | Rh.DVI.1.cr | aaggccaagagggttgaaat | SEQ ID NO:586 |
| | Rh.DVI.2.cr | aaacaaaggccaagagggtt | SEQ ID NO:587 |
| | Rh.DVI.3.cr | caaggaaacaaaggccaaga | SEQ ID NO:588 |
| | Rh.DVI.4.cr | cgagctctcaagctgatacc | SEQ ID NO:589 |
| | Rh.DVI.5.cr | ctccgagctctcaagctgat | SEQ ID NO:590 |

| Rhesus RHD-Pseudogene-mutation-allele: | | | |
|---|---|---|---|
| | RhD Y 1 | tttctttgcagacttaggtgc | SEQ ID NO:591 |
| | RhD Y 2 | ctttgcagacttaggtgcaca | SEQ ID NO:592 |
| | RhD Y 3 | tttgcagacttaggtgcacagt | SEQ ID NO:593 |
| | RhD Y 4 | acttaggtgcacagtgcgg | SEQ ID NO:594 |
| | RhD Y 5 | cttaggtgcacagtgcggt | SEQ ID NO:595 |
| | RhD Y cr 1 | caccgcactgtgcacctaa | SEQ ID NO:596 |
| | RhD Y cr 2 | ccgcactgtgcacctaagtc | SEQ ID NO:597 |
| | RhD Y cr 3 | gcactgtgcacctaagtctgc | SEQ ID NO:598 |
| | RhD Y cr 4 | tgcacctaagtctgcaaaga | SEQ ID NO:599 |
| | RhD Y cr 5 | cacctaagtctgcaaagaaatagcg | SEQ ID NO:600 |

| Rhesus RHD-Pseudogene-normal-allele: | | | |
|---|---|---|---|
| | RhD non Y 1 | ctatttctttgcagacttatgtgc | SEQ ID NO:601 |
| | RhD non Y 2 | tctttgcagacttatgtgcaca | SEQ ID NO:602 |
| | RhD non Y 3 | ttgcagacttatgtgcacagtg | SEQ ID NO:603 |
| | RhD non Y 4 | acttatgtgcacagtgcggt | SEQ ID NO:604 |
| | RhD non Y 5 | cttatgtgcacagtgcggtg | SEQ ID NO:605 |
| | RhD non Y cr 1 | acaccgcactgtgcacataa | SEQ ID NO:606 |
| | RhD non Y cr 2 | accgcactgtgcacataagtc | SEQ ID NO:607 |
| | RhD non Y cr 3 | gcactgtgcacataagtctgc | SEQ ID NO:608 |
| | RhD non Y cr 4 | tgtgcacataagtctgcaaag | SEQ ID NO:609 |
| | RhD non Y cr 5 | cacataagtctgcaaagaaatagcg | SEQ ID NO:610 |

| Yt-a-allele: | | | |
|---|---|---|---|
| | Yt.a.1 | gcgggagacttccacgg | SEQ ID NO:611 |
| | Yt.a.2 | gggagacttccacggcct | SEQ ID NO:612 |
| | Yt.a.3 | gagacttccacggcctgc | SEQ ID NO:613 |
| | Yt.a.4 | gacttccacggcctgca | SEQ ID NO:614 |
| | Yt.a.5 | ttccacggcctgcaggta | SEQ ID NO:615 |
| | Yt.a.1 CR | tacctgcaggccgtggaa | SEQ ID NO:616 |
| | Yt.a.2 CR | tgcaggccgtggaagtc | SEQ ID NO:617 |
| | Yt.a.3 CR | gcaggccgtggaagtctc | SEQ ID NO:618 |
| | Yt.a.4 CR | aggccgtggaagtctccc | SEQ ID NO:619 |
| | Yt.a.5 CR | ccgtggaagtctcccgc | SEQ ID NO:620 |

| Yt-b-allele: | | | |
|---|---|---|---|
| | Yt.b.1 | gcgggagacttcaacggc | SEQ ID NO:621 |
| | Yt.b.2 | gggagacttcaacggcctg | SEQ ID NO:622 |
| | Yt.b.3 | gagacttcaacggcctgca | SEQ ID NO:623 |
| | Yt.b.4 | gacttcaacggcctgcag | SEQ ID NO:624 |
| | Yt.b.5 | ttcaacggcctgcaggtaa | SEQ ID NO:625 |
| | Yt.b.1 CR | ttacctgcaggccgttgaa | SEQ ID NO:626 |
| | Yt.b.2 CR | ctgcaggccgttgaagtc | SEQ ID NO:627 |
| | Yt.b.3 CR | tgcaggccgttgaagtctc | SEQ ID NO:628 |
| | Yt.b.4 CR | caggccgttgaagtctccc | SEQ ID NO:629 |
| | Yt.b.5 CR | gccgttgaagtctcccgc | SEQ ID NO:630 |

Using the available software programs, it would be possible for a skilled person to design probes for other blood cell antigen alleles.

The DNA array may be a limited array comprising for example only 72 different probes, but preferably it comprises substantially all probes shown in the above list.

In order to facilitate binding of the probes to the array support and to allow sufficient access to the amplification products, the probe oligos will normally be provided with a linker molecule and a reactive group, preferably at the 5'-end of the oligo, for binding to the array support. All of this is well known to the skilled person and only by way of example it is mentioned that a suitable linker is C6 (hexamethylene, i.e. -(CH₂)₆-) and a suitable reactive group is amino.

### ARRAY SUPPORT

The probes are located on a suitable support to which they are usually attached by chemical means. The support may be a glass slide or any other suitable support, such as plastic, silicon or porous metal oxide. Usually, the support will be pretreated to improve binding of the oligos, for example pretreated by coating with poly-L-lysine, aminosilane, aldehyde or epoxy. To achieve binding of the probes, various techniques may be used, such as UV irradiation, nonspecific electrostatic absorption, covalent attachment via 5'amino or phosphate group using carbodiimide (EDC) chemistry, etc.

To avoid non-specific hybridization it will usually be preferable to incubate the array support carrying the probes thereon with prehybridization solution, as is well known to a skilled person, and to prepare the probes for hybridization it will be preferred to denature them, for example by heating at about 80°C or higher.

### CONTROLS

In order to allow correction for background it is preferred to include some probes with a sequence not occurring in the DNA of the donor and which may be expected not to bind by hybridization to amplification products. The signal measured for allele-specific probe spots may be corrected by subtracting the signal measured for these background control spots. Suitable examples of such background correction probes or antitags are the following probes a3, a9, a17, a23, a27, a33, a35, a38, a42 and a43:

| | | |
|---|---|---|
| a3 | cagaccataagcacaggcgt | SEQ ID NO:631, |
| a9 | gctcgtccacagtgcgttat | SEQ ID NO:632, |
| a17 | cggcgttcaagcaaaccgaa | SEQ ID NO:633, |
| a23 | gacatatagctccactcaga | SEQ ID NO:634, |
| a27 | tagggtactgatgagcactc | SEQ ID NO:635, |
| a33 | tcagccctatcgcaggatgt | SEQ ID NO:169, |
| a35 | gagacacttgacagtagcca | SEQ ID NO:170, |
| a38 | ggcagggcacctcagtttat | SEQ ID NO:171, |
| a42 | tcaccagccagactgtgtag | SEQ ID NO:172, |
| a43 | cttcacgcaagttgtccaga | SEQ ID NO:173. |

Further, it may be advisable to include at least one positive control probe. This probe should be the complement of a labeled positive control oligomer added to the products of the amplification. For example, the positive control probe may be the following oligo CS05:

| | | |
|---|---|---|
| CS05 | gtcctgacttctagctcatg | SEQ ID NO:174, |

which is capable of hybridizing to its labelled complement catgagctagaagtcaggac which is added to the mixture of amplification products before the mixture is applied onto the DNA array.

### HYBRIDIZATION CONDITIONS

Hybridization of products of the amplification to the probes in the DNA array is usually carried out at a temperature of between 50 and 65°C, preferably at about 56°C, for a sufficient time, such as for 15 minutes to 10 hours, more preferably for 30 minutes to 4 hours.

### ARRAY CONFIGURATION

The configuration of the array is fully arbitrary. Usually, one array support will carry a large number of spots present in several blocks that may be identical (preferred) or different from eachother. Each spot contains one probe and it is preferred that similar probes are spotted as distant from eachother as possible. It is preferable that each block contains one spot of each of the allele-specific probes, and in addition some control spots. Thus, one block may comprise 128 allele-specific spots, 5 background controls and a positive control, in total 134 spots.

### MEASURING THE FLUORESCENCE SIGNAL

The binding of labelled amplification products to certain spots is determined on the basis of the fluorescence signal emitted by these spots. The signal can be measured with a photomultiplier tube, scanning laser systems or any other apparatus and technique known to the skilled person.

### EVALUATION OF THE RESULTING DATA

To evaluate the resulting data as to the blood cell antigen genotype of the donor of the DNA, the fluorescence signal intensities may be converted using Genepix Pro 5.0 (Axon Instruments Inc.) to signal values which can be analyzed further, for example in Excel.

### EXAMPLES

In the Examples below, that only serve to illustrate the invention without limiting it in any way, the concept of genotyping blood cell antigens by the present combination of a particular multiplex PCR and a DNA microarray of allele-specific probes is tested in Example 1 on the biallelic Human Platelet Antigen systems HPA-1 through 5 and Gov. A blind panel of 58 donor samples was genotyped for these 6 HPA systems and only one discrepancy was found in one HPA system. The discrepancy can be overcome by adjusting the scoring criteria and validating the new format. Then, in Example 2, it was shown that the multiplex PCR of this invention worked well with all primers of Table II together.

### EXAMPLE 1

Sequences and Tm values of the probes spotted on poly-Lysine-coated glass slide are shown in Table I. The SNPs of HPA-3 and -5 are located in a GC-rich and -poor area, respectively. Therefore, these probes are shorter or longer than the other oligonucleotides, and have a Tm outside the 60-65°C range. The probes, dissolved at a concentration of 50 µM in 0.4 M NaHCO₃ (pH 9.4), were spotted on poly-L-lysine coated glass slides by the Omnigrid 100^{®} microarrayer (Genemachines) supplied with 16 SMP 3 Microspotting pins (Telechem). Each glass slide contains 48 blocks of 134 spots corresponding to the 128 allele-specific probes, the 5 background controls and the positive control CS05. Similar probes were spotted as distant as possible from each other. DNA was cross-linked by UV irradiation at 250 mJ/cm² (Stratalinker model 1800 UV Illuminator, Stratagene). To prevent non-specific hybridisation, the slides were incubated with 100 µl of prehybridisation solution [400 ng/µl yeast tRNA (Roche), 400 ng/µl herring sperm DNA (Gibco BRL), 5x Denhardt's solution, 3.2x SSC and 0.4% SDS] at 65°C for 30 min. Prior to hybridisation, the slide containing the prehybridisation mixture was incubated for 2 min at 80°C to denature the spotted DNA. After prehybridisation, the slides were washed twice in 2x SSC for 5 min at room temperature and dehydrated with a series of 70 (twice), 90 and 100% ethanol, for 5 min each, respectively.

A multiplex PCR for only the six platelet antigens was performed using 5 nM of each HPA and gov primer (7.5 nM of each HPA-3 primer) and 1.2 µM of each Cy5 labeled universal primer. Further, the multiplex PCR was performed as described for the multiplex PCR in Example 2. For hybridisation reaction chambers were made as described by Pastinen et al. (2000). A silicon rubber grid was used to divide the glass slide in 24 different compartments. While the slide was preheated (65°C for 75 min), 40 µl of the multiplex PCR products was denatured at 95°C for 5 min, put on ice and 120 µl of ice-cold hybridisation solution [2 ng Cy5-labelled complement of CS05 (5'-catgagctagaagtcaggac-3') and 4x SSC] was added. For hybridisation, 80 µl of this mixture was added per array and incubated for 3 ½ hours at 56°C. Next, slides were lifted out of the hybridisation holder in 600 ml 3x SSC and washed twice with 2x SSC / 0.1% SDS at 50°C and twice with 0.2x SSC at RT, for 5 min each, respectively. Glass slides were dried by centrifugation at 800 rpm for 5 min and scanned in an Agilent G2565BA micro array scanner at 10 µm resolution. Photomultiplier tube voltage was always set at 100%. PCR products of six different donors were analysed per slide as shown in Figure 3. After Genepix analysis the obtained data was converted to Excel. A panel of 12 donor samples with known HPA typing was used to define the HPA scoring criteria. Table III lists the scoring criteria. The median signal intensities F_{med} of the negative-controls (C) were averaged and this background value plus three times its standard deviation was subtracted from the median intensity F_{med} of each oligo, giving a threshold value, Fₜₕ, shown in the third column in Table III. If the Fₜₕ showed a negative value for both the matched (allele a) and the mismatched (allele b) probe, the primer pair was excluded for the genotyping result. For genotyping, the ratio of the intensities of two oligonucleotides (one from allele a and the other from allele b) in one set was calculated. The ratios were calculated of the F_{med}-F_{bg} intensities for the a-allele to the sum of the F_{med}-F_{bg} intensities for both alleles in the sixth column. This formula a/(a+b) has also been described by Hinds et al. (2004). In principle, these ratios should take on values near 1.0, 0.5 or 0.0 for aa, ab or bb genotypes. Only positive F_{med}-F_{bg} values were used and therefore negative values were set to 1 in the fifth column. Next, the ratios were converted to genotypes in the seventh column. For HPA-1, -2, -4 and -5 ratio values above 0.75 were typed aa, below 0.25 bb and between 0.35 and 0.65 ab. Due to a high binding of the PCR fragments to the probes representing the a allele, the criteria for HPA-3 and Gov were adjusted to 0.95 and 0.8 for aa, 0.45 and 0.35 for bb and for ab between 0.55 and 0.85 and between 0.45 and 0.7, respectively. The most frequent genotype was coloured orange in the seventh column. This was done for every blood group, every block and every sample. The samples of the 12 donors were used to accept probe-sets that correctly predicted the genotype. As criteria used for the final scoring: 50% of the used probe-sets should predict a genotype if a homozygote genotype was predicted and 40% if a heterozygote genotype was predicted (30% for HPA-3). Using these criteria a blind panel of 58 samples were genotyped. The results of nine of them are shown in Table IV. The genotypes of all 58 samples are listed in Table V. The genotypes of 56 samples could be scored with the preset criteria. Only in one sample a result discrepant with its known genotype was obtained. In this case, 33.3 % of the probe sets indicated an HPA-3bb genotype and 37.5% indicated an HPA-3ab genotype as shown in Table VI. Adjustment of the scoring criteria leads to complete concordance of the typing results.

### EXAMPLE 2

A primer mix was constructed with the primers listed in Table II. The concentration of the primers in the PCR mixture is also listed in Table II. The Qiagen multiplex kit was used for the multiplex PCR. In the multiplex PCR, the annealing temperature was not changed during PCR and two fluorescent labelled universal primers were used. In more detail, at the start the PCR mixture contains a very low amount of chimeric primers (5 nM) and an excess of fluorescent-labelled universal primers (0.2 µM of each universal primer per chimeric primer). The temperature profile of the PCR started with 15 min at 95°C, followed by 45 cycles of 94°C for 30 sec, 57°C for 90 sec, 72°C for 90 sec and the protocol ended with a final polymerization step at 72°C for 10 min. Very little amount of PCR product is amplified during the first amplification cycles, but enough to be used as template by the universal MAPH primers in the following cycles as can be seen on 8% acrylamide gel in Figure 1. By tagging the MAPH primers at their 5'end with the fluorescent label Cy5, only two fluorescent-labelled primers are necessary to label the PCR product efficiently. As described by White et al. (2002), a better discrmination of the multiplex PCR products can be visualized on an ABI 3700 capillary sequencer (Applied Biossystems), in this case only MAPH-rev is fluorescent (FAM) labeled. Multiplex PCR with all designed PCR primers yielded the expected pattern of peaks as shown in the chromatogram in Figure 2. This means that all gene-fragments were amplified at similar yields.

**Table I. Sequences and melting temperatures of the probes.**

| Probe | Tm | Sequence (5'-3') |
|---|---|---|
| HPA-1aa | 65.86 | tacaggccctgcctctggg |
| HPA-1ab | 66.92 | aggccctgcctctgggct |
| HPA-1ac | 67.79 | ccctgcctctgggctcacc |
| HPA-1ad | 67.35 | tgcctctgggctcacctcg |
| HPA-1ae | 64.65 | ctctgggctcacctcgctg |
| HPA-1aa CR | 64.65 | cagcgaggtgagcccagag |
| HPA-1ab CR | 67.35 | cgaggtgagcccagaggca |
| HPA-1ac CR | 67.79 | ggtgagcccagaggcaggg |
| HPA-1ad CR | 66.92 | agcccagaggcagggcct |
| HPA-1ae CR | 65.86 | cccagaggcagggcctgta |
| HPA-1ba | 69.93 | tacaggccctgcctccggg |
| HPA-1bb | 70.94 | aggccctgcctccgggct |
| HPA-1bc | 68.84 | ccctgcctccgggctcac |
| HPA-1bd | 66.21 | ctgcctccgggctcacct |
| HPA-1be | 66.33 | ctccgggctcacctcgct |
| HPA-1ba CR | 66.33 | agcgaggtgagcccggag |
| HPA-1bb CR | 66.21 | aggtgagcccggaggcag |
| HPA-1bc CR | 68.84 | gtgagcccggaggcaggg |
| HPA-1bd CR | 70.94 | agcccggaggcagggcct |
| HPA-1be CR | 69.93 | cccggaggcagggcctgta |
| HPA-2aa | 63.81 | ctgacgcccacacccaag |
| HPA-2ab | 65.21 | ctcctgacgcccacaccc |
| HPA-2ac | 65.37 | ggctcctgacgcccacac |
| HPA-2ad | 66.12 | agggctcctgacgcccac |
| HPA-2ae | 68.84 | ccagggctcctgacgccc |
| HPA-2aa CR | 63.81 | cttgggtgtgggcgtcag |
| HPA-2ab CR | 65.21 | gggtgtgggcgtcaggag |
| HPA-2ac CR | 65.37 | gtgtgggcgtcaggagcc |
| HPA-2ad CR | 66.12 | gtgggcgtcaggagccct |
| HPA-2ae CR | 68.84 | gggcgtcaggagccctgg |
| HPA-2ba | 64.88 | cctgatgcccacacccaag |
| HPA-2bb | 65.57 | ctcctgatgcccacaccca |
| HPA-2bc | 66.37 | ggctcctgatgcccacacc |
| HPA-2bd | 66.45 | agggctcctgatgcccaca |
| HPA-2be | 66.45 | ccagggctcctgatgccc |
| HPA-2ba CR | 64.88 | cttgggtgtgggcatcagg |
| HPA-2bb CR | 65.57 | tgggtgtgggcatcaggag |
| HPA-2bc CR | 66.37 | ggtgtgggcatcaggagcc |
| HPA-2bd CR | 66.45 | tgtgggcatcaggagccct |
| HPA-2be CR | 66.45 | gggcatcaggagccctgg |
| HPA-3aa | 69.45 | ccatccccagcccctccc |
| HPA-3ab | 69.60 | gcccatccccagcccctc |
| HPA-3ac | 70.57 | ctgcccatccccagcccc |
| HPA-3ad1 | 70.72 | gctgcccatccccagccc |
| HPA-3ad | 70.72 | ggctgcccatccccagcc |
| HPA-3ad2 | 70.72 | gggctgcccatccccagc |
| HPA-3ae | 70.08 | ggggctgcccatcccca |
| HPA-3aa CR | 69.45 | gggaggggctggggatgg |
| HPA-3ab CR | 69.60 | gaggggctggggatgggc |
| HPA-3ac CR | 70.57 | ggggctggggatgggcag |
| HPA-3ad1 CR | 70.72 | gggctggggatgggcagc |
| HPA-3ad CR | 70.72 | ggctggggatgggcagcc |
| HPA-3ad2 CR | 70.72 | gctggggatgggcagccc |
| HPA-3ae CR | 70.08 | tggggatgggcagcccc |
| HPA-3ba | 68.75 | ccagccccagcccctcc |
| HPA-3bb | 70.39 | gcccagccccagcccct |
| HPA-3bc | 69.91 | ctgcccagccccagccc |
| HPA-3bd1 | 70.07 | gctgcccagccccagcc |
| HPA-3bd | 70.07 | ggctgcccagccccagc |
| HPA-3bd2 | 69.91 | gggctgcccagccccag |
| HPA-3be | 72.50 | ggggctgcccagcccca |
| HPA-3ba CR | 68.75 | ggaggggctggggctgg |
| HPA-3bb CR | 70.39 | aggggctggggctgggc |
| HPA-3bc CR | 69.91 | gggctggggctgggcag |
| HPA-3bd1 CR | 70.07 | ggctggggctgggcagc |
| HPA-3bd CR | 70.07 | gctggggctgggcagcc |
| HPA-3bd2 CR | 69.91 | ctggggctgggcagccc |
| HPA-3be CR | 72.50 | tggggctgggcagcccc |
| HPA-4aa | 62.77 | gccacccagatgcgaaag |
| HPA-4ab | 59.94 | cacccagatgcgaaagct |
| HPA-4ac | 61.08 | cccagatgcgaaagctca |
| HPA-4ad | 57.99 | cagatgcgaaagctcacc |
| HPA-4ae | 57.99 | gatgcgaaagctcaccag |
| HPA-4aa CR | 57.99 | ctggtgagctttcgcatc |
| HPA-4ab CR | 57.99 | ggtgagctttcgcatctg |
| HPA-4ac CR | 61.08 | tgagctttcgcatctggg |
| HPA-4ad CR | 59.94 | agctttcgcatctgggtg |
| HPA-4ae CR | 62.77 | ctttcgcatctgggtggc |
| HPA-4ba | 60.20 | gccacccagatgcaaaag |
| HPA-4bb | 61.19 | ccacccagatgcaaaagct |
| HPA-4bc | 60.26 | acccagatgcaaaagctcac |
| HPA-4bd | 58.50 | cagatgcaaaagctcacca |
| HPA-4be | 58.04 | gatgcaaaagctcaccagtaa |
| HPA-4ba CR | 58.04 | ttactggtgagcttttgcatc |
| HPA-4bb CR | 58.50 | tggtgagcttttgcatctg |
| HPA-4bc CR | 60.26 | gtgagcttttgcatctgggt |
| HPA-4bd CR | 61.19 | agcttttgcatctgggtgg |
| HPA-4be CR | 60.20 | cttttgcatctgggtggc |
| HPA-5aa | 57.46 | gagtctacctgtttactatcaaagagg |
| HPA-5ab | 56.77 | agtctacctgtttactatcaaagaggta |
| HPA-5ac | 57.08 | gtctacctgtttactatcaaagaggtaa |
| HPA-5ad | 57.16 | ctacctgtttactatcaaagaggtaaaa |
| HPA-5ae | 57.38 | acctgtttactatcaaagaggtaaaaa |
| HPA-5aa CR | 57.38 | tttttacctctttgatagtaaacaggt |
| HPA-5ab CR | 57.16 | ttttacctctttgatagtaaacaggtag |
| HPA-5ac CR | 57.08 | ttacctctttgatagtaaacaggtagac |
| HPA-5ad CR | 56.77 | tacctctttgatagtaaacaggtagact |
| HPA-5ae CR | 57.46 | cctctttgatagtaaacaggtagactc |
| HPA-5ba | 57.42 | gagtctacctgtttactatcaaaaagg |
| HPA-5bb | 56.75 | agtctacctgtttactatcaaaaaggta |
| HPA-5bc | 57.05 | gtctacctgtttactatcaaaaaggtaa |
| HPA-5ad | 57.13 | ctacctgtttactatcaaaaaggtaaaa |
| HPA-5be | 57.34 | acctgtttactatcaaaaaggtaaaaa |
| HPA-5ba CR | 57.34 | tttttacctttttgatagtaaacaggt |
| HPA-5bb CR | 57.13 | ttttacctttttgatagtaaacaggtag |
| HPA-5bc CR | 57.05 | ttacctttttgatagtaaacaggtagac |
| HPA-5bd CR | 56.75 | tacctttttgatagtaaacaggtagact |
| HPA-5be CR | 57.42 | cctttttgatagtaaacaggtagactc |
| Gov-aa | 58.44 | ttattatcttgacttcagttacaggattt |
| Gov-ab | 59.20 | tcttgacttcagttacaggatttacc |
| Gov-ac | 59.15 | tgacttcagttacaggatttaccaa |
| Gov-ad | 58.36 | cttcagttacaggatttaccaagaat |
| Gov-ae | 59.44 | cagttacaggatttaccaagaatttg |
| Gov-aa CR | 59.44 | caaattcttggtaaatcctgtaactg |
| Gov-ab CR | 58.36 | attcttggtaaatcctgtaactgaag |
| Gov-ac CR | 59.15 | tggtaaatcctgtaactgaagtcaa |
| Gov-ad CR | 59.20 | ggtaaatcctgtaactgaagtcaaga |
| Gov-ae CR | 58.44 | aaatcctgtaactgaagtcaagataataa |
| Gov-ba | 58.35 | tatcttgacttcagttccaggatt |
| Gov-bb | 58.39 | cttgacttcagttccaggatttac |
| Gov-bc | 59.51 | gacttcagttccaggatttacca |
| Gov-bd | 58.24 | ttcagttccaggatttaccaag |
| Gov-be | 59.79 | cagttccaggatttaccaagaatt |
| Gov-ba CR | 59.79 | aattcttggtaaatcctggaactg |
| Gov-bb CR | 58.24 | cttggtaaatcctggaactgaa |
| Gov-bc CR | 59.51 | ggtaaatcctggaactgaac |
| Gov-bd CR | 58.39 | gtaaatcctggaactgaagtcaag |
| Gov-be CR | 58.35 | aatcctggaactgaagtcaagata |
| a33 | 63.03 | tcagccctatcgcaggatgt |
| a35 | 53.75 | gagacacttgacagtagcca |
| a38 | 61.40 | ggcagggcacctcagtttat |
| a42 | 57.37 | tcaccagccagactgtgtag |
| a43 | 60.02 | cttcacgcaagttgtccaga |
| CS05 | 52.41 | gtcctgacttctagctcatg |

| | | |
|---|---|---|
| Each oligonucleotide probe has an amine group and a C6 spacer at its The polymorphic sites in the allele-specific probes are highlighted. | | |

**Table II. Primers used for multiplex PCR**

| **MAPH PCR-primer** | **Ag system** | **Sequence** | **Tm*** | **Product size (bp)** | **concentration of primer in mpx(nM)** |
|---|---|---|---|---|---|
| HPA1-left | HPA-1 | gccgcgaattcactagtgcttcaggtcacagcgaggt | 58.98 | | 5 |
| HPA1-right | HPA-1 | ggccgcgggaattcgattgctccaatgtacggggtaaa | 59.82 | 185 | 5 |
| HPA2-left | HPA-2 | gccgcgaattcactagtgtgaaaggcaatgagctgaag | 59.15 | | 5 |
| HPA2-right | HPA-2 | ggccgcgggaattcgattagccagactgagcttctcca | 60.28 | 113 | 5 |
| HPA3-left | HPA-3 | gccgcgaattcactagtggcctgaccactcctttgc | 59.35 | | 7.5 |
| HPA3-right | HPA-3 | ggccgcgggaattcgattggaagatctgtctgcgatcc | 59.77 | 141 | 7.5 |
| HPA4-left | HPA-4 | gccgcgaattcactagtgatccgcaggttactggtgag | 60.13 | | 5 |
| HPA4-right | HPA-4 | ggccgcgggaattcgattccatgaaggatgatctgtgg | 58.88 | 121 | 5 |
| HPA5-left | HPA-5 | gccgcgaattcactagtgtccaaatgcaagttaaattaccag | 59.47 | | 5 |
| HPA5-right | HPA-5 | ggccgcgggaattcgattacagacgtgctcttggtaggt | 58.88 | 190 | 5 |
| Gov-left | Gov | gccgcgaattcactagtgtgtatcagttcttggttttgtgatg | 60.31 | | 5 |
| Gov-right | Gov | ggccgcgggaattcgattaaaaccagtagccacccaag | 59.10 | 156 | 5 |
| JK1/2-left | Kidd | gccgcgaattcactagtggtctttcagccccatttgag | 59.67 | | 10 |
| JK1/2-right | Kidd | ggccgcgggaattcgattgttgaaaccccagagtccaa | 59.94 | 86 | 10 |
| FY1/2-left | Duffy | gccgcgaattcactagtggaattcttcctatggtgtgaatga | 59.38 | | 5 |
| FY1/2-right | Duffy | ggccgcgggaattcgattaagaagggcagtgcagagtc | 59.60 | 153 | 5 |
| GATAbox-left | Duffy | gccgcgaattcactagtgggccctcattagtccttgg | 59.49 | | 5 |
| GATAbox-right | Duffy | ggccgcgggaattcgattgaaatgaggggcatagggata | 60.12 | 200 | 5 |
| KEL1/2-left | Kell | gccgcgaattcactagtgaagggaaatggccatactga | 59.39 | | 5 |
| KEL1/2-right | Kell | ggccgcgggaattcgattagctgtgtaagagccgatcc | 59.46 | 207 | 5 |
| KEL3/4-left | Kell | gccgcgaattcactagtggcctcagaaactggaacagc | 60.00 | | 10 |
| KEL3/4-right | Kell | ggccgcgggaattcgattagcaaggtgcaagaacactct | 59.15 | 103 | 10 |
| RHCEex2for | Rhesus | gccgcgaattcactagtgcgtctgcttccccctcc | 61.78 | | 5 |
| RHex2rev | Rhesus | ggccgcgggaattcgattctgaacagtgtgatgaccacc | 59.01 | 268 | 5 |
| RHCEex5-left | Rhesus | gccgcgaattcactagtgggatgttctggccaagtg | 57.47 | | 5 |
| RHex5rev | Rhesus | ggccgcgggaattcgattggctgtcaccacactgactg | 60.37 | 143 | 5 |
| RHDΨ-left | Rhesus | ggccgcgggaattcgattgtagtgagctggcccatca | 59.80 | | 5 |
| RHDΨ-right | Rhesus | gccgcgaattcactagtgtgtctagtttcttaccggcaagt | 59.38 | 380 | 5 |
| RHD-leftB | Rhesus | gccgcgaattcactagtgttataataacacttgtccacaggg | 57.25 | | 5 |
| RHD-rightC | Rhesus | ggccgcgggaattcgattcggctccgacggtatc | 58.61 | 182 | 5 |
| BigC-left | Rhesus | gccgcgaattcactagtgggccaccaccatttgaa | 58.77 | | 15 |
| BigC-rightintron2 | Rhesus | ggccgcgggaattcgattccatgaacatgccacttcac | 59.97 | 393 | 15 |
| MN-left | MNS | gccgcgaattcactagtgtgagggaatttgtcttttgca | 60.60 | | 5 |
| MN-right | MNS | ggccgcgggaattcgattcagaggcaagaattcctcca | 60.33 | 394 | 5 |
| U-left | MNS | gccgcgaattcactagtgcgctgatgttatctgtcttatttttc | 59.64 | | 5 |
| U-right | MNS | ggccgcgggaattcgattgatcgttccaataataccagcc | 59.71 | 175 | 5 |
| JO-left | Dombrock | gccgcgaattcactagtgcctggcttaaccaaggaaaa | 59.20 | | 5 |
| JO-right | Dombrock | ggccgcgggaattcgatttcatactgctgtggagtcctg | 58.89 | 181 | 5 |
| MAPH-reverse# | | gccgcgaattcactagtg | 57.94 | | 200 |
| MAPH-forward# | | ggccgcgggaattcgatt | 67.55 | | 200 |

| | | | | | |
|---|---|---|---|---|---|
| * Tm values of chimeric primers are without MAPH-tags. # Cy5 labelled at 5'-end The gene specific part of the RHDϕ-right and the RHD-rightC primer has been published (Maaskant-van Wijk et al. Transfusion 39, 546, 1999). Published primer sequences Rex6AD3: 5'-TGTCTAGTTTCTTACCGGCAAGT-3'; R1068: 5'-ATTGCCGGCTCCGACGGTATC-3') The gene specific part of BigC-left has been published as part of a published primer and part of the bigC-rightintron2, RHex2rev, RHCEex5for primers have been published (Tax et al. Transfusion 42, 634-44, 2002). Published primer sequences R348: 5'-CTGAACAGTGTGATGACC-3'; c/rev: 5'-TGATGACCACCTTCCCAGG-3'; R637: 5'-GCCCTCTTCTTGTGGATG-3'; C/for: 5'-CAGGGCCACCACCATTTGAA-3'; C/rev: 5'-GAACATGCCACTTCACTCCAG-3') Part of the MN-primers has been published (Akane et al. Vox Sang, 79, 183-7, 2000) Published primer sequences MN-Left: 5'-GAGGGAATTTGTCTTTTGCA-3' and MN-Right: 5'-AGAGGCAAGAATTCCTCC-3'. | | | | | |

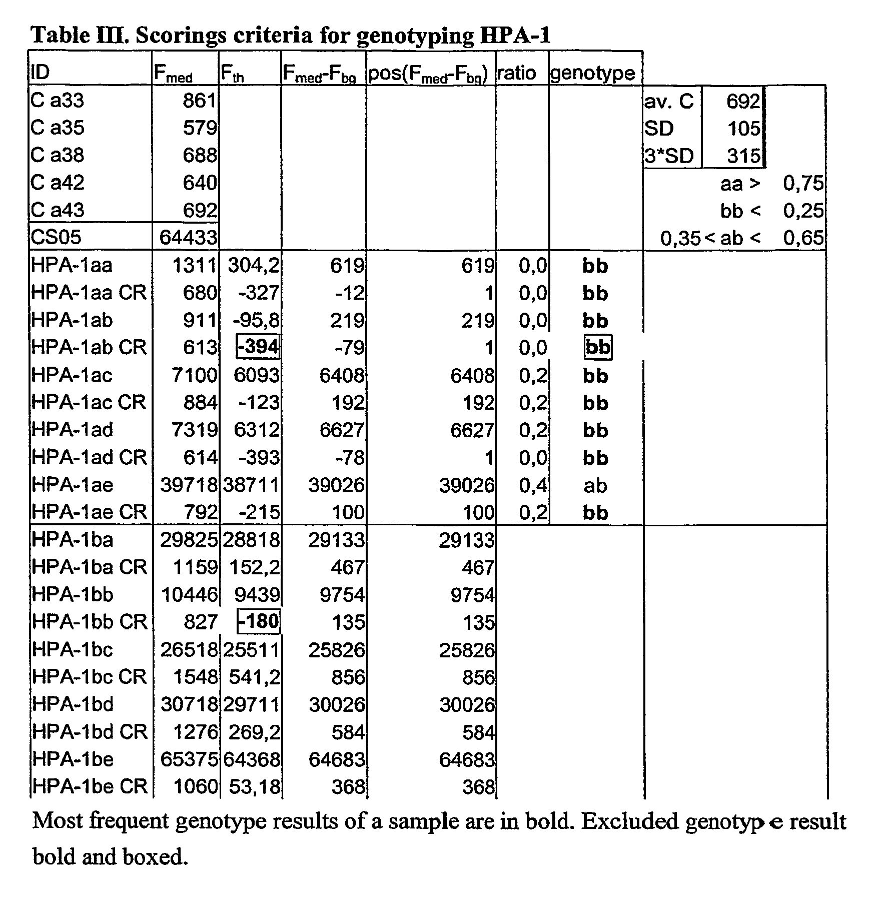

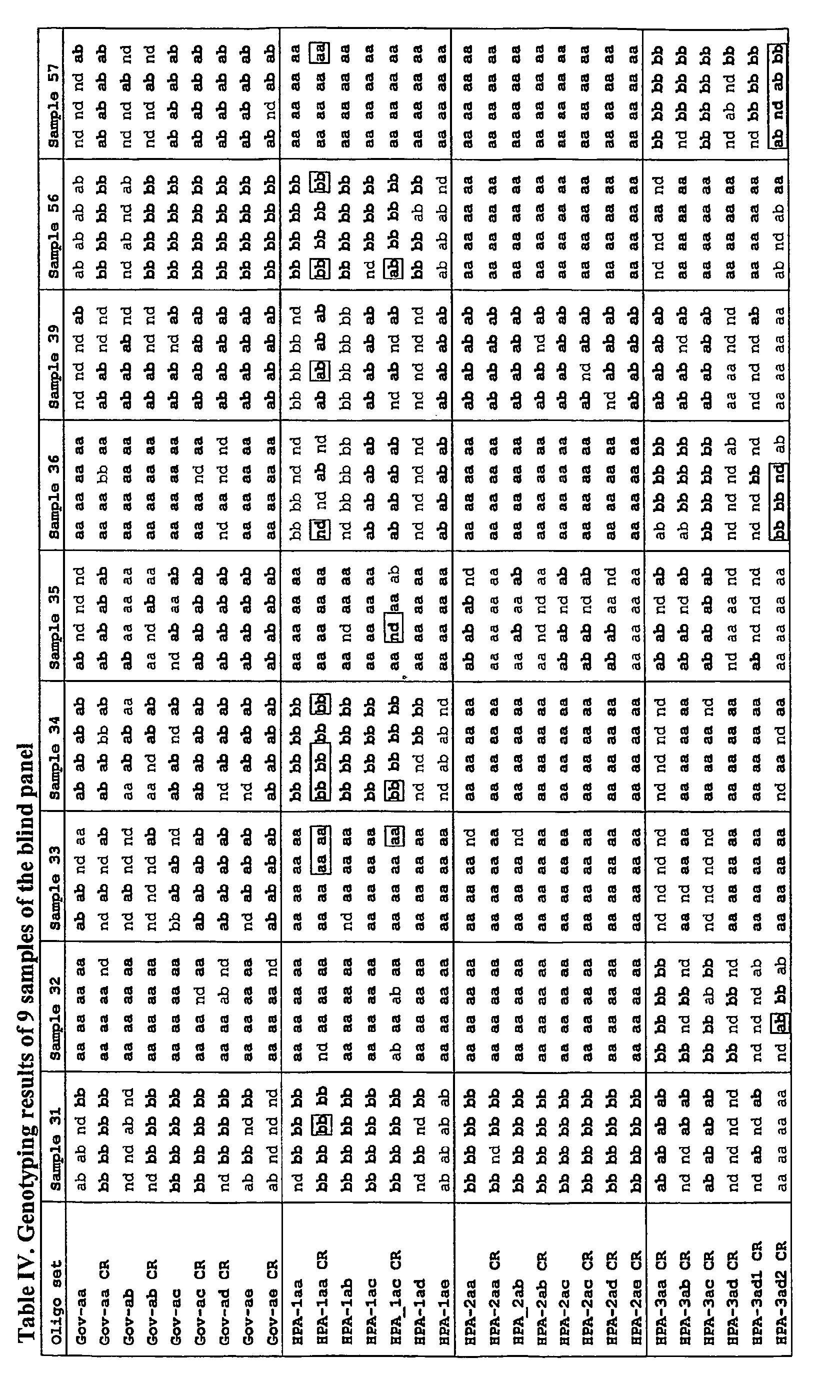

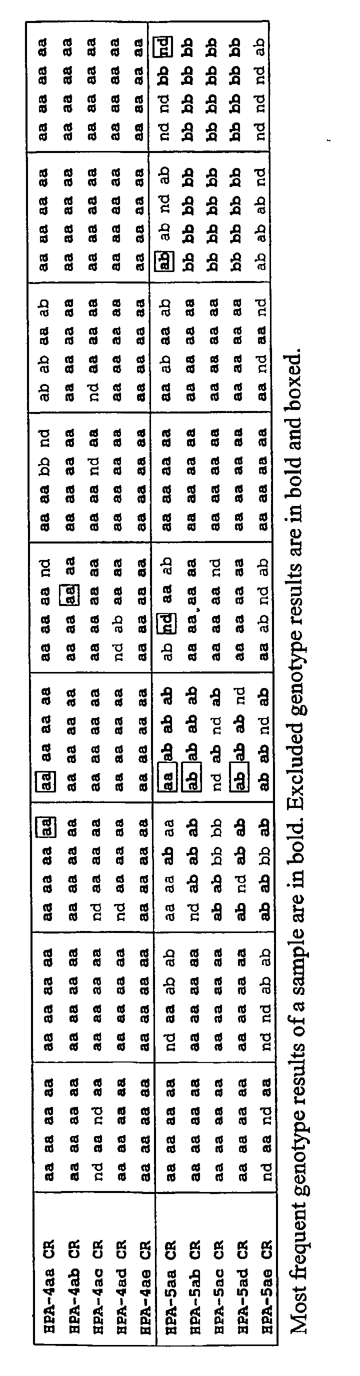

**Table V. Genotypes of blind panel of 58 samples**

| Sample | HPA-1 | % pos HPA-1 | | HPA-2 | % pos HPA-2 | | HPA-3 | % pos HPA-3 | | HPA-4 | % pos HPA-4 | | HPA-5 | % pos HPA-5 | | Gov | % pos gov | |
|---|---|---|---|---|---|---|---|---|---|---|---|---|---|---|---|---|---|---|
| 1 | **aa** | 100% | 28/28 | **aa** | 94% | 30/32 | **bb** | 54% | 13/24 | **aa** | 85% | 17/20 | **aa** | 75% | 15/20 | **ab** | 75% | 27/36 |
| 2 | **aa** | 96% | 24/25 | **aa** | 100% | 32/32 | **aa** | 83% | 20/24 | **aa** | 100% | 20/20 | **aa** | 79% | 15/19 | **ab** | 64% | 23/36 |
| 3 | **bb** | 70% | 19/27 | **aa** | 100% | 31/31 | **ab** | 50% | 12/24 | **aa** | 84% | 16/19 | **ab** | 80% | 16/20 | **bb** | 72% | 26/36 |
| 4 | **ab** | 43% | 10/23 | **aa** | 100% | 32/32 | **aa** | 79% | 19/24 | **aa** | 94% | 17/18 | **ab** | 45% | 5/11 | **aa** | 67% | 24/36 |
| 5 | **aa** | 96% | 27/28 | **aa** | 100% | 32/32 | **aa** | 83% | 20/24 | **aa** | 100% | 20/20 | **aa** | 68% | 13/19 | **aa** | 94% | 34/36 |
| 6 | **aa** | 93% | 25/27 | **ab** | 69% | 22/32 | **ab** | 42% | 10/24 | **aa** | 100% | 20/20 | **ab** | 53% | 10/19 | **aa** | 58% | 21/36 |
| 7 | **ab** | 54% | 15/28 | **ab** | 81% | 26/32 | **aa** | 79% | 19/24 | **aa** | 100% | 20/20 | **ab** | 60% | 12/20 | **ab** | 72% | 26/36 |
| 8 | **aa** | 96% | 27/28 | **aa** | 100% | 32/32 | **aa** | 83% | 20/24 | **aa** | 95% | 19/20 | **bb** | 65% | 13/20 | **ab** | 81% | 29/36 |
| 9 | **bb** | 81% | 21/26 | **aa** | 100% | 32/32 | **ab** | 38% | 9/24 | **aa** | 90% | 18/20 | **ab** | 65% | 13/20 | **ab** | 78% | 28/36 |
| 10 | **aa** | 96% | 27/28 | **ab** | 72% | 23/32 | **aa** | 79% | 19/24 | **aa** | 95% | 19/20 | **aa** | 85% | 17/20 | **ab** | 83% | 30/36 |
| 11 | **bb** | 70% | 16/23 | **aa** | 97% | 30/31 | **bb** | 44% | 7/16 | **aa** | 93% | 13/14 | **bb** | 81% | 13/16 | **aa** | 83% | 30/36 |
| 12 | **aa** | 100% | 23/23 | **ab** | 55% | 17/31 | **ab** | 46% | 11/24 | **aa** | 83% | 10/12 | **aa** | 80% | 16/20 | **bb** | 75% | 27/36 |
| 13 | **ab** | 54% | 15/28 | **ab** | 63% | 20/32 | **ab** | 50% | 12/24 | **aa** | 85% | 17/20 | **ab** | 65% | 13/20 | **aa** | 64% | 23/36 |
| 14 | **ab** | 44% | 12/27 | **bb** | 94% | 30/32 | **ab** | 50% | 12/24 | **aa** | 100% | 20/20 | **aa** | 85% | 17/20 | **bb** | 64% | 23/36 |
| 15 | **aa** | 93% | 26/28 | **aa** | 100% | 32/32 | **ab** | 38% | 9/24 | **aa** | 90% | 18/20 | **ab** | 55% | 11/20 | **aa** | 72% | 26/36 |
| 16 | **aa** | 96% | 27/28 | **aa** | 75% | 24/32 | **ab** | 42% | 10/24 | **aa** | 89% | 17/19 | **ab** | 70% | 14/20 | **ab** | 81% | 29/36 |
| 17 | **bb** | 74% | 20/27 | **ab** | 53% | 17/32 | **ab** | 39% | 9/23 | **aa** | 95% | 19/20 | **aa** | 78% | 14/18 | **ab** | 67% | 24/36 |
| 18 | **aa** | 93% | 25/27 | **ab** | 81% | 26/32 | **ab** | 33% | 8/24 | **aa** | 75% | 15/20 | **ab** | 75% | 15/20 | **aa** | 94% | 34/36 |
| 19 | **aa** | 96% | 27/28 | **ab** | 78% | 25/32 | **ab** | 54% | 13/24 | **aa** | 100% | 20/20 | **aa** | 65% | 13/20 | **ab** | 69% | 25/36 |
| 20 | **bb** | 75% | 21/28 | **aa** | 97% | 31/32 | **aa** | 83% | 20/24 | **aa** | 100% | 20/20 | **aa** | 90% | 18/20 | **ab** | 64% | 23/36 |
| 21 | **aa** | 100% | 28/28 | **ab** | 66% | 21/32 | **bb** | 61% | 14/23 | **aa** | 95% | 19/20 | **aa** | 100% | 20/20 | **bb** | 61% | 22/36 |
| 22 | **aa** | 96% | 26/27 | **aa** | 100% | 32/32 | **aa** | 88% | 21/24 | **aa** | 90% | 18/20 | **aa** | 80% | 16/20 | **bb** | 83% | 30/36 |
| 23 | **aa** | 100% | 28/28 | **aa** | 100% | 29/29 | **aa** | 88% | 21/24 | **aa** | 85% | 17/20 | **aa** | 80% | 16/20 | **bb** | 78% | 28/36 |
| 24 | **aa** | 100% | 28/28 | **aa** | 100% | 31/31 | **ab** | 42% | 10/24 | **aa** | 100% | 20/20 | **ab** | 65% | 13/20 | **bb** | 53% | 19/36 |
| 25 | **bb** | 85% | 22/26 | **ab** | 78% | 25/32 | **ab** | 38% | 9/24 | **aa** | 72% | 13/18 | **aa** | 79% | 15/19 | **aa** | 94% | 34/36 |
| 26 | **aa** | 96% | 27/28 | **aa** | 100% | 32/32 | **aa** | 79% | 19/24 | **aa** | 95% | 18/19 | **ab** | 95% | 18/19 | **ab** | 64% | 23/36 |
| 27 | **aa** | 81% | 21/26 | **aa** | 100% | 31/31 | **aa** | 63% | 15/24 | **aa** | 86% | 12/14 | **aa** | 100% | 19/19 | **ab** | 64% | 23/36 |
| 28 | **aa** | 79% | 22/28 | **aa** | 100% | 32/32 | **bb** | 68% | 16/22 | **aa** | 80% | 16/20 | **aa** | 75% | 15/20 | **ab** | 72% | 26/36 |
| 29 | **aa** | 100% | 28/28 | **ab** | 69% | 22/32 | **ab** | 54% | 13/24 | **aa** | 84% | 16/19 | **aa** | 80% | 16/20 | **ab** | 67% | 24/36 |
| 30 | **bb** | 70% | 16/23 | **aa** | 100% | 32/32 | **bb** | 72% | 13/18 | **aa** | 85% | 11/13 | **aa** | 94% | 15/16 | **ab** | 54% | 19/35 |
| 31 | **bb** | 74% | 20/27 | **bb** | 97% | 31/32 | **ab** | 50% | 12/24 | **aa** | 90% | 18/20 | **aa** | 90% | 18/20 | **bb** | 58% | 21/36 |
| 32 | **aa** | 89% | 25/28 | **aa** | 100% | 32/32 | **bb** | 52% | 12/23 | **aa** | 100% | 20/20 | **aa** | 65% | 13/20 | **aa** | 86% | 31/36 |
| 33 | **aa** | 96% | 24/25 | **aa** | 94% | 30/32 | **aa** | 63% | 15/24 | **aa** | 94% | 17/18 | **ab** | 60% | 12/20 | **ab** | 64% | 23/36 |
| 34 | **bb** | 75% | 18/24 | **aa** | 100% | 32/32 | **aa** | 71% | 17/24 | **aa** | 100% | 19/19 | **ab** | 76% | 13/17 | **ab** | 78% | 28/36 |
| 35 | **aa** | 93% | 25/27 | **ab** | 41% | 13/32 | **ab** | 46% | 11/24 | **aa** | 84% | 16/19 | **aa** | 68% | 13/19 | **ab** | 69% | 25/36 |
| 36 | **ab** | 48% | 13/27 | **aa** | 100% | 32/32 | **bb** | 52% | 11/21 | **aa** | 85% | 17/20 | **aa** | 100% | 20/20 | **aa** | 86% | 31/36 |
| 37 | **aa** | 100% | 28/28 | **ab** | 72% | 23/32 | **bb** | 63% | 15/24 | **aa** | 100% | 19/19 | **aa** | 100% | 20/20 | **ab** | 78% | 28/36 |
| 38 | **aa** | 96% | 27/28 | **ab** | 78% | 25/32 | **ab** | 46% | 11/24 | **aa** | 100% | 20/20 | **aa** | 85% | 17/20 | **ab** | 78% | 28/36 |
| 39 | **ab** | 48% | 13/27 | **ab** | 91% | 29/32 | **ab** | 50% | 12/24 | **aa** | 80% | 16/20 | **aa** | 80% | 16/20 | **ab** | 75% | 27/36 |
| 40 | **ab** | 44% | 12/27 | **aa** | 100% | 32/32 | **bb** | 64% | 14/22 | **aa** | 84% | 16/19 | **ab** | 65% | 13/20 | **aa** | 86% | 31/36 |
| 41 | **ab** | 56% | 15/27 | **aa** | 100% | 32/32 | **ab** | 54% | 13/24 | **aa** | 100% | 19/19 | **aa** | 65% | 11/17 | **ab** | 75% | 27/36 |
| 42 | **aa** | 100% | 28/28 | **ab** | 59% | 19/32 | **ab** | 54% | 13/24 | **aa** | 90% | 18/20 | **aa** | 100% | 20/20 | **bb** | 67% | 24/36 |
| 43 | **aa** | 96% | 27/28 | **ab** | 72% | 23/32 | **aa** | 79% | 19/24 | **aa** | 100% | 20/20 | **aa** | 100% | 20/20 | **bb** | 53% | 19/36 |
| 44 | **aa** | 100% | 28/28 | **aa** | 100% | 32/32 | **bb** | 71% | 17/24 | **aa** | 95% | 19/20 | **aa** | 90% | 18/20 | **bb** | 53% | 19/36 |
| 45 | **aa** | 100% | 28/28 | **ab** | 63% | 20/32 | **bb** | 68% | 15/22 | **aa** | 100% | 20/20 | **aa** | 95% | 19/20 | **bb** | 56% | 20/36 |
| 46 | **ab** | 35% | 7/20 | **aa** | 94% | 30/32 | **ab** | 35% | 8/23 | **aa** | 79% | 11/14 | **aa** | 83% | 15/18 | **ab** | 61% | 22/36 |
| 47 | **aa** | 81% | 21/26 | **ab** | 78% | 25/32 | **ab** | 50% | 12/24 | **aa** | 71% | 12/17 | **aa** | 73% | 11/15 | **ab** | 69% | 24/35 |
| 48 | **aa** | 93% | 25/27 | **ab** | 94% | 30/32 | **ab** | 42% | 10/24 | **aa** | 84% | 16/19 | **aa** | 80% | 16/20 | **ab** | 64% | 23/36 |
| 49 | **bb** | 70% | 19/27 | **aa** | 100% | 32/32 | **ab** | 67% | 16/24 | **aa** | 100% | 20/20 | **aa** | 63% | 10/16 | **bb** | 64% | 23/36 |
| 50 | **aa** | 93% | 26/28 | **aa** | 100% | 32/32 | **ab** | 46% | 11/24 | **aa** | 95% | 19/20 | **aa** | 80% | 16/20 | **ab** | 72% | 26/36 |
| 51 | **aa** | 96% | 27/28 | **ab** | 72% | 23/32 | **aa** | 83% | 20/24 | **aa** | 85% | 17/20 | **aa** | 75% | 15/20 | **ab** | 61% | 22/36 |
| 52 | **aa** | 100% | 22/22 | **aa** | 100% | 32/32 | **aa** | 79% | 19/24 | **aa** | 89% | 16/18 | **aa** | 84% | 16/19 | **bb** | 75% | 27/36 |
| 53 | **bb** | 71% | 17/24 | **aa** | 100% | 32/32 | **bb** | 64% | 14/22 | **aa** | 94% | 15/16 | **aa** | 78% | 14/18 | **bb** | 69% | 25/36 |
| 54 | **aa** | 89% | 24/27 | **aa** | 100% | 32/32 | **ab** | 46% | 11/24 | **aa** | 83% | 15/18 | **ab** | 67% | 12/18 | **ab** | 67% | 24/36 |
| 55 | **aa** | 100% | 27/27 | **aa** | 100% | 32/32 | **ab** | 50% | 12/24 | **aa** | 100% | 19/19 | **aa** | 90% | 18/20 | **aa** | 83% | 30/36 |
| 56 | **bb** | 76% | 19/25 | **aa** | 100% | 32/32 | **aa** | 75% | 18/24 | **aa** | 100% | 20/20 | **bb** | 63% | 12/19 | **bb** | 78% | 28/36 |
| 57 | **aa** | 100% | 27/27 | **aa** | 100% | 32/32 | **bb** | 80% | 16/20 | **aa** | 100% | 20/20 | **bb** | 68% | 13/19 | **ab** | 72% | 26/36 |
| 58 | **ab** | 50% | 14/28 | **bb** | 100% | 32/32 | **ab** | 50% | 12/24 | **aa** | 70% | 14/20 | aa | 75% | 15/20 | **bb** | 83% | 30/36 |

| | | | | | | | | | | | | | | | | | | |
|---|---|---|---|---|---|---|---|---|---|---|---|---|---|---|---|---|---|---|
| Genotypes are in bold when the predicted genotype is higher then 50% for the homozygote genotypes or 40 % for the heterozygote genotypes (HPA-3ab > 30%) | | | | | | | | | | | | | | | | | | |

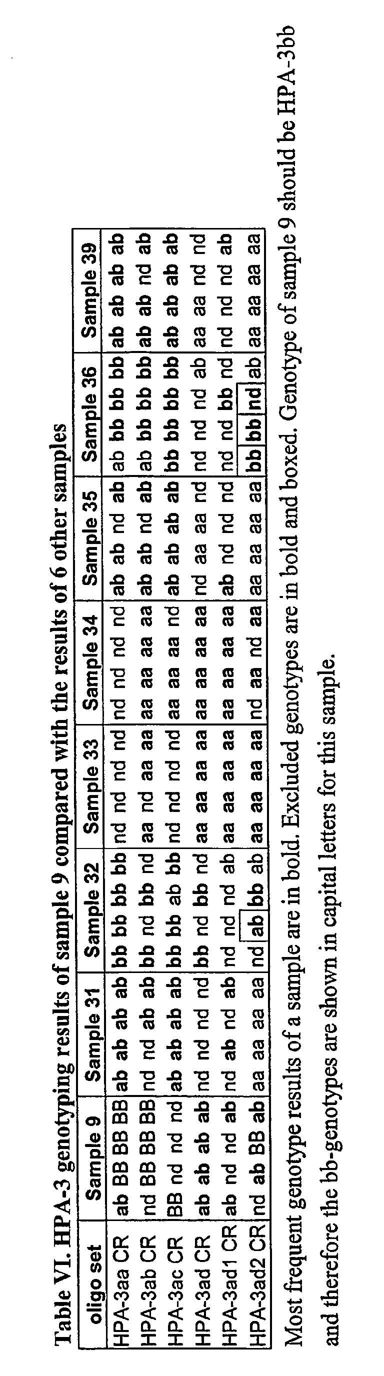

**Table VII. Additionally tested primers for multiplex PCR**

| These primers have been tested together with primers of SEQ ID NOS:15 to 40 in one multiplex PCR. PCR products of the correct product size were obtained. | | | | |
|---|---|---|---|---|
| | | | | |
| Ag system | MAPH PCR-primer | Sequence | Tm* | Product size (bp) |
| Duffy | Fyx-left(MAPH) | gccgcgaattcactagtgTCATGCTTTTCAGACCTCTCTTC | 60.01 | |
| Duffy | Fyx-right(MAPH2) | ggccgcgggaattcgattCAAGACGGGCACCACAAT | 60.53 | 143 |
| Kell | KEL6/7-left(MAPH) | gccgcgaattcactagtgGCAGCACCAACCCTATGTTC | 60.53 | |
| Kell | KEL6/7-right(MAPH) | ggccgcgggaattcgattTCAGGCACAGGTGAGCTTC | 60.13 | 154 |
| Rhesus | RHDex3-left(MAPH) | gccgcgaattcactagtgTCCTGGCTCTCCCTCTCT | 57.48 | |
| Rhesus | RHCEex3-right(MAPH) | ggccgcgggaattcgattTTTTTCAAAACCCCGGAAG | 59.90 | 294 |
| Rhesus | RhDVI-left(MAPH)(fw) | ggccgcgggaattcgattCTTTGAATTAAGCACTTCACAGA | 56.48 | |
| Rhesus | RhDVI-right(MAPH)(rev) | gccgcgaattcactagtgGCCAGAATCACACTCCTGCT | 60.42 | 277 |
| MNS | Ss-left(MAPH3) | gccgcgaattcactagtgTTTTTCTTTGCACATGTCTTT | 55.20 | |
| MNS | Ss-right(MAPH2) | ggccgcgggaattcgattTCTTTGTCTTTACAATTTCGTGTG | 58.42 | 276 |
| Dombrock | DO-left(MAPH) | ggccgcgggaattcgattTGATCCCTCCCTATGAGCTG | 60.17 | |
| Dombrock | DO-right(MAPH) | gccgcgaattcactagtgTTATATGTGCTCAGGTTCCCAGT | 59.91 | 136 |
| Colton | Colton-left(MAPH) | gccgcgaattcactagtgGCCACGACCCTCTTTGTCT | 60.25 | |
| Colton | Colton-right(MAPH) | ggccgcgggaattcgattTACATGAGGGCACGGAAGAT | 60.48 | 269 |
| Diego | Diego-left(MAPH) | gccgcgaattcactagtgACTTATTCACGGGCATCCAG | 59.96 | |
| Diego | Diego-right(MAPH) | ggccgcgggaattcgattAAGCTCCACGTTCCTGAAGA | 59.99 | 185 |
| Diego | Wr-left (MAPH) | gccgcgaattcactagtgGGCTTCAAGGTGTCCAACTC | 59.70 | |
| Diego | Wr-right (MAPH) | ggccgcgggaattcgattAGGATGAAGACCAGCAGAGC | 59.56 | 158 |
| Yt | Yt-left (MAPH) | ggccgcgggaattcgattCCTTCGTGCCTGTGGTAGAT | 60.13 | |
| Yt | Yt-right (MAPH) | gccgcgaattcactagtgTTCTGGGACTTCTGGGAATG | 60.04 | 235 |
| Lutheran | Lu-left (MAPH) | gccgcgaattcactagtgGGACCCAGAGAGAGAGAGACTG | 59.61 | |
| Lutheran | Lu-right (MAPH) | ggccgcgggaattcgattGGGAGTCCAGCTGGTATGG | 60.48 | 220 |

| | | | | |
|---|---|---|---|---|
| * Tm values are without MAPH tags. | | | | |

### References

- Akane A, Mizukami H and Shiono H. 2000. Classification of standard alleles of the MN blood group system. Vox Sang 79, 183-187.
- Belgrader P, Marino MM, Lubin M and Barany F. 1996. A Multiplex PCR-Ligase Detection Reaction Assay for Human Identity Testing. Genome Sci. Technol. 1, 77-87.
- Berry JE, Murphy CM, Smith GA, Ranasinghe E, Finberg R, Walton J, Brown J, Navarrete C, Metcalfe P and Ouwehand WH. 2000. Detection of Gov system antibodies by MAIPA reveals an immunogenicity similar to the HPA-5 alloantigens. Br J Haematol. 110, 735-742.
- Beuningen R van, van Damme H, Boender P, Bastiaensen N, Chan A and Kievits T. 2001. Fast and specific hybridisation using flow-through microarrays on porous metal oxide. Clin. Chem. 47, 1931-1933.
- Brownie J, Shawcross S, Theaker J, Whitcombe D, Ferrie R, Newton C and Little S. 1997. The elimination of primer-dimer accumulation in PCR. Nucl. Acids Res. 25, 3235-3241.
- Chamberlain JS, Gibbs RA, Ranier JE, Nguyen PN, and Caskey CT. 1988. Deletion screening of the Duchenne muscular dystrophy locus via multiplex DNA amplification. Nucl. Acids Res. 16, 11141-11156.
- Cheek BJ, Steel AB, Torres MP, Yu YY and Yang H. 2001. Chemiluminescence detection for hybridization assays on the flow-thru chip, a three-dimensional microchannel biochip. Anal Chem. 73, 5777-5783.
- Evans JG and Lee-Tataseo C. 2002. Determination of the factor V Leiden single-nucleotide polymorphism in a commercial clinical laboratory by use of NanoChip microelectronic array technology. Clin Chem. 48, 1406-1411.
- Fan JB, Chen X, Halushka MK, Berno A, Huang X, Ryder T, Lipshutz RJ, Lockhart DJ and Chakravarti A. 2000. Parallel genotyping of human SNPs using generic high-density oligonucleotide tag arrays. Genome Res. 10, 853-860.
- Fodor SP, Read JL, Pirrung MC, Stryer L, Lu AT and Solas D. 1991. Light-directed, spatially addressable parallel chemical synthesis. Science 251, 767-773.
- Guo Z, Gatterman MS, Hood L, Hansen JA and Petersdorf EW. 2001. Oligonucleotide arrays for high-throughput SNPs detection in the MHC class I genes: HLA-B as a model system. Genome Res. 12, 447-457.
- Hacia JG, Brody LC, Chee MS, Fodor SP and Collins FS. 1996. Detection of heterozygous mutations in BRCA1 using high density oligonucleotide arrays and two-colour fluorescence analysis. Nat Genet. 14, 441-447.
- Heath KE, Day INM and Humphries SE. 2000. Universal primer quantitative fluorescent multiplex (UPQFM) PCR: a method to detect major and minor rearrangements of the low density lipoprotein receptor gene. J.Med.Genet. 37, 272-280.
- Hinds DA, Stokowski RP, Patil N, Konvicka K, Kershenobich D, Cox DR and Ballinger DG. 2004. Matching strategies for genetic association studies in structured populations. Am J Hum Genet. 74, 317-325.
- Huang JX, Mehrens D, Wiese R, Lee S, Tam SW, Daniel S, Gilmore J, Shi M and Lashkari D. 2001. High-throughput genomic and proteomic analysis using microarray technology. Clin Chem. 47, 1912-1916.
- Iwasaki H, Ezura Y, Ishida R, Kajita M, Kodaira M, Knight J, Daniel S, Shi M and Emi M. 2002. Accuracy of genotyping for single nucleotide polymorphisms by a microarray-based single nucleotide polymorphism typing method involving hybridization of short allele-specific oligonucleotides. DNA Res. 9, 59-62.
- Jobs M, Howell WM, Stromqvist L, Mayr T and Brookes AJ. 2003. DASH-2: flexible, low-cost, and high-throughput SNP genotyping by dynamic allele-specific hybridization on membrane arrays. Genome Res. 13, 916-924.
- Kajiyama T, Miyahara Y, Kricka LJ, Wilding P, Graves DJ, Surrey S and Fortina P. 2003. Genotyping on a thermal gradient DNA chip. Genome Res. 13, 467-475.
- Lindroos K, Sigurdsson S, Johansson K, Ronnblom L and Syvanen AC. 2002. Multiplex SNP genotyping in pooled DNA samples by a four-colour microarray system. Nucleic Acids Res. 30, e70.
- Lu M, Shortreed MR, Hall JG, Wang L, Berggren T, Stevens PW, Kelso DM, Lyamichev V, Neri B and Smith LM. 2002. A surface invasive cleavage assay for highly parallel SNP analysis. Hum. Mutat. 19,416-422.
- Maaskant-van Wijk PA, Faas BH, de Ruijter JA, Overbeeke MA, von dem Borne AE, van Rhenen DJ and van der Schoot CE. 1999. Genotyping of RHD by multiplex polymerase chain reaction analysis of six RHD-specific exons. Transfusion 39, 546.
- Metcalfe P, Watkins NA, Ouwehand WH, Kaplan C, Newman P, Kekomaki R, De Haas M, Aster R, Shibata Y, Smith J, Kiefel V and Santoso S. 2003. Nomenclature of human platelet antigens. Vox Sang. 85, 240-245.
- Miller SA, Dykes DD and Polesky HF. 1988. A simple salting out procedure for extracting DNA from human nucleated cells. Nucl. Acids Res. 16, 1215.
- Mujumdar RB, Ernst LA, Mujumdar SR, Lewis CJ and Waggoner AS. 1993. Cyanine dye labeling reagents: sulfoindocyanine succinimidyl esters. Bioconjug. Chem. 4, 105-111.
- Park SJ, Taton TA and Mirkin CA. 2002. Array-based electrical detection of DNA with nanoparticle probes. Science 295, 1503-1506.
- Pastinen T, Kurg A, Metspalu A, Peltonen L and Syvanen AC. 1997. Minisequencing: a specific tool for DNA analysis and diagnostics on oligonucleotide arrays. Genome Res. 7, 606-614.
- Pastinen T, Raitio M, Lindroos K, Tainola P, Peltonen L and Syvanen AC. 2000. A system for specific, high-throughput genotyping by allele-specific primer extension on microarrays. Genome Res. 10, 1031-1042.
- Prix L, Uciechowski P, Bockmann B, Giesing M and Schuetz AJ. 2002. Diagnostic biochip array for fast and sensitive detection of K-ras mutations in stool. Clin. Chem. 48, 428-435.
- Randen I, Sorensen K, Killie MK and Kjeldsen-Kragh J. 2003. Rapid and reliable genotyping of human platelet antigen (HPA)-1, -2, -3, -4, and -5 a/b and Gov a/b by melting curve analysis. Transfusion 43, 445-450.
- Reid ME. 2003. Applications of DNA-based assays in blood group antigen and antibody identification. Transfusion 43, 1748-1757.
- Seltsam A, Wagner FF, Salama A and Flegel WA. 2003. Antibodies to high-frequency antigens may decrease the quality of transfusion support: an observational study. Transfusion 43, 1563-1566.
- Shuber AP, Grondin VJ and Klinger KW. 1995. A simplified procedure for developing multiplex PCRs. Genome Research 5, 488-493.
- Tax MG, van der Schoot CE, van Doom R, Douglas-Berger L, van Rhenen DJ and MaaskantvanWijk PA. 2002. RHC and RHc genotyping in different ethnic groups. Transfusion 42, 634-444.
- Wang DG, Fan JB, Siao CJ, Berno A, Young P, Sapolsky R, Ghandour G, Perkins N, Winchester E, Spencer J, Kruglyak L, Stein L, Hsie L, Topaloglou T, Hubbell E, Robinson E, Mittmann M, Morris MS, Shen N, Kilburn D, Rioux J, Nusbaum C, Rozen S, Hudson TJ and Lander ES. 1998. Large-Scale Identification, Mapping, and Genotyping of Single-Nucleotide Polymorphisms in the Human Genome. Science 280, 1077-1082.
- Wen WH, Bernstein L, Lescallett J, Beazer-Barclay Y, Sullivan-Halley J, White M and Press MF. 2000. Comparison of TP53 mutations identified by oligonucleotide microarray and conventional DNA sequence analysis. Cancer Res. 60, 2716-2722.
- Wen SY, Wang H, Sun OJ, Wang SQ. 2003. Rapid detection of the known SNPs of CYP2C9 using oligonucleotide microarray. World J Gastroenterol. 9, 1342-1346.
- White S, Kalf M, Liu Q, Villerius M, Engelsma D, Kriek M, Vollebregt E, Bakker B, van Ommen GJ, Breuning MH and den Dunnen JT. 2002. Comprehensive detection of genomic duplications and deletions in the DMD gene, by use of multiplex amplifiable probe hybridisation. Am.J.Hum.Genet. 71, 365-374.

## Claims

1. A method of genotyping blood cell antigens comprising subjecting DNA from an individual of a mammalian species to a multiplex Polymerase Chain Reaction (PCR) to amplify and detectably label a region of the locus of at least two different blood cell antigens which contains the site of nucleotide polymorphism of said blood cell antigen and using the thus amplified and labeled DNA fragments to determine the genotype for each of said blood cell antigens, said multiplex PCR comprising the use of at least one pair of blood cell antigen-specific chimeric primers for each blood cell antigen to be genotyped and at least one detectably labeled universal primer, wherein said at least one universal primer has a unique sequence not occurring in the DNA of said mammalian species, and wherein each chimeric primer pair comprises a left chimeric primer and a right chimeric primer, each of them comprising a blood cell antigen-specific part at the 3' end and a universal part at the 5' end, wherein the base sequence of the universal part of the chimeric primers corresponds to the base sequence of said at least one universal primer, and wherein said blood cell antigen-specific parts of the chimeric primer pair enclose a region of the locus of the blood cell antigen which contains the site of nucleotide polymorphism of said blood cell antigen.

2. A method according to claim 1, wherein said at least one detectably labeled universal primer is used in a molar amount which is at least 100 times, more preferably at least 200 times the molar amount of each chimeric primer.

3. A method according to claim 1 or 2, wherein a pair of detectably labeled universal primers with a unique sequence not occurring in the DNA of said mammalian species is used and wherein for each chimeric primer pair the base sequence of the universal part of one member of the chimeric primer pair corresponds to the base sequence of one member of the universal primer pair and the base sequence of the universal part of the other member of the chimeric primer pair corresponds to the base sequence of the other member of the universal primer pair.

4. A method according to claim 3, wherein one of said universal primers has the base sequence gccgcgaattcactagtg (SEQ ID NO:2) and the other universal primer has the base sequence ggccgcgggaattcgatt (SEQ ID NO:1).

5. A method according to any one of claims 1-4, wherein each universal primer carries a fluorescent label, preferably Cy5, at its 5' end.

6. A method according to any one of claims 1-5, wherein said mammalian species is human, said DNA is genomic DNA, and said blood cell antigens comprise at least two; preferably all, members selected from the group consisting of HPA1, HPA2, HPA3, HPA4, HPA5, Gov, JK1/2, FY1/2, GATAbox, KEL1/2, KEL3/4, RHCEex2/RHex2, RHCEex5/RHex5, RHDψ, RHD, BigC, MN, U and JO.

7. A method according to claim 6, wherein said chimeric primers comprise at least two, preferably substantially all, pairs selected from the group consisting of:
| | | |
|---|---|---|
| HPA1-left | gccgcgaattcactagtgcttcaggtcacagcgaggt | SEQ ID NO:3 |
| HPA1-right | ggccgcgggaattcgattgctccaatgtacggggtaaa | SEQ ID NO:4 |
| | | |
| HPA2-left | gccgcgaattcactagtgtgaaaggcaatgagctgaag | SEQ ID NO:5 |
| HPA2-right | ggccgcgggaattcgattagccagactgagcttctcca | SEQ ID NO:6 |
| | | |
| HPA3-left | gccgcgaattcactagtggcctgaccactcctttgc | SEQ ID NO:7 |
| HPA3-right | ggccgcgggaattcgattggaagatctgtctgcgatcc | SEQ ID NO:8 |
| | | |
| HPA4-left | gccgcgaattcactagtgatccgcaggttactggtgag | SEQ ID NO:9 |
| HPA4-right | ggccgcgggaattcgattccatgaaggatgatctgtgg | SEQ ID NO:10 |
| | | |
| HPA5-left | gccgcgaattcactagtgtccaaatgcaagttaaattaccag | SEQ ID NO:11 |
| HPA5-right | ggccgcgggaattcgattacagacgtgctcttggtaggt | SEQ ID NO:12 |
| | | |
| HPA15-left | gccgcgaattcactagtgtgtatcagttcttggttttgtgatg | SEQ ID NO:13 |
| HPA15-right | ggccgcgggaattcgattaaaaccagtagccacccaag | SEQ ID NO:14 |
| | | |
| JK1/2-left | gccgcgaattcactagtggtctttcagccccatttgag | SEQ ID NO:15 |
| JKl/2-right | ggccgcgggaattcgattgttgaaaccccagagtccaa | SEQ ID NO:16 |
| | | |
| FY1/2-left | gccgcgaattcactagtggaattcttcctatggtgtgaatga | SEQ ID NO:17 |
| FY1/2-right | ggccgcgggaattcgattaagaagggcagtgcagagtc | SEQ ID NO:18 |
| GATAbox-left | gccgcgaattcactagtgggccctcattagtccttgg | SEQ ID NO:19 |
| GATAbox-right | ggccgcgggaattcgattgaaatgaggggcatagggata | SEQ ID NO:20 |
| | | |
| Fyx-left | gccgcgaattcactagtgtcatgcttttcagacctctcttc | SEQ ID NO:175 |
| Fyx-right | ggccgcgggaattcgattcaagacgggcaccacaat | SEQ ID NO:176 |
| | | |
| KEL1/2-left | gccgcgaattcactagtgaagggaaatggccatactga | SEQ ID NO:21 |
| KEL1/2-right | ggccgcgggaattcgattagctgtgtaagagccgatcc | SEQ ID NO:22 |
| | | |
| KEL3/4-left | gccgcgaattcactagtggcctcagaaactggaacagc | SEQ ID NO:23 |
| KEL3/4-right | ggccgcgggaattcgattagcaaggtgcaagaacactct | SEQ ID NO:24 |
| | | |
| KEL6/7-left | gccgcgaattcactagtggcagcaccaaccctatgttc | SEQ ID NO:177 |
| KEL6/7-right | ggccgcgggaattcgatttcaggcacaggtgagcttc | SEQ ID NO:178 |
| | | |
| RHCEex2for | gccgcgaattcactagtgcgtctgcttccccctcc | SEQ ID NO:25 |
| RHex2rev | ggccgcgggaattcgattctgaacagtgtgatgaccacc | SEQ ID NO:26 |
| | | |
| RHDex3-left | gccgcgaattcactagtgtcctggctctccctctct | SEQ ID NO:179 |
| RHCEex3-right | ggccgcgggaattcgatttttttcaaaaccccggaag | SEQ ID NO:180 |
| | | |
| RHCEex5-left | gccgcgaattcactagtgggatgttctggccaagtg | SEQ ID NO:27 |
| RHex5rev | ggccgcgggaattcgattggctgtcaccacactgactg | SEQ ID NO:28 |
| | | |
| RHDΨ-left | ggccgcgggaattcgattgtagtgagctggcccatca | SEQ ID NO:29 |
| RHDΨ-right | gccgcgaattcactagtgtgtctagtttcttaccggcaagt | SEQ ID NO:30 |
| | | |
| RHD-leftB | gccgcgaattcactagtgttataataacacttgtccacaggg | SEQ ID NO:31 |
| RHD-rightC | ggccgcgggaattcgattcggctccgacggtatc | SEQ ID NO:32 |
| | | |
| BigC-left | gccgcgaattcactagtgggccaccaccatttgaa | SEQ ID NO:33 |
| BigC-rightintron2 | ggccgcgggaattcgattccatgaacatgccacttcac | SEQ ID NO:34 |
| | | |
| RhDVI-left (fw) | ggccgcgggaattcgattctttgaattaagcacttcacaga | SEQ ID NO:181 |
| RhDVI-right (rev) | gccgcgaattcactagtggccagaatcacactcctgct | SEQ ID NO:182 |
| MN-left | gccgcgaattcactagtgtgagggaatttgtcttttgca | SEQ ID NO:35 |
| MN-right | ggccgcgggaattcgattcagaggcaagaattcctcca. | SEQ ID NO:36 |
| | | |
| Ss-left | gccgcgaattcactagtgtttttctttgcacatgtcttt | SEQ ID NO:183 |
| Ss-right | ggccgcgggaattcgatttctttgtctttacaatttcgtgtg | SEQ ID NO:184 |
| | | |
| U-left | gccgcgaattcactagtgcgctgatgttatctgtcttatttttc | SEQ ID NO:37 |
| U-right | ggccgcgggaattcgattgatcgttccaataataccagcc | SEQ ID NO:38 |
| | | |
| DO-left | ggccgcgggaattcgatttgatccctccctatgagctg | SEQ ID NO:185 |
| DO-right | gccgcgaattcactagtgttatatgtgctcaggttcccagt | SEQ ID NO:186 |
| | | |
| JO-left | gccgcgaattcactagtgcctggcttaaccaaggaaaa | SEQ ID NO:39 |
| JO-right | ggccgcgggaattcgatttcatactgctgtggagtcctg | SEQ ID NO:40 |
| | | |
| Colton-left | gccgcgaattcactagtggccacgaccctctttgtct | SEQ ID NO:187 |
| Colton-right | ggccgcgggaattcgatltacatgagggcacggaagat | SEQ ID NO:188 |
| | | |
| Diego-left | gccgcgaattcactagtgacttattcacgggcatccag | SEQ ID NO:189 |
| Diego-right | ggccgcgggaattcgattaagctccacgttcctgaaga | SEQ ID NO:190 |
| | | |
| Wr-left | gccgcgaattcactagtgggcttcaaggtgtccaactc | SEQ ID NO:636 |
| Wr-right | ggccgcgggaattcgattaggatgaagaccagcagagc | SEQ ID NO:637 |
| | | |
| Yt-left | ggccgcgggaattcgattccttcgtgcctgtggtagat | SEQ ID NO:638 |
| Yt-right | gccgcgaattcactagtgttctgggacttctgggaatg | SEQ ID NO:639 |
| | | |
| Lu-left | gccgcgaattcactagtgggacccagagagagagagactg | SEQ ID NO:640 |
| Lu-right | ggccgcgggaattcgattgggagtccagctggtatgg | SEQ ID NO:641. |

8. A method according to any one of claims 1-7, wherein the DNA polymerase used in the multiplex PCR is Taq polymerase or a similar heat-resistant polymerase and each cycle of the multiplex PCR comprises a heat denaturation step of 15 to 60 sec at a temperature of 90 to 98°C (preferably about 30 sec at about 94°C), an annealing step of 60 to 120 sec at a temperature of 54 to 60°C (preferably about 90 sec at about 57°C), and a primer extension step of 60 to 120 sec at a temperature of 68 to 76°C (preferably about 90 sec at about 72°C).

9. A method according to any one of claims 1-8, wherein said multiplex PCR uses, based on a reaction volume of 50 µl, about 100 ng of genomic DNA from said individual, about 5 nM of each chimeric primer, per chimeric primer pair about 0.2 µM of each detectably labeled universal primer and about 25 µl of 2x MasterMix containing buffer, dNTP's and DNA polymerase.

10. A method according to any one of claims 1-9, wherein the genotype for each of said blood cell antigens is determined by hybridizing the products of the multiplex PCR amplification, after denaturation, to blood cell antigen allele-specific oligonucleotide probes contained in a DNA array or presented in any other form, such as supported on beads, and analyzing the hybridization pattern.

11. A method according to claim 10, wherein the allele-specific probes have a length of 15 to 40 nucleotides, preferably 17 to 30 nucleotides.

12. A method according to claim 10 or 11, wherein the array contains for each allele of a blood cell antigen at least two, preferably at least five, different sense probes and at least two, preferably at least five, different antisense probes, each of them covering the site of the nucleotide polymorphism but at varying positions.

13. A method according to claim 11, wherein the array contains at least 72 different probes, preferably substantially all probes, selected from the group consisting of:
| HPA1-allele a: | | | |
|---|---|---|---|
| | HPA-1aa | tacaggccctgcctctggg | SEQ ID NO:41 |
| | HPA-1ab | aggccctgcctctgggct | SEQ ID NO:42 |
| | HPA-1ac | ccctgcctctgggctcacc | SEQ ID NO:43 |
| | HPA-1ad | tgcctctgggctcacctcg | SEQ ID NO:44 |
| | HPA-1ae | ctctgggctcacctcgctg | SEQ ID NO:45 |
| | HPA-1aa CR | cagcgaggtgagcccagag | SEQ ID NO:46 |
| | HPA-1ab CR | cgaggtgagcccagaggca | SEQ ID NO:47 |
| | HPA-1ac CR | ggtgagcccagaggcaggg | SEQ ID NO:48 |
| | HPA-1ad CR | agcccagaggcagggcct | SEQ ID NO:49 |
| | HPA-1ae CR | cccagaggcagggcctgta | SEQ ID NO:50 |
| HPA1-allele b: | | | |
|---|---|---|---|
| | HPA-1ba | tacaggccctgcctccggg | SEQ ID NO:51 |
| | HPA-1bb | aggccctgcctccgggct | SEQ ID NO:52 |
| | HPA-1bc | ccctgcctccgggctcac | SEQ ID NO:53 |
| | HPA-1bd | ctgcctccgggctcacct | SEQ ID NO:54 |
| | HPA-1be | ctccgggctcacctcgct | SEQ ID NO:55 |
| | HPA-1ba CR | agcgaggtgagcccggag | SEQ ID NO:56 |
| | HPA-1bb CR | aggtgagcccggaggcag | SEQ ID NO:57 |
| | HPA-1bc CR | gtgagcccggaggcaggg | SEQ ID NO:58 |
| | HPA-1bd CR | agcccggaggcagggcct | SEQ ID NO:59 |
| | HPA-1be CR | cccggaggcagggcctgta | SEQ ID NO:60 |
| HPA2-allele a: | | | |
|---|---|---|---|
| | HPA-2aa | ctgacgcccacacccaag | SEQ ID NO:61 |
| | HPA-2ab | ctcctgacgcccacaccc | SEQ ID NO:62 |
| | HPA-2ac | ggctcctgacgcccacac | SEQ ID NO:63 |
| | HPA-2ad | agggctcctgacgcccac | SEQ ID NO:64 |
| | HPA-2ae | ccagggctcctgacgccc | SEQ ID NO:65 |
| | HPA-2aa CR | cttgggtgtgggcgtcag | SEQ ID NO:66 |
| | HPA-2ab CR | gggtgtgggcgtcaggag | SEQ ID NO:67 |
| | HPA-2ac CR | gtgtgggcgtcaggagcc | SEQ ID NO:68 |
| | HPA-2ad CR | gtgggcgtcaggagccct | SEQ ID NO:69 |
| | HPA-2ae CR | gggcgtcaggagccctgg | SEQ ID NO:70 |
| HPA2-allele b: | | | |
|---|---|---|---|
| | HPA-2ba | cctgatgcccacacccaag | SEQ ID NO:71 |
| | HPA-2bb | ctcctgatgcccacaccca | SEQ ID NO:72 |
| | HPA-2bc | ggctcctgatgcccacacc | SEQ ID NO:73 |
| | HPA-2bd | agggctcctgatgcccaca | SEQ ID NO:74 |
| | HPA-2be | ccagggctcctgatgccc | SEQ ID NO:75 |
| | HPA-2ba CR | cttgggtgtgggcatcagg | SEQ ID NO:76 |
| | HPA-2bb CR | tgggtgtgggcatcaggag | SEQ ID NO:77 |
| | HPA-2bc CR | ggtgtgggcatcaggagcc | SEQ ID NO:78 |
| | HPA-2bd CR | tgtgggcatcaggagccct | SEQ ID NO:79 |
| | HPA-2be CR | gggcatcaggagccctgg | SEQ ID NO:80 |
| HPA3-allele a: | | | |
|---|---|---|---|
| | HPA-3 aa | ccatccccagcccctccc | SEQ ID NO:81 |
| | HPA-3ab | gcccatccccagcccctc | SEQ ID NO:82 |
| | HPA-3ac | ctgcccatccccagcccc | SEQ ID NO:83 |
| | HPA-3ad1 | gctgcccatccccagccc | SEQ ID NO:84 |
| | HPA-3ad | ggctgcccatccccagcc | SEQ ID NO:85 |
| | HPA-3ad2 | gggctgcccatccccagc | SEQ ID NO:86 |
| | HPA-3ae | ggggctgcccatcccca | SEQ ID NO:87 |
| | HPA-3aa CR | gggaggggctggggatgg | SEQ ID NO:88 |
| | HPA-3ab CR | gaggggctggggatgggc | SEQ ID NO:89 |
| | HPA-3ac CR | ggggctggggatgggcag | SEQ ID NO:90 |
| | HPA-3ad1 CR | gggctggggatgggcagc | SEQ ID NO:91 |
| | HPA-3ad CR | ggctggggatgggcagcc | SEQ ID NO:92 |
| | HPA-3ad2 CR | gctggggatgggcagccc | SEQ ID NO:93 |
| | HPA-3ae CR | tggggatgggcagcccc | SEQ ID NO:94 |
| HPA3-allele b: | | | |
|---|---|---|---|
| | HPA-3ba | ccagccccagcccctcc | SEQ ID NO:95 |
| | HPA-3bb | gcccagccccagcccct | SEQ ID NO:96 |
| | HPA-3bc | ctgcccagccccagccc | SEQ ID NO:97 |
| | HPA-3bd1 | gctgcccagccccagcc | SEQ ID NO:98 |
| | HPA-3bd | ggctgcccagccccagc | SEQ ID NO:99 |
| | HPA-3bd2 | gggctgcccagccccag | SEQ ID NO:100 |
| | HPA-3be | ggggctgcccagcccca | SEQ ID NO:101 |
| | HPA-3ba CR | ggaggggctggggctgg | SEQ ID NO:102 |
| | HPA-3bb CR | aggggctggggctgggc | SEQ ID NO:103 |
| | HPA-3bc CR | gggctggggctgggcag | SEQ ID NO:104 |
| | HPA-3bd1 CR | ggctggggctgggcagc | SEQ ID NO:105 |
| | HPA-3bd CR | gctggggctgggcagcc | SEQ ID NO:106 |
| | HPA-3bd2 CR | ctggggctgggcagccc | SEQ ID NO:107 |
| | HPA-3be CR | tggggctgggcagcccc | SEQ ID NO:108 |
| HPA4-allele a: | | | |
|---|---|---|---|
| | HPA-4aa | gccacccagatgcgaaag | SEQ ID NO:109 |
| | HPA-4ab | cacccagatgcgaaagct | SEQ ID NO:110 |
| | HPA-4ac | cccagatgcgaaagctca | SEQ ID NO:111 |
| | HPA-4ad | cagatgcgaaagctcacc | SEQ ID NO:112 |
| | HPA-4ae | gatgcgaaagctcaccag | SEQ ID NO:113 |
| | HPA-4aa CR | ctggtgagctttcgcatc | SEQ ID NO:114 |
| | HPA-4ab CR | ggtgagctttcgcatctg | SEQ ID NO:115 |
| | HPA-4ac CR | tgagctttcgcatctggg | SEQ ID NO:116 |
| | HPA-4ad CR | agctttcgcatctgggtg | SEQ ID NO:117 |
| | HPA-4ae CR | ctttcgcatctgggtggc | SEQ ID NO:118 |
| HPA4-allele b: | | | |
|---|---|---|---|
| | HPA-4ba | gccacccagatgcaaaag | SEQ ID NO:119 |
| | HPA-4bb | ccacccagatgcaaaagct | SEQ ID NO:120 |
| | HPA-4bc | acccagatgcaaaagctcac | SEQ ID NO:121 |
| | HPA-4bd | cagatgcaaaagctcacca | SEQ ID NO:122 |
| | HPA-4be | gatgcaaaagctcaccagtaa | SEQ ID NO:123 |
| | HPA-4ba CR | ttactggtgagcttttgcatc | SEQ ID NO:124 |
| | HPA-4bb CR | tggtgagcttttgcatctg | SEQ ID NO:125 |
| | HPA-4bc CR | gtgagcttttgcatctgggt | SEQ ID NO:126 |
| | HPA-4bd CR | agcttttgcatctgggtgg | SEQ ID NO:127 |
| | HPA-4be CR | cttttgcatctgggtggc | SEQ ID NO:128 |
| HPA5-allele a: | | | |
|---|---|---|---|
| | HPA-5aa | gagtctacctgtttactatcaaagagg | SEQ ID NO:129 |
| | HPA-5ab | agtctacctgtttactatcaaagaggta | SEQ ID NO:130 |
| | HPA-5ac | gtctacctgtttactatcaaagaggtaa | SEQ ID NO:131 |
| | HPA-5ad | ctacctgtttactatcaaagaggtaaaa | SEQ ID NO:132 |
| | HPA-5ae | acctgtttactatcaaagaggtaaaaa | SEQ ID NO:133 |
| | HPA-5aa CR | tttttacctctttgatagtaaacaggt | SEQ ID NO:134 |
| | HPA-5ab CR | ttttacctctttgatagtaaacaggtag | SEQ ID NO:135 |
| | HPA-5ac CR | ttacctctttgatagtaaacaggtagac | SEQ ID NO:136 |
| | HPA-5ad CR | tacctctttgatagtaaacaggtagact | SEQ ID NO:137 |
| | HPA-5ae CR | cctctttgatagtaaacaggtagactc | SEQ ID NO:138 |
| HPA5-allele b: | | | |
|---|---|---|---|
| | HPA-5ba | gagtctacctgtttactatcaaaaagg | SEQ ID NO:139 |
| | HPA-5bb | agtctacctgtttactatcaaaaaggta | SEQ ID NO:140 |
| | HPA-5bc | gtctacctgtttactatcaaaaaggtaa | SEQ ID NO:141 |
| | HPA-5ad | ctacctgtttactatcaaaaaggtaaaa | SEQ ID NO:142 |
| | HPA-5be | acctgtttactatcaaaaaggtaaaaa | SEQ ID NO:143 |
| | HPA-5ba CR | tttttacctttttgatagtaaacaggt | SEQ ID NO:144 |
| | HPA-5bb CR | ttttacctttttgatagtaaacaggtag | SEQ ID NO:145 |
| | HPA-5bc CR | ttacctttttgatagtaaacaggtagac | SEQ ID NO:146 |
| | HPA-5bd CR | tacctttttgatagtaaacaggtagact | SEQ ID NO:147 |
| | BPA-5be CR | cctttttgatagtaaacaggtagactc | SEQ ID NO:148 |
| HPA15-allele a: | | | |
|---|---|---|---|
| | Gov-aa | ttattatcttgacttcagttacaggattt | SEQ ID NO:149 |
| | Gov-ab | tcftgactteagtta-caggatttacc | SEQ ID NO:150 |
| | Gov-ac | tgacttcagttacaggatttaccaa | SEQ ID NO:151 |
| | Gov-ad | cttcagtfacaggatttaccaagaat | SEQ ID NO:152 |
| | Gov-ae | cagttacaggatttaccaagaatttg | SEQ ID NO:153 |
| | Gov-aa CR | caaattcttggtaaatcctgtaactg | SEQ ID NO:154 |
| | Gov-ab CR | attcttggtaaatcctgtaactgaag | SEQ ID NO:155 |
| | Gov-ac CR | tggtaaatcctgtaactgaagtcaa | SEQ ID NO:156 |
| | Gov-ad CR | ggtaaatcctgtaactgaagtcaaga | SEQ ID NO:157 |
| | Gov-ae CR | aaatcctgtaactgaagtcaagataataa | SEQ ID NO:158 |
| HPA15-allele b: | | | |
|---|---|---|---|
| | Gov-ba | tatcttgacttcagttccaggatt | SEQ ID NO:159 |
| | Gov-bb | cttgacttcagttccaggatttac | SEQ ID NO:160 |
| | Gov-bc | gacttcagttccaggatttacca | SEQ ID NO:161 |
| | Gov-bd | ttcagttccaggatttaccaag | SEQ ID NO:162 |
| | Gov-be | cagttccaggatttaccaagaatt | SEQ ID NO:163 |
| | Gov-ba CR | aattcttggtaaatcctggaactg | SEQ ID NO:164 |
| | Gov-bb CR | cttggtaaatcctggaactgaa | SEQ ID NO:165 |
| | Gov-bc CR | ggtaaatcctggaactgaagtca | SEQ ID NO:166 |
| | Gov-bd CR | gtaaatcctggaactgaagtcaag | SEQ ID NO:167 |
| | Gov-be CR | aatcctggaactgaagtcaagata. | SEQ ID NO:168 |
| Colton-a-allele: | | | |
|---|---|---|---|
| | Co.a.1 | aacaaccagacggcggt | SEQ ID NO:191 |
| | Co.a.2 | accagacggcggtccag | SEQ ID NO:192 |
| | Co.a.3 | cagacggcggtccagga | SEQ ID NO:193 |
| | Co.a.4 | gacggcggtccaggacaa | SEQ ID NO:194 |
| | Co.a.5 | cggcggtccaggacaac | SEQ ID NO:195 |
| | Co.a.1.cr | gttgtcctggaccgccgt | SEQ ID NO:196 |
| | Co.a.2.cr | tcctggaccgccgtctg | SEQ ID NO:197 |
| | Co.a.3.cr | ctggaccgccgtctggt | SEQ ID NO:198 |
| | Co.a.4.cr | ggaccgccgtctggttg | SEQ ID NO:199 |
| | Co.a.5.cr | accgccgtctggttgtt | SEQ ID NO:200 |
| Colton-b-allele: | | | |
|---|---|---|---|
| | Co.b.1 | ggaacaaccagacggtggt | SEQ ID NO:201 |
| | Co.b.2 | acaaccagacggtggtccag | SEQ ID NO:202 |
| | Co.b.3 | accagacggtggtccagga | SEQ ID NO:203 |
| | Co.b.4 | gacggtggtccaggacaacg | SEQ ID NO:204 |
| | Co.b.5 | cggtggtccaggacaacg | SEQ ID NO:205 |
| | Co.b.1.cr | cgttgtcctggaccaccgt | SEQ ID NO:206 |
| | Co.b.2.cr | ttgtcctggaccaccgtctg | SEQ ID NO:207 |
| | Co.b.3.cr | ctggaccaccgtctggttgt | SEQ ID NO:208 |
| | Co.b.4.cr | ggaccaccgtctggttgttc | SEQ ID NO:209 |
| | Co.b.5.cr | accaccgtctggttgttcc | SEQ ID NO:210 |
| Diego-a-allele: | | | |
|---|---|---|---|
| | Di.a.1 | gtgaagtccacgccggc | SEQ ID NO:211 |
| | Di.a.2 | gaagtccacgccggcct | SEQ ID NO:212 |
| | Di.a.3 | agtccacgccggcctcc | SEQ ID NO:213 |
| | Di.a.4 | tccacgccggcctccct | SEQ ID NO:214 |
| | Di.a.5 | acgccggcctccctggcc | SEQ ID NO:215 |
| | Di.a.1.cr | gccagggaggccggcgt | SEQ ID NO:216 |
| | Di.a.2.cr | agggaggccggcgtgga | SEQ ID NO:217 |
| | Di.a.3.cr | ggaggccggcgtggact | SEQ ID NO:218 |
| | Di.a.4.cr | aggccggcgtggacttc | SEQ ID NO:219 |
| | Di.a.5.cr | ggccggcgtggacttca | SEQ ID NO:220 |
| Diego-b-allele: | | | |
|---|---|---|---|
| | Di.b.1 | ggtgaagtccacgctggc | SEQ ID NO:221 |
| | Di.b.2 | gtgaagtccacgctggcct | SEQ ID NO:222 |
| | Di.b.3 | tgaagtccacgctggcctcc | SEQ ID NO:223 |
| | Di.b.4 | gaagtccacgctggcctccct | SEQ ID NO:224 |
| | Di.b.5 | acgctggcctccctggccc | SEQ ID NO:225 |
| | Di.b.1.cr | ggccagggaggccagcgt | SEQ ID NO:226 |
| | Di.b.2.cr | cagggaggccagcgtgga | SEQ ID NO:227 |
| | Di.b.3.cr | agggaggccagcgtggact | SEQ ID NO:228 |
| | Di.b.4.cr | ggaggccagcgtggacttc | SEQ ID NO:229 |
| | Di.b.5.cr | ggccagcgtggacttcacc | SEQ ID NO:230 |
| Diego Wr-a-allele: | | | |
|---|---|---|---|
| | Wr.a.1 | tgggcttgcgttccaagt | SEQ ID NO:231 |
| | Wr.a.2 | ggcttgcgttccaagtttc | SEQ ID NO:232 |
| | Wr.a.3 | ttgcgttccaagtttccca | SEQ ID NO:233 |
| | Wr.a.4 | cgttccaagtttcccatct | SEQ ID NO:234 |
| | Wr.a.5 | tccaagtttcccatctgga | SEQ ID NO:235 |
| | Wr.a.1 CR | tccagatgggaaacttgga | SEQ ID NO:236 |
| | Wr.a.2 CR | agatgggaaacttggaacg | SEQ ID NO:237 |
| | Wr.a.3 CR | tgggaaacttggaacgcaa | SEQ ID NO:238 |
| | Wr.a.4 CR | gaaacttggaacgcaagcc | SEQ ID NO:239 |
| | Wr.a.5 CR | acttggaacgcaagccca | SEQ ID NO:240 |
| Diego Wr-b-allele: | | | |
|---|---|---|---|
| | Wr.b.1 | gggcttgcgttccgagtt | SEQ ID NO:241 |
| | Wr.b.2 | gcttgcgttccgagtttc | SEQ ID NO:242 |
| | Wr.b.3 | ttgcgttccgagtttccc | SEQ ID NO:243 |
| | Wr.b.4 | cgttccgagtttcccatc | SEQ ID NO:244 |
| | Wr.b.5 | tccgagtttcccatctgg | SEQ ID NO:245 |
| | Wr.b.1 CR | ccagatgggaaactcgga | SEQ ID NO:246 |
| | Wr.b.2 CR | gatgggaaactcggaacg | SEQ ID NO:247 |
| | Wr.b.3 CR | gggaaactcggaacgcaa | SEQ ID NO:248 |
| | Wr.b.4 CR | gaaactcggaacgcaagc | SEQ ID NO:249 |
| | Wr.b.5 CR | aactcggaacgcaagccc | SEQ ID NO:250 |
| Dombrock-a-allele: | | | |
|---|---|---|---|
| | Do.a.1 | taccacccaagaggaaact | SEQ ID NO:251 |
| | Do.a.2 | ccacccaagaggaaactgg | SEQ ID NO:252 |
| | Do.a.3 | acccaagaggaaactggttg | SEQ ID NO:253 |
| | Do.a.4 | caagaggaaactggttgca | SEQ ID NO:254 |
| | Do.a.5 | aggaaactggttgcagttga | SEQ ID NO:255 |
| | Do a 6 cr | ctcaactgcaaccagtttcc | SEQ ID NO:256 |
| | Do a 7 cr | caactgcaaccagtttcctc | SEQ ID NO:257 |
| | Do a 8 cr | tgcaaccagtttcctcttgg | SEQ ID NO:258 |
| | Do a 9 cr | accagtttcctcttgggtgg | SEQ ID NO:259 |
| | Do a 10 cr | cagtttcctcttgggtggta | SEQ ID NO:260 |
| Dombrock-b-allele: | | | |
|---|---|---|---|
| | Do.b.1 | taccacccaagaggagact | SEQ ID NO:261 |
| | Do.b.2 | ccacccaagaggagactgg | SEQ ID NO:262 |
| | Do.b.3 | acccaagaggagactggttg | SEQ ID NO:263 |
| | Do.b.4 | caagaggagactggttgca | SEQ ID NO:264 |
| | Do.b.5 | aggagactggttgcagttga | SEQ ID NO:265 |
| | Do b 6 cr | ctcaactgcaaccagtctcc | SEQ ID NO:266 |
| | Do b 7 cr | caactgcaaccagtctcctc | SEQ ID NO:267 |
| | Do b 8 cr | tgcaaccagtctcctcttgg | SEQ ID NO:268 |
| | Do b 9 er | accagtctcctcttgggtgg | SEQ ID NO:269 |
| | Do b 10 cr | cagtctcctcttgggtggt | SEQ ID NO:270 |
| Dombrock-Joseph(a) positive allele: | | | |
|---|---|---|---|
| | Jo.a.pos.1 | ccccagaacatgactaccac | SEQ ID NO:271 |
| | Jo.a.pos.2 | ccagaacatgactaccacaca | SEQ ID NO:272 |
| | Jo.a.pos.3 | agaacatgactaccacacacgc | SEQ ID NO:273 |
| | Jo.a.pos.4 | catgactaccacacacgctgt | SEQ ID NO:274 |
| | Jo.a.pos.5 | actaccacacacgctgtgg | SEQ ID NO:275 |
| | Jo.a.pos.1.cr | gccacagcgtgtgtggtagt | SEQ ID NO:276 |
| | Jo.a.pos.2.cr | acagcgtgtgtggtagtcatg | SEQ ID NO:277 |
| | Jo.a.pos.3.cr | agcgtgtgtggtagtcatgtt | SEQ ID NO:278 |
| | Jo.a.pos.4.cr | cgtgtgtggtagtcatgttctg | SEQ ID NO:279 |
| | Jo.a.pos.5.cr | gtggtagtcatgttctgggg | SEQ ID NO:280 |
| Dombrock-Joseph(a) negative allele: | | | |
|---|---|---|---|
| | Jo.a.neg.1 | ctaccccagaacatgactatcac | SEQ ID NO:281 |
| | Jo.a.neg.2 | cccagaacatgactatcacaca | SEQ ID NO:282 |
| | Jo.a.neg.3 | cagaacatgactatcacacacgc | SEQ ID NO:283 |
| | Jo.a.neg.4 | catgactatcacacacgctgtg | SEQ ID NO:284 |
| | Jo.a.neg.5 | actatcacacacgctgtggc | SEQ ID NO:285 |
| | Jo.a.neg.1.cr | aatagccacagcgtgtgtgatagt | SEQ ID NO:286 |
| | Jo.a.neg.2.cr | cacagcgtgtgtgatagtcatg | SEQ ID NO:287 |
| | Jo.a.neg.3.cr | cagcgtgtgtgatagtcatgtt | SEQ ID NO:288 |
| | Jo.a.neg.4.cr | cgtgtgtgatagtcatgttctgg | SEQ ID NO:289 |
| | Jo.a.neg.5.cr | gtgatagtcatgttctggggtag | SEQ ID NO:290 |
| Duffy-a-allele: | | | |
|---|---|---|---|
| | Fy.A.1 | ccagatggagactatggtgcc | SEQ ID NO:291 |
| | Fy.A.2 | atggagactatggtgccaac | SEQ ID NO:292 |
| | Fy.A.3 | ggagactatggtgccaacctg | SEQ ID NO:293 |
| | Fy.A.4 | gactatggtgccaacctgga | SEQ ID NO:294 |
| | Fy.A.5 | tatggtgccaacctggaag | SEQ ID NO:295 |
| | Fy.A.1.cr | cttccaggttggcaccata | SEQ ID NO:296 |
| | Fy.A.2.cr | tccaggttggcaccatagtc | SEQ ID NO:297 |
| | Fy.A.3.cr | aggttggcaccatagtctcc | SEQ ID NO:298 |
| | Fy.A.4.cr | gttggcaccatagtctccat | SEQ ID NO:299 |
| | Fy.A.5.cr | gcaccatagtctccatctgg | SEQ ID NO:300 |
| Duffy-b-allele: | | | |
|---|---|---|---|
| | Fy.B.1 | cccagatggagactatgatgcc | SEQ ID NO:301 |
| | Fy.B.2 | gatggagactatgatgccaac | SEQ ID NO:302 |
| | Fy.B.3 | tggagactatgatgccaacctg | SEQ ID NO:303 |
| | Fy.B.4 | gactatgatgccaacctggaa | SEQ ID NO:304 |
| | Fy.B.5 | tatgatgccaacctggaagc | SEQ ID NO:305 |
| | Fy.B.1.cr | gcttccaggttggcatcata | SEQ ID NO:306 |
| | Fy.B.2.cr | ttccaggttggcatcatagtc | SEQ ID NO:307 |
| | Fy.B.3.cr | caggttggcatcatagtctcc | SEQ ID NO:308 |
| | Fy.B.4.cr | gttggcatcatagtctccatc | SEQ ID NO:309 |
| | Fy.B.5.cr | gcatcatagtctccatctggg | SEQ ID NO:310 |
| Duffy GATAbox-normal-allele: | | | |
|---|---|---|---|
| | Fy.GATA.normal.1 | agtccttggctcttatcttg | SEQ ID NO:311 |
| | Fy.GATA.normal.2 | agtccttggctcttatcttgga | SEQ ID NO:312 |
| | Fy.GATA.normal.3 | cttggctcttatcttggaagc | SEQ ID NO:313 |
| | Fy.GATA.normal.4 | gctcttatcttggaagcacagg | SEQ ID NO:314 |
| | Fy.GATA.normal.5 | cttatcttggaagcacaggcgc | SEQ ID NO:315 |
| | Fy.GATA.normal.1.cr | gcctgtgcttccaagataag | SEQ ID NO:316 |
| | Fy.GATA.normal.2.cr | tgtgcttccaagataagagc | SEQ ID NO:317 |
| | Fy.GATA.normal.3.cr | tgcttccaagataagagcca | SEQ ID NO:318 |
| | Fy.GATA.normal.4.cr | ttccaagataagagccaagga | SEQ ID NO:319 |
| | Fy.GATA.normal.5.cr | caagataagagccaaggact | SEQ ID NO:320 |
| Duffy GATAbox-mutation-allele: | | | |
|---|---|---|---|
| | Fy.GATA.mut.1 | tccttggctcttaccttg | SEQ ID NO:321 |
| | Fy.GATA.mut.2 | tccttggctcttaccttgga | SEQ ID NO:322 |
| | Fy.GATA.mut.3 | tggctcttaccttggaagc | SEQ ID NO:323 |
| | Fy.GATA.mut.4 | gctcttaccttggaagcacag | SEQ ID NO:324 |
| | Fy.GATA.mut.5 | cttaccttggaagcacaggcg | SEQ ID NO:325 |
| | Fy.GATA.mut.1.cr | cctgtgcttccaaggtaag | SEQ ID NO:326 |
| | Fy.GATA.mut.2.cr | gtgcttccaaggtaagagc | SEQ ID NO:327 |
| | Fy.GATA.mut.3.cr | gcttccaaggtaagagcca | SEQ ID NO:328 |
| | Fy.GATA.mut.4.cr | ttccaaggtaagagccaagg | SEQ ID NO:329 |
| | Fy.GATA.mut.5.cr | caaggtaagagccaagga | SEQ ID NO:330 |
| Duffy Fyx-normal-allele: | | | |
|---|---|---|---|
| | Fy.X.(b).1 | ttttcagacctctcttccgct | SEQ ID NO:331 |
| | Fy.X.(b).2 | agacctctcttccgctggc | SEQ ID NO:332 |
| | Fy.X.(b).3 | ctctcttccgctggcagc | SEQ ID NO:333 |
| | Fy.X.(b).4 | ctcttccgctggcagctc | SEQ ID NO:334 |
| | Fy.X.(b).5 | cttccgctggcagctctg | SEQ ID NO:335 |
| | Fy.X.(b).1.cr | cagagctgccagcggaa | SEQ ID NO:336 |
| | Fy.X.(b).2.cr | agctgccagcggaagag | SEQ ID NO:337 |
| | Fy.X.(b).3.cr | ctgccagcggaagagagg | SEQ ID NO:338 |
| | Fy.X.(b).4.cr | gccagcggaagagaggtc | SEQ ID NO:339 |
| | Fy.X.(b).5.cr | cagcggaagagaggtctg | SEQ ID NO:340 |
| Duffy Fyx-mutation-allele: | | | |
|---|---|---|---|
| | Fy.X.1 | gcttttcagacctctcttctgct | SEQ ID NO:341 |
| | Fy.X.2 | tcagacctctcttctgctggc | SEQ ID NO:342 |
| | Fy.X.3 | acctctcttctgctggcagc | SEQ ID NO:343 |
| | Fy.X.4 | ctcttctgctggcagctctg | SEQ ID NO:344 |
| | Fy.X.5 | cttctgctggcagctctgc | SEQ ID NO:345 |
| | Fy.X.1.cr | gcagagctgccagcagaa | SEQ ID NO:346 |
| | Fy.X.2.cr | gagctgccagcagaagagag | SEQ ID NO:347 |
| | Fy.X.3.cr | agctgccagcagaagagagg | SEQ ID NO:348 |
| | Fy.X.4.cr | gccagcagaagagaggtctg | SEQ ID NO:349 |
| | Fy.X.5.cr | cagcagaagagaggtctgaaa | SEQ ID NO:350 |
| Kidd-allele-a: | | | |
|---|---|---|---|
| | Jk.a.1 | cagccccatttgaggaca | SEQ ID NO:351 |
| | Jk.a.2 | gccccatttgaggacatcta | SEQ ID NO:352 |
| | Jk.a.3 | ccatttgaggacatctactttg | SEQ ID NO:353 |
| | Jk.a.4 | atttgaggacatctactttgga | SEQ ID NO:354 |
| | Jk.a.5 | gaggacatctactttggactct | SEQ ID NO:355 |
| | Jk.a.1.cr | cagagtccaaagtagatgtcctc | SEQ ID NO:356 |
| | Jk.a.2.cr | agtccaaagtagatgtcctcaaa | SEQ ID NO:357 |
| | Jk.a.3.cr | aaagtagatgtcctcaaatggg | SEQ ID NO:358 |
| | Jk.a.4.cr | tagatgtcctcaaatggggc | SEQ ID NO:359 |
| | Jk.a.5.cr | atgtcctcaaatggggctg | SEQ ID NO:360 |
| Kidd-allele-b: | | | |
|---|---|---|---|
| | Jk.b.1 | tcagccccatttgagaaca | SEQ ID NO:361 |
| | Jk.b.2 | gccccatttgagaacatcta | SEQ ID NO:362 |
| | Jk.b.3 | cccatttgagaacatctactttg | SEQ ID NO:363 |
| | Jk.b.4 | atttgagaacatctactttggac | SEQ ID NO:364 |
| | Jk.b.5 | gagaacatctactttggactctg | SEQ ID NO:365 |
| | Jk.b.1.cr | ccagagtccaaagtagatgttctc | SEQ ID NO:366 |
| | Jk.b.2.cr | agagtccaaagtagatgttctcaaa | SEQ ID NO:367 |
| | Jk.b.3.cr | ccaaagtagatgttctcaaatgg | SEQ ID NO:368 |
| | Jk.b.4.cr | agtagatgttctcaaatggggc | SEQ ID NO:369 |
| | Jk.b.5.cr | atgttctcaaatggggctga | SEQ ID NO:370 |
| Kell-K1-allele: | | | |
|---|---|---|---|
| | KEL.1.1 | tccttaaactttaaccgaatgct | SEQ ID NO:371 |
| | KEL.1.2 | ttaaactttaaccgaatgctgaga | SEQ ID NO:372 |
| | KEL.1.3 | aactttaaccgaatgctgagactt | SEQ ID NO:373 |
| | KEL.1.4 | aaccgaatgctgagacttctg | SEQ ID NO:374 |
| | KEL.1.5 | cgaatgctgagacttctgatgag | SEQ ID NO:375 |
| | KEL 1.6 CR | actcatcagaagtctcagcattc | SEQ ID NO:376 |
| | KEL 1.7 CR | tcagaagtctcagcattcggt | SEQ ID NO:377 |
| | KEL 1.8 CR | aagtctcagcattcggttaaag | SEQ ID NO:378 |
| | KEL 1.9 CR | tctcagcattcggttaaagtttaa | SEQ ID NO:379 |
| | KEL 1.10 CR | agcattcggttaaagtttaagga | SEQ ID NO:380 |
| Kell-K2-allele: | | | |
|---|---|---|---|
| | KEL.2.1 | ccttaaactttaaccgaacgct | SEQ ID NO:381 |
| | KEL.2.2 | aactttaaccgaacgctgaga | SEQ ID NO:382 |
| | KEL.2.3 | ctttaaccgaacgctgagactt | SEQ ID NO:383 |
| | KEL.2.4 | aaccgaacgctgagacttct | SEQ ID NO:384 |
| | KEL.2.5 | cgaacgctgagacttctgatg | SEQ ID NO:385 |
| | KEL 2.6 CR | tcatcagaagtctcagcgttc | SEQ ID NO:386 |
| | KEL 2.7 CR | cagaagtctcagcgttcggt | SEQ ID NO:387 |
| | KEL 2.8 CR | agtctcagcgttcggttaaag | SEQ ID NO:388 |
| | KEL 2.9 CR | tctcagcgttcggttaaagtt | SEQ ID NO:389 |
| | KEL 2.10 CR | agcgttcggttaaagtttaagg | SEQ ID NO:390 |
| Kell-K3-allele: | | | |
|---|---|---|---|
| | KEL.3.1 | aatctccatcacttcatggct | SEQ ID NO:391 |
| | KEL.3.2 | tccatcacttcatggctgtt | SEQ ID NO:392 |
| | KEL.3.3 | atcacttcatggctgttcca | SEQ ID NO:393 |
| | KEL.3.4 | acttcatggctgttccagtt | SEQ ID NO:394 |
| | KEL.3.5 | tcatggctgttccagtttc | SEQ ID NO:395 |
| | KEL.3.1.cr | agaaactggaacagccatgaa | SEQ ID NO:396 |
| | KEL.3.2.cr | aactggaacagccatgaagtg | SEQ ID NO:397 |
| | KEL.3.3.cr | tggaacagccatgaagtgatg | SEQ ID NO:398 |
| | KEL.3.4.cr | acagccatgaagtgatggag | SEQ ID NO:399 |
| | KEL.3.5.cr | gccatgaagtgatggagatt | SEQ ID NO:400 |
| Kell-K4-allele: | | | |
|---|---|---|---|
| | KEL.4.1 | tctccatcacttcacggct | SEQ ID NO:401 |
| | KEL.4.2 | ccatcacttcacggctgtt | SEQ ID NO:402 |
| | KEL.4.3 | cacttcacggctgttcca | SEQ ID NO:403 |
| | KEL.4.4 | acttcacggctgttccag | SEQ ID NO:404 |
| | KEL.4.5 | tcacggctgttccagttt | SEQ ID NO:405 |
| | KEL.4.1.cr | aaactggaacagccgtgaa | SEQ ID NO:406 |
| | KEL.4.2.cr | ctggaacagccgtgaagtg | SEQ ID NO:407 |
| | KEL.4.3.cr | ggaacagccgtgaagtgatg | SEQ ID NO:408 |
| | KEL.4.4.cr | acagccgtgaagtgatgg | SEQ ID NO:409 |
| | KEL.4.5.cr | gccgtgaagtgatggaga | SEQ ID NO:410 |
| Kell-K6-allele: | | | |
|---|---|---|---|
| | KEL.6.1 | tactgcctgggggctgccccgcc | SEQ ID NO:411 |
| | KEL.6.2 | tgggggctgccccgcctgt | SEQ ID NO:412 |
| | KEL.6.3 | gctgccccgcctgtgac | SEQ ID NO:413 |
| | KEL.6.4 | ctgccccgcctgtgacaa | SEQ ID NO:414 |
| | KEL.6.5 | gccccgcctgtgacaac | SEQ ID NO:415 |
| | KEL.6.1.cr | gttgtcacaggcggggc | SEQ ID NO:416 |
| | KEL.6.2.cr | ttgtcacaggcggggcag | SEQ ID NO:417 |
| | KEL.6.3.cr | tcacaggcggggcagccc | SEQ ID NO:418 |
| | KEL.6.4.cr | acaggcggggcagccccca | SEQ ID NO:419 |
| | KEL.6.5.cr | aggcggggcagccccca | SEQ ID NO:420 |
| Kell-K7-allele: | | | |
|---|---|---|---|
| | KEL.7.1 | actgcctgggggctgcctcgcc | SEQ ID NO:421 |
| | KEL.7.2 | cctgggggctgcctcgcctgt | SEQ ID NO:422 |
| | KEL.7.3 | ggctgcctcgcctgtgac | SEQ ID NO:423 |
| | KEL.7.4 | ctgcctcgcctgtgacaacc | SEQ ID NO:424 |
| | KEL.7.5 | gcctcgcctgtgacaacc | SEQ ID NO:425 |
| | KEL.7.1.cr | ggttgtcacaggcgaggc | SEQ ID NO:426 |
| | KEL.7.2.cr | ggttgtcacaggcgaggcag | SEQ ID NO:427 |
| | KEL.7.3.cr | ttgtcacaggcgaggcagccc | SEQ ID NO:428 |
| | KEL.7.4.cr | acaggcgaggcagcccccagg | SEQ ID NO:429 |
| | KEL.7.5.cr | aggcgaggcagcccccagg | SEQ ID NO:430 |
| Lutheran a-allele: | | | |
|---|---|---|---|
| | Lu.a.1 | ggagctcgcccccgcct | SEQ ID NO:431 |
| | Lu.a.2 | gctcgcccccgcctagc | SEQ ID NO:432 |
| | Lu.a.3 | cgcccccgcctagcctc | SEQ ID NO:433 |
| | Lu.a.4 | cccccgcctagcctcgg | SEQ ID NO:434 |
| | Lu.a.5 | cccgcctagcctcggct | SEQ ID NO:435 |
| | Lu.a.1 CR | agccgaggctaggcggg | SEQ ID NO:436 |
| | Lu.a.2 CR | ccgaggctaggcggggg | SEQ ID NO:437 |
| | Lu.a.3 CR | gaggctaggcgggggcg | SEQ ID NO:438 |
| | Lu.a.4 CR | gctaggcgggggcgagc | SEQ ID NO:439 |
| | Lu.a.5 CR | aggcgggggcgagctcc | SEQ ID NO:440 |
| Lutheran b-allele: | | | |
|---|---|---|---|
| | Lu.b.1 | gggagctcgcccccacct | SEQ ID NO:441 |
| | Lu.b.2 | gagctcgcccccacctagc | SEQ ID NO:442 |
| | Lu.b.3 | ctcgcccccacctagcctc | SEQ ID NO:443 |
| | Lu.b.4 | cccccacctagcctcggct | SEQ ID NO:444 |
| | Lu.b.5 | cccacctagcctcggctga | SEQ ID NO:445 |
| | Lu.b.1 CR | tcagccgaggctaggtggg | SEQ ID NO:446 |
| | Lu.b.2 CR | agccgaggctaggtggggg | SEQ ID NO:447 |
| | Lu.b.3 CR | gaggctaggtgggggcgag | SEQ ID NO:448 |
| | Lu.b.4 CR | gctaggtgggggcgagctc | SEQ ID NO:449 |
| | Lu.b.5 CR | aggtgggggcgagctccc | SEQ ID NO:450 |
| MNS M-allele: | | | |
|---|---|---|---|
| | M.1 | gtgagcatatcagcatcaag | SEQ ID NO:451 |
| | M.2 | atcagcatcaagtaccactgg | SEQ ID NO:452 |
| | M.3 | catcaagtaccactggtgtg | SEQ ID NO:453 |
| | M.4 | taccactggtgtggcaatgc | SEQ ID NO:454 |
| | M.5 | ctggtgtggcaatgcaca | SEQ ID NO:455 |
| | M.1.cr | tgtgcattgccacaccagt | SEQ ID NO:456 |
| | M.2.cr | gcattgccacaccagtggta | SEQ ID NO:457 |
| | M.3.cr | ccacaccagtggtacttgatg | SEQ ID NO:458 |
| | M.4.cr | accagtggtacttgatgct | SEQ ID NO:459 |
| | M.5.cr | cttgatgctgatatgctcac | SEQ ID NO:460 |
| MNS N-allele: | | | |
|---|---|---|---|
| | N.1 | tgtgagcatatcagcattaag | SEQ ID NO:461 |
| | N.2 | atcagcattaagtaccactgagg | SEQ ID NO:462 |
| | N.3 | cattaagtaccactgaggtgg | SEQ ID NO:463 |
| | N.4 | accactgaggtggcaatgc | SEQ ID NO:464 |
| | N.5 | ctgaggtggcaatgcacact | SEQ ID NO:465 |
| | N.1.cr | gtgtgcattgccacctcagt | SEQ ID NO:466 |
| | N.2.cr | gcattgccacctcagtggta | SEQ ID NO:467 |
| | N.3.cr | ccacctcagtggtacttaatgc | SEQ ID NO:468 |
| | N.4.cr | cctcagtggtacttaatgct | SEQ ID NO:469 |
| | N.5.cr | cttaatgctgatatgctcaca | SEQ ID NO:470 |
| MNS S-allele: | | | |
|---|---|---|---|
| | big.S.1 | tttgctttataggagaaatggga | SEQ ID NO:471 |
| | big.S.2 | ctttataggagaaatgggaca | SEQ ID NO:472 |
| | big.S.3 | ttataggagaaatgggacaacttg | SEQ ID NO:473 |
| | big.S.4 | gagaaatgggacaacttgtcc | SEQ ID NO:474 |
| | big.S.5 | aaatgggacaacttgtccatc | SEQ ID NO:475 |
| | big.S.1.cr | gatggacaagttgtcccattt | SEQ ID NO:476 |
| | big.S.2.cr | gacaagttgtcccatttctcc | SEQ ID NO:477 |
| | big.S.3.cr | aagttgtcccatttctcctata | SEQ ID NO:478 |
| | bigS.4.cr | tgtcccatttctcctataaagca | SEQ ID NO:479 |
| | big.S.5.cr | cccatttctcctataaagcaaaa | SEQ ID NO:480 |
| MNS s-allele: | | | |
|---|---|---|---|
| | little.s.1 | tgctttataggagaaacggga | SEQ ID NO:481 |
| | little.s.2 | tttataggagaaacgggaca | SEQ ID NO:482 |
| | little.s.3 | ggagaaacgggacaacttg | SEQ ID NO:483 |
| | little.s.4 | gagaaacgggacaacttgtc | SEQ ID NO:484 |
| | little.s.5 | aaacgggacaacttgtccat | SEQ ID NO:485 |
| | little.s.1.cr | tggacaagttgtcccgttt | SEQ ID NO:486 |
| | little.s.2.cr | acaagttgtcccgtttctcc | SEQ ID NO:487 |
| | little.s.3.cr | agttgtcccgtttctcctata | SEQ ID NO:488 |
| | little.s.4.cr | tgtcccgtttctcctataaagc | SEQ ID NO:489 |
| | little.s.5.cr | cccgtttctcctataaagca | SEQ ID NO:490 |
| MNS U-positive-allele: | | | |
|---|---|---|---|
| | U.pos.1 | ttgctgctctctttagctcc | SEQ ID NO:491 |
| | U.pos.2 | ctctctttagctcctgtagtgat | SEQ ID NO:492 |
| | U.pos.3 | agctcctgtagtgataatactca | SEQ ID NO:493 |
| | U.pos.4 | gtagtgataatactcattatttttg | SEQ ID NO:494 |
| | U.pos.5 | taatactcattatttttggggtg | SEQ ID NO:495 |
| | U.pos.1.cr | caccccaaaaataatgagtatta | SEQ ID NO:496 |
| | U.pos.2.cr | caaaaataatgagtattatcactaca | SEQ ID NO:497 |
| | U.pos.3.cr | agtattatcactacaggagctaaa | SEQ ID NO:498 |
| | U.pos.4.cr | atcactacaggagctaaagag | SEQ ID NO:499 |
| | U.pos.5.cr | gagctaaagagagcagcaaa | SEQ ID NO:500 |
| MNS U-negative-allele: | | | |
|---|---|---|---|
| | U.neg.1 | ttttgctgctctctttatctcc | SEQ ID NO:501 |
| | U.neg.2 | gctctctttatctcctgtagagat | SEQ ID NO:502 |
| | U.neg.3 | tatctcctgtagagataacactca | SEQ ID NO:503 |
| | U.neg.4 | gtagagataacactcattattttt | SEQ ID NO:504 |
| | U.neg.5 | taacactcattatttttggggt | SEQ ID NO:505 |
| | U.neg.1.cr | accccaaaaataatgagtgtta | SEQ ID NO:506 |
| | U.neg.2.cr | aaaaataatgagtgttatctctaca | SEQ ID NO:507 |
| | U.neg.3.cr | agtgttatctctacaggagataaa | SEQ ID NO:508 |
| | U.neg.4.cr | atctctacaggagataaagagag | SEQ ID NO:509 |
| | U.neg.5.cr | gagataaagagagcagcaaaatta | SEQ ID NO:510 |
| Rhesus C-allele(307T): | | | |
|---|---|---|---|
| | Rh.big.C.1 | tgagccagttcccttctgg | SEQ ID NO:511 |
| | Rh.big.C.2 | gagccagttcccttctgg | SEQ ID NO:512 |
| | Rh.big.C.3 | ctgagccagttcccttctg | SEQ ID NO:513 |
| | Rh.big.C.4 | ccttctgggaaggtggtc | SEQ ID NO:514 |
| | Rh.big.C.5 | ccttctgggaaggtggtca | SEQ ID NO:515 |
| | Rh.big.C.1.cr | tgaccaccttcccagaagg | SEQ ID NO:516 |
| | Rh.big.C.2.cr | gaccaccttcccagaagg | SEQ ID NO:517 |
| | Rh.big.C.3.cr | ccagaagggaactggctc | SEQ ID NO:518 |
| | Rh.big.C.4.cr | ccagaagggaactggctca | SEQ ID NO:519 |
| | Rh.big.C.5.cr | cagaagggaactggctcag | SEQ ID NO:520 |
| Rhesus c-allele(307C): | | | |
|---|---|---|---|
| | Rh.little.c.1 | gagccagttccctcctgg | SEQ ID NO:521 |
| | Rh.little.c.2 | agccagttccctcctgg | SEQ ID NO:522 |
| | Rh.little.c.3 | tgagccagttccctcctg | SEQ ID NO:523 |
| | Rh.little.c.4 | cctcctgggaaggtggt | SEQ ID NO:524 |
| | Rh.little.c.5 | cctcctgggaaggtggtc | SEQ ID NO:525 |
| | Rh.little.c.1.cr | gaccaccttcccaggagg | SEQ ID NO:526 |
| | Rh.little.c.2.cr | accaccttcccaggagg | SEQ ID NO:527 |
| | Rh.little.c.3.cr | ccaggagggaactggct | SEQ ID NO:528 |
| | Rh.little.c.4.cr | ccaggagggaactggctc | SEQ ID NO:529 |
| | Rh.little.c.5.cr | caggagggaactggctca | SEQ ID NO:530 |
| Rhesus BigC-intron2-specific-insert-allele: | | | |
|---|---|---|---|
| | RhC.intron.2.1 | agggtgccctttgtcacttc | SEQ ID NO:531 |
| | RhC.intron.2.2 | gccctttgtcacttcccagt | SEQ ID NO:532 |
| | RhC.intron.2.3 | cctttgtcacttcccagtgg | SEQ ID NO:533 |
| | RhC.intron.2.4 | ttgtcacttcccagtggtacaa | SEQ ID NO:534 |
| | RhC.intron.2.5 | tcacttcccagtggtacaatca | SEQ ID NO:535 |
| | RhC.intron.2.1.cr | gaagtgacaaagggcaccct | SEQ ID NO:536 |
| | RhC.intron.2.2.cr | actgggaagtgacaaagggc | SEQ ID NO:537 |
| | RhC.intron.2.3.cr | ccactgggaagtgacaaagg | SEQ ID NO:538 |
| | RhC.intron.2.4.cr | tgtaccactgggaagtgacaaa | SEQ ID NO:539 |
| | RhC.intron.2.5.cr | tgattgtaccactgggaagtga | SEQ ID NO:540 |
| Rhesus E-allele: | | | |
|---|---|---|---|
| | Rh.big.E.1 | gccaagtgtcaactctcctct | SEQ ID NO:541 |
| | Rh.big.E.2 | caagtgtcaactctcctctgct | SEQ ID NO:542 |
| | Rh.big.E.3 | gtgtcaactctcctctgctgag | SEQ ID NO:543 |
| | Rh.big.E.4 | caactctcctctgctgagaagtc | SEQ ID NO:544 |
| | Rh.big.E.5 | tctcctctgctgagaagtcc | SEQ ID NO:545 |
| | Rh.big.E.1.cr | ggacttctcagcagaggagag | SEQ ID NO:546 |
| | Rh.big.E.2.cr | cttctcagcagaggagagttga | SEQ ID NO:547 |
| | Rh.big.E.3.cr | ctcagcagaggagagttgacac | SEQ ID NO:548 |
| | Rh.big.E.4.cr | gcagaggagagttgacacttg | SEQ ID NO:549 |
| | Rh.big.E.5.cr | gaggagagttgacacttggc | SEQ ID NO:550 |
| Rhesus e-allele: | | | |
|---|---|---|---|
| | Rh.little.e.1 | gccaagtgtcaactctgctct | SEQ ID NO:551 |
| | Rh.little.e.2 | caagtgtcaactctgctctgct | SEQ ID NO:552 |
| | Rh.little.e.3 | tgtcaactctgctctgctgag | SEQ ID NO:553 |
| | Rh.little.e.4 | caactctgctctgctgagaag | SEQ ID NO:554 |
| | Rh.little.e.5 | tctgctctgctgagaagtcc | SEQ ID NO:555 |
| | Rh.little.e.1.cr | ggacttctcagcagagcagag | SEQ ID NO:556 |
| | Rh.little.e.2.cr | ttctcagcagagcagagttga | SEQ ID NO:557 |
| | Rh.little.e.3.cr | tcagcagagcagagttgacac | SEQ ID NO:558 |
| | Rh.little.e.4.cr | gcagagcagagttgacacttg | SEQ ID NO:559 |
| | Rh.little.e.5.cr | gagcagagttgacacttggc | SEQ ID NO:560 |
| Rhesus RHD-allele: | | | |
|---|---|---|---|
| | RhD 1 | ttccccacagctccatcat | SEQ ID NO:561 |
| | RhD 2 | acagctccatcatgggctac | SEQ ID NO:562 |
| | RhD 3 | tccatcatgggctacaacttc | SEQ ID NO:563 |
| | RhD 4 | tcatgggctacaacttcagct | SEQ ID NO:564 |
| | RhD 5 | gggctacaacttcagcttgct | SEQ ID NO:565 |
| | RhD cr 1 | agcaagctgaagttgtagccc | SEQ ID NO:566 |
| | RhD cr 2 | agctgaagttgtagcccatga | SEQ ID NO:567 |
| | RhD cr 3 | gaagttgtagcccatgatgg | SEQ ID NO:568 |
| | RhD cr 4 | gcccatgatggagctgt | SEQ ID NO:569 |
| | RhD cr 5 | tgatggagctgtggggaa | SEQ ID NO:570 |
| Rhesus r's-allele: | | | |
|---|---|---|---|
| | r's.T.1 | ggaaggtcaacttggtgca | SEQ ID NO:571 |
| | r's.T.2 | ggaaggtcaacttggtgcagt | SEQ ID NO:572 |
| | r's.T.3 | caacttggtgcagttggtg | SEQ ID NO:573 |
| | r's.T.4 | acttggtgcagttggtggt | SEQ ID NO:574 |
| | r's.T.5 | ttggtgcagttggtggtgat | SEQ ID NO:575 |
| | r's.T.1.cr | catcaccaccaactgcacca | SEQ ID NO:576 |
| | r's.T.2.cr | caccaccaactgcaccaagt | SEQ ID NO:577 |
| | r's.T.3.cr | accaactgcaccaagttgac | SEQ ID NO:578 |
| | r's.T.4.cr | actgcaccaagttgaccttcc | SEQ ID NO:579 |
| | r's.T.5.cr | tgcaccaagttgaccttcc | SEQ ID NO:580 |
| Rhesus DVI-allele: | | | |
|---|---|---|---|
| | Rh.DVI.1 | atttcaaccctcttggcctt | SEQ ID NO:581 |
| | Rh.DVI.2 | aaccctcttggcctttgttt | SEQ ID NO:582 |
| | Rh.DVI.3 | tcttggcctttgtttccttg | SEQ ID NO:583 |
| | Rh.DVI.4 | ggtatcagcttgagagctcg | SEQ ID NO:584 |
| | Rh.DVI.5 | atcagcttgagagctcggag | SEQ ID NO:585 |
| | Rh.DVI.1.cr | aaggccaagagggttgaaat | SEQ ID NO:586 |
| | Rh.DVI.2.cr | aaacaaaggccaagagggtt | SEQ ID NO:587 |
| | Rh.DVI.3.cr | caaggaaacaaaggccaaga | SEQ ID NO:588 |
| | Rh.DVI.4.cr | cgagctctcaagctgatacc | SEQ ID NO:589 |
| | Rh.DVI.5.cr | ctccgagctctcaagctgat | SEQ ID NO:590 |
| Rhesus RHD-Pseudogene-mutation-allele: | | | |
|---|---|---|---|
| | RhD Y 1 | tttctttgcagacttaggtgc | SEQ ID NO:591 |
| | RhDY2 | ctttgcagacttaggtgcaca | SEQ ID NO:592 |
| | RhDY3 | tttgcagacttaggtgcacagt | SEQ ID NO:593 |
| | RhD Y 4 | acttaggtgcacagtgcgg | SEQ ID NO:594 |
| | RhD Y 5 | cttaggtgcacagtgcggt | SEQ ID NO:595 |
| | RhD Y cr 1 | caccgcactgtgcacctaa | SEQ ID NO:596 |
| | RhD Y cr 2 | ccgcactgtgcacctaagtc | SEQ ID NO:597 |
| | RhDYcr3 | gcactgtgcacctaagtctgc | SEQ ID NO:598 |
| | RhD Y cr 4 | tgcacctaagtctgcaaaga | SEQ ID NO:599 |
| | RhD Y cr 5 | cacctaagtctgcaaagaaatagcg | SEQ ID NO:600 |
| Rhesus RHD-Pseudogene-normal-allele: | | | |
|---|---|---|---|
| | RhD non Y 1 | ctatttctttgcagacttatgtgc | SEQ ID NO:601 |
| | RhD non Y 2 | tctttgcagacttatgtgcaca | SEQ ID NO:602 |
| | RhD non Y 3 | ttgcagacttatgtgcacagtg | SEQ ID NO:603 |
| | RhD non Y 4 | acttatgtgcacagtgcggt | SEQ ID NO:604 |
| | RhD non Y 5 | cttatgtgcacagtgcggtg | SEQ ID NO:605 |
| | RhD non Y cr 1 | acaccgcactgtgcacataa | SEQ ID NO:606 |
| | RhD non Y cr 2 | accgcactgtgcacataagtc | SEQ ID NO:607 |
| | RhD non Y cr 3 | gcactgtgcacataagtctgc | SEQ ID NO:608 |
| | RhD non Y cr 4 | tgtgcacataagtctgcaaag | SEQ ID NO:609 |
| | RhD non Y cr 5 | cacataagtctgcaaagaaatagcg | SEQ ID NO:610 |
| Yt-a-allele: | | | |
|---|---|---|---|
| | Yt.a.1 | gcgggagacttccacgg | SEQ ID NO:611 |
| | Yt.a.2 | gggagacttccacggcct | SEQ ID NO:612 |
| | Yt.a.3 | gagacttccacggcctgc | SEQ ID NO:613 |
| | Yt.a.4 | gacttccacggcctgca | SEQ ID NO:614 |
| | Yt.a.5 | ttccacggcctgcaggta | SEQ ID NO:615 |
| | Yt.a.1 CR | tacctgcaggccgtggaa | SEQ ID NO:616 |
| | Yt.a.2 CR | tgcaggccgtggaagtc | SEQ ID NO:617 |
| | Yt.a.3 CR | gcaggccgtggaagtctc | SEQ ID NO:618 |
| | Yt.a.4 CR | aggccgtggaagtctccc | SEQ ID NO:619 |
| | Yt.a.5 CR | ccgtggaagtctcccgc | SEQ ID NO:620 |
| Yt-b-allele: | | | |
|---|---|---|---|
| | Yt.b.1 | gcgggagacttcaacggc | SEQ ID NO:621 |
| | Yt.b.2 | gggagacttcaacggcctg | SEQ ID NO:622 |
| | Yt.b.3 | gagacttcaacggcctgca | SEQ ID NO:623 |
| | Yt.b.4 | gacttcaacggcctgcag | SEQ ID NO:624 |
| | Yt.b.5 | ttcaacggcctgcaggtaa | SEQ ID NO:625 |
| | Yt.b.1 CR | ttacctgcaggccgttgaa | SEQ ID NO:626 |
| | Yt.b.2 CR | ctgcaggccgttgaagtc | SEQ ID NO:627 |
| | Yt.b.3 CR | tgcaggccgttgaagtctc | SEQ ID NO:628 |
| | Yt.b.4 CR | caggccgttgaagtctccc | SEQ ID NO:629 |
| | Yt.b.5 CR | gccgttgaagtctcccgc | SEQ ID NO:630. |

14. A method according to any one of claims 10-13, wherein the oligonucleotide probes comprise at their 5' end a linker, preferably a -(CH₂)₆- moiety, and a reactive group, preferably an amino group, for attachment to an array support, preferably a poly-L-lysine-coated glass slide.

15. A method according to any one of claims 10-14, wherein the array includes one or more oligonucleotides with a sequence not occurring in the DNA of said mammalian species to allow for background subtraction, preferably selected from the group consisting of:
| | | |
|---|---|---|
| a3 | cagaccataagcacaggcgt | SEQ ID NO:631, |
| a9 | gctcgtccacagtgcgttat | SEQ ID NO:632, |
| a17 | cggcgttcaagcaaaccgaa | SEQ ID NO:633, |
| a23 | gacatatagctccactcaga | SEQ ID NO:634, |
| a27 | tagggtactgatgagcactc | SEQ ID NO:635, |
| a33 | tcagccctatcgcaggatgt | SEQ ID NO: 169, |
| a35 | gagacacttgacagtagcca | SEQ ID NO: 170, |
| a38 | ggcagggcacctcagtttat | SEQ ID NO:171, |
| a42 | tcaccagccagactgtgtag | SEQ ID NO: 172, |
| a43 | cttcacgcaagttgtccaga | SEQ ID NO:173. |

16. A method according to any one of claims 10-15, wherein the array includes one or more positive hybridization controls, preferably
| | | |
|---|---|---|
| CS05 | gtcctgacttctagctcatg | SEQ ID NO:174, |
and its detectably labeled complement is added to the products of the multiplex PCR amplification.

17. A method according to any one of claims 10-16, wherein the probes, before adding the products of the multiplex PCR amplification, are subjected to prehybridization and DNA denaturation treatments.

18. A method according to any one of claims 10-17, wherein the denatured products of the multiplex PCR amplification are applied to the DNA array without prepurification.

19. A method according to any one of claims 10-18, wherein for each blood cell antigen the ratio of the signal intensities for each of the alleles is used to assign the genotype.

20. A kit for genotyping blood cell antigens by a method as claimed in any one of claims 1-19, comprising one pair of blood cell antigen-specific chimeric primers for each blood cell antigen to be genotyped and at least one detectably labeled universal primer, preferably a pair of detectably labeled universal primers, as defined in any one of claims 1-7.

21. A kit as claimed in claim 20, further comprising a DNA array as defined in any one of claims 10-15.

22. A set of blood cell antigen-specific chimeric primer pairs useful in a multiplex PCR, comprising at least two, preferably substantially all, primer pairs selected from the group as defined in claim 7.

23. A set of blood cell antigen allele-specific oligonucleotide probes useful for genotyping blood cell antigens, comprising at least 72 different probes, preferably substantially all probes, selected from the group as defined in claim 13.

## Patentansprüche

1. Verfahren zur Genotypisierung von Blutzellen-Antigenen, welches das Unterziehen von DNA eines Individuums einer Säugetierspezies einer multiplexen Polymerasekettenreaktion (PCR) zur Amplifikation und detektierbaren Markierung einer Region des Locus von mindestens zwei verschiedenen Blutzellen-Antigenen, welche die Stelle des Nucleotidpolymorphismus des Blutzellen-Antigens enthält, und die Verwendung des **dadurch** amplifizierten und markierten DNA-Fragments zur Bestimmung des Genotyps jedes der Blutzellen-Antigene umfasst, wobei die multiplexe PCR die Verwendung von mindestens einem Paar von Blutzellen-Antigen-spezifischen chimären Primern für jedes Blutzellen-Antigen, das zu genotypisieren ist, und mindestens einen detektierbar markierten Universalprimer umfasst, wobei der mindestens eine Universalprimer eine einzigartige Sequenz, die nicht in der DNA der Säugetierspezies vorkommt, hat und wobei jedes der chimären Primerpaare einen linken chimären Primer und einen rechten chimären Primer umfasst, wobei jeder von ihnen einen Blutzellen-Antigen-spezifischen Teil am 3'-Ende und einen universellen Teil am 5'-Ende umfasst, wobei die Basensequenz des universellen Teils der chimären Primer der Basensequenz des mindestens einen Universalprimers entspricht und wobei die Blutzellen-Antigen-spezifischen Teile des chimären Primerpaars eine Region des Locus des Blutzellen-Antigens, welche die Stelle mit dem Nucleotidpolymorphismus des Blutzellen-Antigens enthält, umfassen.

2. Verfahren gemäß Anspruch 1, wobei der mindestens eine detektierbar markierte Universalprimer in einer molaren Menge verwendet wird, die mindestens 100-mal, bevorzugter mindestens 200-mal, der molaren Menge jedes chimären Primers entspricht.

3. Verfahren gemäß Anspruch 1 oder 2, wobei ein Paar detektierbar markierter Universalprimer mit einer einzigartigen Sequenz, welche nicht in der DNA der Säugetierspezies vorkommt, verwendet wird und wobei von jedem chimären Primerpaar die Basensequenz des universellen Teils von einem Teil des chimären Primerpaars der Basensequenz eines Mitglieds (bzw. Teils) des Universalprimerpaars entspricht und die Basensequenz des universellen Teils des anderen Mitglieds des chimären Primerpaars der Basensequenz des anderen Mitglieds des Universalprimerpaars entspricht.

4. Verfahren gemäß Anspruch 3, wobei einer der Universalprimer die Basensequenz gccgcgaattcactagtg (SEQ ID NO:2) und der andere Universalprimer die Basensequenz ggccgcgggaattcgatt (SEQ ID NO:1) hat.

5. Verfahren gemäß einem der Ansprüche 1 bis 4, wobei jeder Universalprimer eine Fluoreszenzmarkierung, vorzugsweise Cy5, an seinem 5'-Ende trägt.

6. Verfahren gemäß einem der Ansprüche 1 bis 5, wobei die Säugetierspezies ein Mensch ist, die DNA genomische DNA ist und die Blutzellen-Antigene mindestens zwei, vorzugsweise alle Mitglieder, ausgewählt aus der Gruppe, bestehend aus HPA1, HPA2, HPA3, HPA4, HPA5, Gov, JK1/2, FY1/2, GATAbox, KEL1/2, KEL3/4, RHCEex2/RHex2, RHCEex5/RHex5, RHDψ, RHD, BigC, MN, U und JO, umfassen.

7. Verfahren gemäß Anspruch 6, wobei die chimären Primer mindestens zwei, vorzugsweise im Wesentlichen alle Paare, ausgewählt aus der Gruppe, bestehend aus:
| | | |
|---|---|---|
| HPA1-links | gccgcgaattcactagtgcttcaggtcacagcgaggt | SEQ ID NO:3 |
| HPA1-rechts | ggccgcgggaattcgattgctccaatgtacggggtaaa | SEQ ID NO:4 |
| | | |
| HPA2-links | gccgcgaattcactagtgtgaaaggcaatgagctgaag | SEQ ID NO:5 |
| HPA2-rechts | ggccgcgggaattcgattagccagactgagcttctcca | SEQ ID NO:6 |
| | | |
| HPA3-links | gccgcgaattcactagtggctgaccactcctttgc | SEQ ID NO:7 |
| HPA3-rechts | ggccgcgggaattcgattggaagatctgtctgcgatcc | SEQ ID NO:8 |
| | | |
| HPA4-links | gccgcgaattcactagtgatccgcaggttactggtgag | SEQ ID NO:9 |
| HPA4-rechts | ggccgcgggaattcgattccatgaaggatgatctgtgg | SEQ ID NO:10 |
| | | |
| HPA5-tinks | gccgcgaattcactagtgtccaaatgcaagttaaattaccag | SEQ ID NO:11 |
| HPA5-rechts | ggccgcgggaattcgattacagacgtgctcttggtaggt | SEQ ID NO:12 |
| | | |
| HPA15-links | gccgcgaattcactagtgtgtatcagttcttggttttgtgtgatg | SEQ ID NO:13 |
| HPA15-rechts | ggccgcgggaattcgattaaaaccagtagccacccaag | SEQ ID NO:14 |
| | | |
| JK1/2-links | gccgcgaattctatagtggtctttcagccccatttgag | SEQ ID NO:15 |
| JK1/2-rechts | ggccgcgggaattcgattgttgaaaccccagagtccaa | SEQ ID NO:16 |
| | | |
| FY1/2-links | gccgcgaattcactagtggaattcttcctatggtgtgaatga | SEQ ID NO:17 |
| FY1/2-rechts | ggccgcgggaatcgattaagaagggcagtgcagagtc | SEQ ID NO:18 |
| | | |
| GATABox-links | gccgcgaattcactagtgggccctcattagtccttgg | SEQ ID NO:19 |
| GATABox-rechts | ggccgcgggaattcgattgaaatgaggggcatagggata | SEQ ID NO:20 |
| | | |
| Fyx-links | gccgcgaattcactagtgtcatgcttttcagacctctcttc | SEQ ID NO:175 |
| Fyx-rechts | ggccgcgggaattcgattcaagacgggcaccacaat | SEQ ID NO:176 |
| | | |
| KEL1/2-links | gccgcgaattcactagtgaagggaaatggccatactga | SEQ ID NO:21 |
| KEL1/2-rechts | ggccgcgggaattcgattagctgtgtaagagccgatcc | SEQ ID NO:22 |
| | | |
| KEL3/4-links | gccgcgaatcactagtggctcagaaactggaacagc | SEQ ID NO:23 |
| KEL3/4-rechts | ggccgcgggaattcgattagcaaggtgcaagaacactct | SEQ ID NO:24 |
| | | |
| KEL6/7-links | gccgcgaattcactagtggcagcaccaaccctatgttc | SEQ ID NO:177 |
| KEL6/7-rechts | ggccgcgggaattcgatttcaggcacaggtgagcttc | SEQ ID NO:178 |
| | | |
| RHCEex2vonvärts | gccgcgaattcactagtgcgtctgcttccccctcc | SEQ ID NO:25 |
| RHex2rückwärts | ggccgcgggaattcggattctgaacagtgtgatgaccacc | SEQ ID NO:26 |
| RHDex3-links | gccgcgaatcactagtgtcctggctctccctctct | SEQ ID NO:179 |
| RHCEex3-rechts | ggccgcgggaattcgatttttttcaaaaccccggaag | SEO ID NO:180 |
| | | |
| RHCEex5-links | gccgcgaattcactagtgggatgttctggccaagtg | SEQ ID NO:27 |
| RHex5rückwärts | ggccgcgggaattcgattggctgtcaccacactgactg | SEQ ID NO:28 |
| | | |
| RHDpsi-links | ggccgcgggaattcgattgtagtgagctggcccatca | SEQ ID NO:29 |
| RHDpsi-rechts | gccgcgaatcactagtgtgtctagtttcttaccggcaagt | SEQ ID NO:30 |
| | | |
| RHD-linksB | gccgcgaattcactagtgttataataacacttgtccacaggg | SEQ ID NO:31 |
| RHD-rechtsC | ggccgcgggaattcagttcggctccgacggtatc | SEQ ID NO:32 |
| | | |
| BigC-links | gccgcgaattcactagtgggccaccaccatttgaa | SEQ ID NO:33 |
| BigC-rechtsIntron2 | ggccgcgggaatcgattccatgaacatgccacttcac | SEQ ID NO:34 |
| | | |
| RhDVI-links (vorwärts) | ggccgcgggaattcgattctttgaattaagcacttcacaga | SEQ ID NO:181 |
| RhDVI-rechts (rückwärts) | gccgcgaattcactagtggcagaatcacactccatgct | SEQ ID NO:182 |
| | | |
| MN-links | gccgcgaattcactagtgtgagggaatttgtcttttgca | SEQ ID NO:35 |
| MN-rechts | ggccgcgggaatcgattcagaggcaagaattcctcca | SEQ ID NO:36 |
| | | |
| Ss-links | gccgcgaatcactagtgtttttctttgcacatgtcttt | SEQ ID NO:183 |
| Ss-rechts | ggccgcgggattcgatttctttgtctttacaatttcgtgtg | SEQ ID NO:184 |
| | | |
| U-links | gccgcgaattcactagtgcgctgatgtcatctgtcttatttttc | SEQ ID NO:37 |
| U-rechts | ggccgcgggaattcgattgatcgttccaataataccagcc | SEQ ID NO:38 |
| | | |
| DO-links | ggccgcgggaattcgatttgatccctccctatgagctg | SEQ ID NO:185 |
| DO-rechts | gccgcgaattcactagtgttatatgtgctcaggttcccagt | SEQ ID NO:186 |
| | | |
| JO-links | gccgcgaattcactagtgcctggcttaaccaggaaaa | SEQ ID NO:39 |
| JO-rechts | ggccgcgggaattcgatttcatactgctgtggagtcctg | SEQ ID NO:40 |
| | | |
| Colton-links | gccgcgaatcactagtggccacgaccctctttgtct | SEQ ID NO:187 |
| Colton-rechts | ggccgcgggaattcgatttacatgagggcacggaagat | SE:Q ID NO:188 |
| | | |
| Diego-links | gccgcgaattcactagtgacttattcacgggcatccag | SEQ ID NO:189 |
| Diego-rechts | ggccgcgggaattcgattaagctccacgttcctgaaga | SEQ ID NO:190 |
| | | |
| Wr-links | gccgcgaattcactagtgggcttcaaggtgtccaactc | SEQ ID NO:636 |
| Wr-rechts | ggccgcgggaattcgattaggatgaagaccagcagagc | SEQ ID NO:637 |
| Yt-links | ggccgcgggaattcgattccttcgtgcctgtggtagat | SEQ ID NO:638 |
| Yt-rechts | gccgcgaattcactagtgttctgggacttctgggaatg | SEQ ID NO:639 |
| | | |
| Lu-links | gccgcgaatcactagtgggacccagagagagagagactg | SEQ ID NO:640 |
| Lu-rechts | ggccgcgggaattcgattgggagtccagctggtatgg | SEQ ID NO:641. |
umfasst.

8. Verfahren gemäß einem der Ansprüche 1 bis 7, wobei die bei der multiplexen PCR verwendete DNA-Polymerase eine Taq-Polymerase oder eine ähnlich hitzeresistente Polymerase ist und jeder Zyklus der multiplexen PCR einen Hitzedenaturierungsschritt von 15 bis 60 s bei einer Temperatur von 90 bis 98°C (vorzugsweise etwa 30 s bei etwa 94°C), einen Hybridisierungsschritt von 60 bis 120 s bei einer Temperatur von 54 bis 60°C (vorzugsweise etwa 90 s bei etwa 57°C) und einen Kettenverlängerungsschritt von 60 bis 120 s bei einer Temperatur von 68 bis 76°C (vorzugsweise etwa 90 s bei etwa 72°C) umfasst.

9. Verfahren gemäß einem der Ansprüche 1 bis 8, wobei die multiplexe PCR, basierend auf einem Reaktionsvolumen von 50 µl, etwa 100 ng genomische DNA von dem Individuum, etwa 5 nM von jedem chimären Primer, pro chimärem Primerpaar etwa 0,2 µM von jedem detektierbar markierten Universalprimer und etwa 25 µl MasterMix, welcher Puffer, dNTP's und DNA-Polymerase enthält, verwendet.

10. Verfahren gemäß einem der Ansprüche 1 bis 9, wobei der Genotyp von jedem der Blutzellen-Antigene durch Hybridisieren der Produkte der multiplexen PCR-Amplifikation nach der Denaturierung mit Blutzellen-Antigen-Allel-spezifischen Oligonucleotidsonden, welche in einem DNA-Array enthalten sind, oder in einer anderen Form, wie zum Beispiel von Kügelchen getragen, vorliegen, und Analysieren der Hybridisierungsmuster bestimmt wird.

11. Verfahren gemäß Anspruch 10, wobei die Allel-spezifischen Sonden eine Länge von 15 bis 40 Nucleotiden, vorzugsweise 17 bis 30 Nucleotiden, haben.

12. Verfahren gemäß Anspruch 10 oder 11, wobei der Array für jedes Allel eines Blutzellen-Antigens mindestens zwei, vorzugsweise mindestens fünf, verschiedene Sense-Sonden und mindestens zwei, vorzugsweise mindestens fünf, verschiedene Antisense-Sonden enthält, wobei jede von ihnen die Stelle des Nucleotidpolymorphismus, aber an variierenden Positionen, abdeckt.

13. Verfahren gemäß Anspruch 11, wobei der Array mindestens 72 verschiedene Sonden, vorzugsweise im Wesentlichen alle Sonden, ausgewählt aus der Gruppe, bestehend aus
| HPA1-Allel a: | | | |
|---|---|---|---|
| | HPA-1aa | tacaggccctgcctctggg | SEQ ID NO:41 |
| | HPA-1ab | aggccctgcctctgggct | SEQ ID NO:42 |
| | HPA-1ac | ccctgcctctgggctcacc | SEQ ID NO:43 |
| | HPA-1ad | tgcctctgggctcacctcg | SEQ ID NO:44 |
| | HPA-1ae | ctctgggctcacctcgctg | SEQ ID NO:45 |
| | HPA-1aa CR | cagcgaggtgagcccagag | SEQ ID NO:46 |
| | HPA-1ab CR | cgaggtgagcccagaggca | SEQ ID NO:47 |
| | HPA-1ac CR | ggtgagcccagaggcaggg | SEQ ID NO:48 |
| | HPA-1ad CR | agcccagaggcagggcct | SEQ ID NO:49 |
| | HPA-1ae CR | cccagaggcagggcctgta | SEQ ID NO:50 |
| HPA1-Allel b: | | | |
|---|---|---|---|
| | HPA-1ba | tacaggccctgcctccggg | SEQ ID NO:51 |
| | HPA-1bb | aggccctgcctccgggct | SEQ ID NO:52 |
| | HPA-1bc | ccctgcctccgggctcac | SEQ ID NO:53 |
| | HPA-1bd | ctgcctccgggctcacct | SEQ ID NO:54 |
| | HPA-1be | ctccgggctcacctcgct | SEQ ID NO:55 |
| | HPA-1ba CR | agcgaggtgaggag | SEQ ID NO:56 |
| | HPA-1bb CR | aggtgagcccggaggcag | SEQ ID NO:57 |
| | HPA-1bc CR | gtgagcccggaggcaggg | SEQ ID NO:58 |
| | HPA-1bd CR | agcccggaggcagggcct | SEQ ID NO:59 |
| | HPA-1be CR | cccggaggcagggcctgta | SEQ ID NO:60 |
| PA2-Allel a: | | | |
|---|---|---|---|
| | HPA-2aa | ctgacgcccacacccaag | SEQ ID NO:61 |
| | HPA-2ab | ctcctgacgcccacaccc | SEQ ID NO:62 |
| | HPA-2ac | ggctcctgacgcccacac | SEQ ID NO:63 |
| | HPA-2ad | agggctcctgacgcccac | SEQ ID NO:64 |
| | HPA-2ae | ccagggctcctgacgccc | SEQ ID NO:65 |
| | HPA-2aa CR | cttgggtgtgggcgtcag | SEQ ID NO:66 |
| | HPA-2ab CR | gggtgtgggcgtcaggag | SEQ ID NO:67 |
| | HPA-2ac CR | gtgtgggcgtcaggagcc | SEQ ID NO:68 |
| | HPA-2ad CR | gtgggcgtcaggagccct | SEQ ID NO:69 |
| | HPA-2ae CR | gggcgtcaggagccctgg | SEQ ID NO:70 |
| HPA2-Allel b: | | | |
|---|---|---|---|
| | HPA-2ba | cctgatgcccacacccaag | SEQ ID NO:41 |
| | HPA-2bb | ctcctgatgcccacaccca | SEQ ID NO:72 |
| | HPA-2bc | ggctcctgatgtccacacc | SEQ ID NO:73 |
| | HPA-2bd | agggctcctgatgcccaca | SEQ ID NO:74 |
| | HPA-2be | ccagggctcctgatgccc | SEQ ID NO:75 |
| | HPA-2ba CR | cttgggtgtgggcatcagg | SEQ ID NO:76 |
| | HPA-2bb CR | tgggtgtgggcatcaggag | SEQ ID NO:77 |
| | HPA-2bc CR | ggtgtgggcatcaggagcc | SEQ ID NO:78 |
| | HPA-2bd CR | tgtgggcatcaggagccct | SEQ ID NO:79 |
| | HPA-2be CR | gggcatcaggagccctgg | SEQ ID NO:80 |
| HPA3-Allel a: | | | |
|---|---|---|---|
| | HPA-3aa | ccatccccagoccctccc | SEQ ID NO:81 |
| | HPA-3ab | gcccatccccagcccctc | SEQ ID NO:82 |
| | HPA-3ac | ctgcccatccccagcccc | SEQ ID NO:83 |
| | HPA-3ad1 | gctgcccatccccagccc | SEQ ID NO:84 |
| | HPA-3ad | ggctgcccatccccagcc | SEQ ID NO:85 |
| | HPA-3ad2 | gggctgcccatccccagc | SEQ ID NO:86 |
| | HPA-3ae | ggggctgcccatcccca | SEQ ID NO:87 |
| | HPA-3aa CR | gggaggggctggggatgg | SEQ ID NO:88 |
| | HPA-3ab CR | gaggggatggggatgggc | SEQ ID NO:89 |
| | HPA-3ac CR | ggggctggggatgggcag | SEQ ID NO:90 |
| | HPA-3ad1 CR | gggctggggatgggcagc | SEQ ID NO:91 |
| | HRA-3ad CR | ggctggggatgggcagcc | SEQ ID NO:92 |
| | HPA-ad2 CR | gctggggatgggcagccc | SEQ ID NO:93 |
| | HPA-3ae CR | tggggatgggcagcccc | SEQ ID NO:94 |
| HPA3-Allel b: | | | |
|---|---|---|---|
| | HPA-3ba | ccagccocagcccctcc | SEQ ID NO:95 |
| | HPA-3bb | gcccagccccagcccct | SEQ ID NO:96 |
| | HPA-3bc | ctgcccagccccagccc | SEQ ID NO:97 |
| | HPA-3bd1 | gctgcccagccccagcc | SEQ ID NO:98 |
| | HPA-3bd | ggctgcccagccccage | SEQ ID NO:99 |
| | HPA-3bd2 | gggctgcccagccccag | SEQ ID NO:100 |
| | HPA-3bc | ggggctgcccagcccca | SEQ ID NO:101 |
| | HPA-3ba CR | ggaggggctggggctgg | SEQ ID NO:102 |
| | HPA-3bb CR | aggggctggggctgggc | SEQ ID NO:103 |
| | HPA-3bc CR | gggctggggctgggcag | SEQ ID NO:104 |
| | HPA-3bd1 CR | ggctggggctgggcagc | SEQ ID NO:105 |
| | HPA-3bd CR | gctggggctgggcagcc | SEQ ID NO:106 |
| | HPA-3bd2 CR | ctggggctgggcagccc | SEQ ID NO:107 |
| | HPA-3bc CR | tggggctgggcagcccc | SEQ ID NO:108 |
| HPA4-Allel a: | | | |
|---|---|---|---|
| | HPA-4aa | gccacccagatgcgaaag | SEQ ID NO:109 |
| | HPA-4ab | cacccagatgcgaaagct | SEQ ID NO:110 |
| | HPA-4ac | cccagatgcgaaagctca | SEQ ID NO:111 |
| | HPA-4ad | cagatgcgaaagctcacc | SEQ ID NO:112 |
| | HPA-4ac | gatgcgaaagctcaccag | SEQ ID NO:113 |
| | HPA-4aa CR | ctggtgagctttcgcatc | SEQ ID NO:114 |
| | HPA-4ab CR | ggtgagctttcgcatctg | SEQ ID NO:115 |
| | HPA-4ac CR | tgagctttcgcatctggg | SEQ ID NO:116 |
| | HPA-4ad CR | agctttcgcatctgggtg | SEQ ID NO:117 |
| | HPA-4ae CR | ctttcgcatctgggtggc | SEQ ID NO:118 |
| HPA4-Allel b: | | | |
|---|---|---|---|
| | HPA-4ba | gccacccagatacaaaag | SEQ ID NO:119 |
| | HPA-4bb | ccacccagatgcaaaagcr | SEQ ID NO:120 |
| | HPA-4bc | acccagatgcaaaagctcac | SEQ ID NO:121 |
| | HPA-4bd | cagatgcaaaagctcacca | SEQ ID NO:122 |
| | HPA-4be | gatgcaaaagctcaccagtaa | SEQ ID NO:123 |
| | HPA-4ba CR | ttactggtgagcttttgcatc | SEQ ID NO:124 |
| | HPA-4bb CR | tggtgagcttttgcatctg | SEQ ID NO:125 |
| | HPA-4bc CR | gtgagcttttgcatctgggt | SEQ ID NO:126 |
| | HPA-4bd CR | agcttttgcatctgggtgg | SEQ ID NO:127 |
| | HPA-4be CR | cttttgcatctgggtggc | SEQ ID NO:128 |
| HPA5-Allel a: | | | |
|---|---|---|---|
| | HPA-5aa | gagtctacctgtttactatcaaagagg | SEQ ID NO:129 |
| | HPA-5ab | agtctacctgtttactatcaaagaggta | SEQ ID NO:130 |
| | HPA-5ac | gtctacctgtttactatcaaagaggtaa | SEQ ID NO:131 |
| | HPA-5ad | ctacctgtttactatcaaagaggtaaaa | SEQ ID NO:132 |
| | HPA-5ac | acctgtttactatcaaagaggtaaaa | SEQ ID NO:133 |
| | HPA-5aa CR | tttttacctctttgatagtaaacaggt | SEQ ID NO:134 |
| | HPA-5ab CR | ttttacctctttgatagtaaacaggtag | SEQ ID NO:135 |
| | HPA-5ac | ttacctctttgatagtaaacaggtagac | SEQ ID NO:136 |
| | HPA-5ad CR | tacctctttgatagtaaacaggtagact | SEQ ID NO:137 |
| | HPA-5ac CR | cctctttgatagtaaacaggtagactc | SEQ ID NO:138 |
| HPA5-Allel b: | | | |
|---|---|---|---|
| | HPA-5ba | gagtctacctgtttactatcaaaaagg | SEQ ID NO:139 |
| | HPA-5bb | agtctacctgtttactatcaaaaaggta | SEQ ID NO:140 |
| | HPA-5bc | gtctacctgtttactatcaaaaaggtaa | SEQ ID NO:141 |
| | HPA-5ad | ctacctgtttactatcaaaaaggtaaaa | SEQ ID NO:142 |
| | HPA-5be | acctgtttactatcaaaaaggtaaaaa | SEQ ID NO:143 |
| | HPA-5ba CR | tttttacctttttgatagtaaacaggt | SEQ ID NO:144 |
| | HPA-5bb CR | ttttacctttttgatagtaaacaggtag | SEQ ID NO:145 |
| | HPA-5bc CR | ttacctttttgatagtaaacaggtagac | SEQ ID NO:146 |
| | HPA-5bd CR | tacctttttgatagtaaacaggtagact | SEQ ID NO:147 |
| | HPA-5bc CR | cctttttgatagtaaacaggtagactc | SEQ ID NO:148 |
| HPA15-Allel a: | | | |
|---|---|---|---|
| | Gov-aa | ttattatcttgacttcagttacaggattt | SEQ ID NO:149 |
| | Gov-ab | tcttgacttcagttacaggatttacc | SEQ ID NO:150 |
| | Gov-ac | tgacttcagttacaggatttaccaa | SEQ ID NO:151 |
| | Gov-ad | cttcagttacaggatttaccaagant | SEQ ID NO:152 |
| | Gov-ae | cagttacaggatttaccangaatttg | SEQ ID NO:153 |
| | Gov-aa CR | caaattcttggtaaatcctgtaactg | SEQ ID NO:154 |
| | Gov-ab CR | attcttggtaaatcctgtaactgaag | SEQ ID NO:155 |
| | Gov-ac CR | tggtaaatctgtaactgaagtcaa | SEQ ID NO:156 |
| | Gov-ad CR | ggtaaatctgtaactgaagtcaaga | SEQ ID NO:157 |
| | Gov-ac CR | aaatcctgtaactgaagtcaagataataa | SEQ ID NO:158 |
| HPA15-Allel b: | | | |
|---|---|---|---|
| | Gov-ba | tatcttgacttcagttccaggatt | SEQ ID NO:159 |
| | Gov-bb | cttgacttcagttccaggattcac | SEQ ID NO:160 |
| | Gov-bc | gacttcagttccaggatttacca | SEQ ID NO:161 |
| | Gov-bd | ttcagtticcaggatttaccaag | SEQ ID NO:162 |
| | Gov-be | cagttccaggatttaccaagaatt | SEQ ID NO:163 |
| | Gov-ba CR | aattcttggtaaatcctggaactg | SEQ ID NO:164 |
| | Gov-bb CR | cttggtaaatcctggaactgaa | SEQ ID NO:165 |
| | Gov-bc CR | ggtaaatcctggaactgaagtca | SEQ ID NO:166 |
| | Gov-bd CR | gtaaatcctggaactgaagtcaag | SEQ ID NO:167 |
| | Gov-be CR | aatcctggaactgaagtcaagata | SEQ ID NO:168 |
| Colton-a-Allel: | | | |
|---|---|---|---|
| | Co.a.1 | aacaaccagacggcggt | SEQ ID NO:191 |
| | Co.a.2 | accagacggcggtccag | SEQ ID NO:192 |
| | Co.a.3 | cagacggcggtccagga | SEQ ID NO:193 |
| | Co.a.4 | gacggcggtccaggacaa | SEQ ID NO:194 |
| | Co.a.5 | caacggtccaggacaac | SBQ ID NO:195 |
| | Co.a.1.cr | gttgtcctggaccgccgt | SEQ ID NO:196 |
| | Co.a.2.cr | acctggaccgccgtctg | SEQ ID NO:197 |
| | Co.a.3.cr | ctggaccgccgtctggt | SEQ ID NO:198 |
| | Co.a.4.cr | ggaccgccgtctggttg | SEQ ID NO:199 |
| | Co.a.5.cr | accgccgtctggttatt | SEQ ID NO:200 |
| Colton-b-Allel: | | | |
|---|---|---|---|
| | Co.b.1 | ggaacaaccagacggtggt | SEQ ID NO:201 |
| | Co.b.2 | acaaccagacggtggtccag | SEQ ID NO:202 |
| | Co.b.3 | accagacggtggtccagga | SEQ ID NO:203 |
| | Co.b.4 | gacggtggtccaggacaacg | SEQ ID NO:204 |
| | Co.b.5 | cggtggtccaggacaacg | SEQ ID NO:205 |
| | Co.b.1.cr | cgttgtcctggaccaccgt | SEQ ID NO:206 |
| | Co.b.2.cr | ttgtcctggaccaccgtctg | SEQ ID NO:207 |
| | Co.b.3.cr | ctggaccaccgtctggttgt | SEQ ID NO:208 |
| | Co.b.4.cr | ggaccaccgtctggttgttc | SEQ ID NO:209 |
| | Co.b.5.cr | accaccgtctggttgttcc | SEQ ID NO:210 |
| Diego-a-Allel: | | | |
|---|---|---|---|
| | Di.a.1 | atgaagtccacgccggc | SEQ ID NO:211 |
| | Di.a.2 | gaagtccacgccggcct | SEQ ID NO:212 |
| | Di.a.3 | agtccacgccggcctcc | SEQ ID NO:213 |
| | Di.a.4 | tccacgccggcctccct | SEQ ID NO:214 |
| | Di.a.5 | acgccggcctccctggcc | SEQ ID NO:215 |
| | Di.a.1.cr | gccagggaggccggcgt | SEQ ID NO:216 |
| | Di.a.2.cr | agggaggccggcgtgga | SEQ ID NO:217 |
| | Di.a.3.cr | ggaggccggcgtggact | SEQ ID NO:218 |
| | Di.a.4.cr | aggccggcgtggacttc | SEQ ID NO:219 |
| | Di.a.5.cr | ggccggcgtggacttca | SEQ ID NO:220 |
| Diego-b-Allel: | | | |
|---|---|---|---|
| | Di.b.1 | ggtgaagtccacgctggc | SEQ ID NO:221 |
| | Di.b.2 | gtgaagtccacgctggcct | SEQ ID NO:222 |
| | Di.b.3 | tgaagtccacgctggcctcc | SEQ ID NO:223 |
| | Di.b.4 | gaagtccacgctggcctccct | SEQ ID NO:224 |
| | Di.b.5 | acgctggcctccctggccc | SEQ ID NO:225 |
| | Di.b.1.cr | ggccagggaggccagcgt | SEQ ID NO:226 |
| | Di.b.2.cr | cagggaggccagctgga | SEQ ID NO:227 |
| | Di.b.3.cr | agggaggccagcgtggact | SEQ ID NO:228 |
| | Di.b.4.cr | ggaggccagcgtggacttc | SEQ ID NO:229 |
| | Di.b.5.cr | ggccagcgtggacttcacc | SEQ ID NO:230 |
| Diego Wr-a-Allel: | | | |
|---|---|---|---|
| | Wr.a.1 | tgggcttgcgttccaagt | SEQ ID NO:231 |
| | Wr.a.2 | ggcttgcgttccaagtttc | SEQ ID NO:232 |
| | Wr.a.3 | ttgcgttccaagtttccca | SEQ ID NO:233 |
| | Wr.a.4 | cgttccaagtttcccatct | SEQ ID NO:234 |
| | Wr.a.5 | accaagtttcccatctgga | SEQ ID NO:235 |
| | Wr.a.1 CR | tccagatgggaaacttgga | SEQ ID NO:236 |
| | Wr.a.2 CR | agatgggaaacttggaacg | SEQ ID NO:237 |
| | Wr.a.3 CR | tgggaaacttggaacgcaa | SEQ ID NO:238 |
| | Wr.a.4 CR | gaaacttggaacgcaagcc | SEQ ID NO:239 |
| | Wr.a.5 CR | acttggaacgcaagcca | SEQ ID NO:240 |
| Diego Wr-b-Allel: | | | |
|---|---|---|---|
| | Wr.b.1 | gggcttgcttccgagtt | SEQ ID NO:241 |
| | Wr.b.2 | gcttgcgttccgagtttc | SEQ ID NO:242 |
| | Wr.b.3 | ttgcgttccgagtttccc | SEQ ID NO:243 |
| | Wr.b.4 | cgttccgagtttcccatc | SEQ ID NO:244 |
| | Wr.b.5 | tccgagtttcccatctgg | SEQ ID NO:245 |
| | Wr.b.1 CR | ccagatgggaaactcgga | SEQ ID NO:246 |
| | Wr.b.2 CR | gatgggaaactcggaacg | SEQ ID NO:247 |
| | Wr.b.3 CR | gggaaactcggaacgcaa | SEQ ID NO:248 |
| | Wr.b.4 CR | gaaaotcggaacgcaagc | SEQ ID NO:249 |
| | Wr.b.5 CR | aactcggaacgcaagccc | SEQ ID NO:250 |
| Dornbrock-a-Allel: | | | |
|---|---|---|---|
| | Do.a.1 | taccacccaagaggaaact | SEQ ID NO:251 |
| | Do.a.2 | ccacccaagaggaaactgg | SEQ ID NO:252 |
| | Do.a.3 | acccaagaggaaactggttg | SEQ ID NO:253 |
| | Do.a.4 | caagaggaaactggttgca | SEQ ID NO:254 |
| | Do.a.5 | aggaaactggttgcagttga | SEQ ID NO:255 |
| | Do a 6 cr | ctcaactgcaaccagtttcc | SEQ ID NO:256 |
| | Do a 7 cr | caactgcaaccagtttcctc | SEQ ID NO:257 |
| | Do a 8 cr | tgcaaccagttcctcttgg | SEQ ID NO:258 |
| | Do a 9 cr | accagtttcctcttgggtgg | SEQ ID NO:259 |
| | Do a 10 cr | cagtttcctcttgggtggta | SEQ ID NO:260 |
| Dornbrock-b-Allel: | | | |
|---|---|---|---|
| | Do.b.1 | taccacccaagaggagact | SEQ ID NO:261 |
| | Do.b.2 | ccacccaagaggagactgg | SEQ ID NO:262 |
| | Do.b.3 | acccaagaggagactggttg | SEQ ID NO:263 |
| | Do.b.4 | caagaggagactggttgca | SEQ ID NO:264 |
| | Do.b.5 | aggagactggttgcagttga | SEQ ID NO:265 |
| | Do b 6 cr | ctcaggctgcaaccagtctcc | SEQ ID NO:266 |
| | Do b 7 cr | caactgcaaccagtctcctc | SEQ ID NO:267 |
| | Do b 8 cr | tgcaaccagtctcctcttgg | SEQ ID NO:268 |
| | Do b 9 cr | accagtctcctcttgggtgg | SEQ ID NO:269 |
| | Do b 10 cr | cagtctcctcttgggtggt | SEQ ID NO:270 |
| Dombrock-Joseph(a) positiv Allel: | | | |
|---|---|---|---|
| | Jo.a.pos.1 | ccccagaacatgactaccac | SEQ ID NO:271 |
| | Jo.a.pos.2 | ccagaacatgactaccacaca | SEQ ID NO:272 |
| | Jo.a.pos.3 | agaacatgactaccacacacgc | SEQ ID NO:273 |
| | Jo.a.pos.4 | catgactaccacacacgctgt | SEQ ID NO:274 |
| | Jo.a.pos.5 | actaccacacacgctgtgg | SEQ ID NO:275 |
| | Jo.a.pos.1.cr | gccacagcgtgtgtggtagt | SEQ ID NO:276 |
| | Jo.a.pos.2.cr | acagcgtgtgtggtagtcatg | SEQ ID NO:277 |
| | Jo.a.pos.3.cr | agcgtgtgtggtagtcatgtt | SEQ ID NO:278 |
| | Jo.a.pos.4.cr | cgtgtgtggtagtcatgttctg | SEQ ID NO:279 |
| | Jo.a.pos.5.cr | gtggtagtcatgttctgggg | SEQ ID NO:280 |
| Dombrock-Joseph(a) negativ Allel: | | | |
|---|---|---|---|
| | Jo.a.neg.1 | ctaccccagaacatgactatcac | SEQ ID NO:281 |
| | Jo.a.neg.2 | cccagaacatgactatcacaca | SEQ ID NO:282 |
| | Jo.a.neg.3 | cagaacatgactatcacacacgc | SEQ ID NO:283 |
| | Jo.a.neg.4 | catgactatcacacacgctgtg | SEQ ID NO:284 |
| | Jo.a.neg.5 | actatcacacacgctgtggc | SEQ ID NO:285 |
| | Jo.a.neg.1.cr | aatagccacagcgtgtgtgatagt | SEQ ID NO:286 |
| | Jo.a.neg.2.cr | cacagcgtgtgatagtcatg | SEQ ID NO:287 |
| | Jo.a.neg.3.cr | cagcgtgtgatagtcatgtt | SEQ ID NO:288 |
| | Jo.a.neg.4.cr | cgtgtgtgatagtcatgttctgg | SEQ ID NO:289 |
| | Jo.a.neg.5.cr | gtgatagtcatgttctggggtag | SEQ ID NO:290 |
| Duffy-a-Allel: | | | |
|---|---|---|---|
| | Fy.A.1 | ccagatggagactatggtgcc | SEQ ID NO:291 |
| | Fy.A.2 | atggagactatggtaccaac | SEQ ID NO:292 |
| | Fy.A.3 | ggagactatggtgcccaactg | SEQ ID NO:293 |
| | Fy.A.4 | gactatggtgccaacctgga | SEQ ID NO:294 |
| | Fy.A.5 | tatggtgccaacctggaag | SEQ ID NO:295 |
| | Fy.A.1.cr | cttccaggttggcaccata | SEQ ID NO:296 |
| | Fy.A.2.cr | tccaggttggcaccatagtc | SEQ ID NO:297 |
| | Fy.A.3.cr | aggttggcaccatagtctcc | SEQ ID NO:298 |
| | Fy.A.4.cr | gttggcaccatagtctccat | SEQ ID NO:299 |
| | Fy.A.5.cr | gcaccatagtctccatctgg | SEQ ID NO:300 |
| Duffy-b-Allel: | | | |
|---|---|---|---|
| | Fy.X.(b).4 | ctcttccgctggcagctc | SEQ ID NO:334 |
| | Fy.X.(b).5 | cttccgctggcagctctg | SEQ ID NO:335 |
| | Fy.X.(b).1.cr | cagagctgccagcggaa | SEQ ID NO:336 |
| | Fy.X.(b).2.cr | agctgccagcggaagag | SEQ ID NO:337 |
| | Fy.X.(b).3.cr | ctgccagcggaagagagg | SEQ ID NO:338 |
| | Fy.X.(b).4.cr | accagcggaagagaggtc | SEQ ID NO:339 |
| | Fy.X.(b).5.cr | cagcggaagagaggtctg | SEQ ID NO:340 |
| Duffy Fyx-Mutation Allel: | | | |
|---|---|---|---|
| | Fy.X.1 | gcttttcagacctctcttctgct | SEQ ID NO:341 |
| | Fy.X.2 | tcagacctctcttctgctggc | SEQ ID NO:342 |
| | Fy.X.3 | acctctcttctgctggcagc | SEQ ID NO:343 |
| | Fy.X.4 | ctcttctgctggcagctctg | SEQ ID NO:344 |
| | Fy.X.5 | cttctgctggcagctctgc | SEQ ID NO:345 |
| | Fy.X.1.cr | gcagagctgccagcagaa | SEQ ID NO:346 |
| | Fy.X.2.cr | gagctgccagcagaagagag | SEQ ID NO:347 |
| | Fy.X.3.cr | agctaccagcagaagagagg | SEQ ID NO:348 |
| | Fy.X.4.cr | gccagcagaagagaggtctg | SEQ ID NO:349 |
| | Fy.X.5.cr | cagcagaagagaggtctgaaa | SEQ ID NO:350 |
| **Kidd-Allel-a:** | | | |
|---|---|---|---|
| | Jk.a.1 | cagccccatttgaggaca | SEQ ID NO:351 |
| | Jk.a.2 | gccccatttgaggacatcta | SEQ ID NO:352 |
| | Jk.a.3 | ccatttgaggactctactttg | SEQ ID NO:353 |
| | Jk.a.4 | atttgaggacatctactttgga | SEQ ID NO:354 |
| | Jk.a.5 | gaggacatctactttggactct | SEQ ID NO:355 |
| | Jk.a.1.cr | cagagtccaaagtagatgtcctc | SEQ ID NO:356 |
| | Jk.a.2.cr | agtccaaagtagatgtcctcaaa | SEQ ID NO:357 |
| | Jk.a.3.cr | aaagtagatgtcctcaaatggg | SEQ ID NO:358 |
| | Jk.a.4.cr | tagatgtcctcaaatggggc | SEQ ID NO:359 |
| | Jk.a.5.cr | atatcctcaaatggggctg | SEQ ID NO:300 |
| Kidd-Allel-b: | | | |
|---|---|---|---|
| | Jk.b.1 | tcagccccatttgagaaca | SEQ ND NO:361 |
| | Jk.b.2 | gccccatttgagaacatcta | SEQ ND NO:362 |
| | Jk.b.3 | cccatttgagaacatctactttg | SEQ ND NO:363 |
| | Jk.b.4 | atttgagaacatctactttggac | SEQ ND NO:364 |
| | Jk.b.5 | gagaacatctactttggactctg | SEQ ND NO:365 |
| | Jk.b.1.cr | ccagagtccaaagtagatgttctc | SEQ ND NO:366 |
| | Jk.b.2.cr | agagtccaaagtagatgttctcaaa | SEQ ND NO:367 |
| | Jk.b.3.cr | ccaaagtagatgttctcaaatgg | SEQ ND NO:368 |
| | Jk.b.4.cr | agtagatgttctcaaatggggc | SEQ ND NO:369 |
| | Jk.b.5.cr | atgttctcaaatggggctga | SEQ ND NO:370 |
| Kell-K1-Allel: | | | |
|---|---|---|---|
| | KEL.1.1 | tccttaaactttaaccgaatgct | SEQ ND NO:371 |
| | KEL.1.2 | ttgggattaaccgaatgctgaga | SEQ ND NO:372 |
| | KEL.1.3 | aactttaaccgaatgctgagactt | SEQ ND NO:373 |
| | KEL.1.4 | aaccgaatgctgagacttctg | SEQ ND NO:374 |
| | KEL.1.5 | cgaatgctgagacttctgatgag | SEQ ND NO:375 |
| | KEL 1.6 CR | actcatcagaagtctcagcattc | SEQ ND NO:376 |
| | KEL 1.7 CR | tcagaagtctcagcattcggt | SEQ ND NO:377 |
| | KEL 1.8 CR | aagtctcagcattcggttaaag | SEQ ND NO:378 |
| | KEL.1.9 CR | tctcagcttcggttaaagtttaa | SEQ ND NO:379 |
| | KEL 1.10 CR | agcattcggttaaagtttaagga | SEQ ND NO:380 |
| Kell-K2-Allel: | | | |
|---|---|---|---|
| | KEL.2.1 | ccttaaactttaaccgaacgct | SEQ ND NO:381 |
| | KEL.2.2 | aactttaaccgaacgctgaga | SEQ ND NO:382 |
| | KEL.2.3 | ctttaaccgaacgctgagactt | SEQ ND NO:383 |
| | KEL.2.4 | aaccgaacgctgagacttct | SEQ ND NO:384 |
| | KEL.2.5 | cgaacgactgagacttctgatg | SEQ ND NO:385 |
| | KEL 2.6 CR | tcatcgaagtctcagcgttc | SEQ ND NO:386 |
| | KEL 2.7 CR | cagaagtctcagcgttcggt | SEQ ND NO:387 |
| | KEL 2.8 CR | agtctcagcgttcggttaaag | SEQ ND NO:388 |
| | KEL 2.9 CR | tctcagcgttcggttaaagtt | SEQ ND NO:389 |
| | KEL 2.10 CR | agcgttcggttaaagtttaagg | SEQ ND NO:390 |
| Kell-K3-Allel: | | | |
|---|---|---|---|
| | KEL.3.1 | aatctccatcacttcatggct | SEQ ND NO:391 |
| | KEL.3.2 | accatcacttcatggctgtt | SEQ ID NO:392 |
| | KEL.3.3 | atcacttcatggctgttcca | SEQ ID NO:393 |
| | KEL.3.4 | acttcatggctgttccagtt | SEQ ND NO:394 |
| | KEL.3.5 | tcatggctgttccagtttc | SEQ ND NO:395 |
| | KEL.3.1.cr | agaaactggaacagccatgaa | SEQ ID NO:396 |
| | KEL.3.2.cr | aactggaacagccaatgaagtg | SEQ ID NO:397 |
| | KEL.3.3.cr | tggaacagccatgaagtagtg | SEQ ID NO:398 |
| | KEL.3.4.cr | acagccatgaagtgatggag | SEQ ID NO:399 |
| | KEL.3.5.cr | gccatgaagtgatggagatt | SEQ ID NO:400 |
| Kell-K4-Allel: | | | |
|---|---|---|---|
| | KEL.4.1 | tctccatcacttcacggct | SEQ ID NO:401 |
| | KEL.4.2 | ccatcacttcacggctgtt | SEQ ID NO:402 |
| | KEL.4.3 | cacttcacggctgttcca | SEQ ID NO:403 |
| | KEL.4.4 | acttcacggctgttccag | SEQ ID NO:404 |
| | KEL.4.5 | tcacggctgttccagttt | SEQ ID NO:405 |
| | KEL.4.1.cr | aaactggaacagccgtgaa | SEQ ID NO:406 |
| | KEL.4.2.cr | ctggaacagccgtaagtg | SEQ ID NO:407 |
| | KEL.4.3.cr | ggaacagccgtgaagtgatg | SEQ ID NO:408 |
| | KEL.4.4.cr | acagccgtgaagtgatgg | SEQ ID NO:409 |
| | KEL.4.5.cr | gccgtgaagtgatggaga | SEQ ID NO:410 |
| Kell-K6-Allel: | | | |
|---|---|---|---|
| | KEL.6.1 | tactgcctgggggctgccccgcc | SEQ ID NO:411 |
| | KEL.6.2 | tgggggctgccccgcctgt | SEQ ID NO:412 |
| | KEL.6.3 | gctgccccgcctgtgac | SEQ ID NO:413 |
| | KEL.6.4 | ctgccccgcctgtgacaa | SEQ ID NO:414 |
| | KEL.6.5 | accccacctatgacaac | SEQ ID NO:415 |
| | KEL.6.1.cr | gttgtcacaggcggggc | SEQ ID NO:416 |
| | KEL.6.2.cr | ttgtcacaggcggggcag | SEQ ID NO:417 |
| | KEL.6.3.cr | tcacaggcggggcagccc | SEQ ID NO:418 |
| | KEL.6.4.cr | acaggcggggcagccccca | SEQ ID NO:419 |
| | KEL.6.5.cr | aggcggggcagccccca | SEQ ID NO:420 |
| Kell-K7-Allel: | | | |
|---|---|---|---|
| | KEL.7.1 | actgcctgggggctgcctcgcc | SEQ ID NO:421 |
| | KEL.7.2 | cctgggggctcctcgcctgt | SEQ ID NO:422 |
| | KEL.7.3 | ggctgcctcgcctgtgac | SEQ ID NO:423 |
| | KEL.7.4 | ctgcctcgcctgtgacaacc | SEQ ID NO:424 |
| | KEL.7.5 | gcctcgcctgtgacaacc | SEQ ID NO:425 |
| | KEL.7.1.cr | ggttgtcacaggcgaggc | SEQ ID NO:426 |
| | KEL.7.2.cr | ggttgtcacaggcgaggcag | SEQ ID NO:427 |
| | KEL.7.3.cr | ttgtcacaggcgaggcagccc | SEQ ID NO:428 |
| | KEL.7.4.cr | acaggcgaggcagcccccagg | SEQ ID NO:429 |
| | KEL.7.5.cr | aggcgaggcagcccccagg | SEQ ID NO:430 |
| Lutheran a-Allel: | | | |
|---|---|---|---|
| | Lu.a.1 | ggagctcgcccccgcct | SEQ ID NO:431 |
| | Lu.a.2 | gctcgcccccgcctagc | SEQ ID NO:432 |
| | Lu.a.3 | cgcccccgcctagcctc | SEQ ID NO:433 |
| | Lu.a.4 | cccccgcctagcctcgg | SEQ ID NO:434 |
| | Lu.a.5 | cccgcctagcctcggct | SEQ ID NO:435 |
| | Lu.a.1 CR | agccgaggctaggcggg | SEQ ID NO:436 |
| | Lu.a.2 CR | ccgaggctaggcggggg | SEQ ID NO:437 |
| | Lu.a.3 CR | gaggctaggcgggggog | SEQ ID NO:438 |
| | Lu.a.4 CR | gctaggcgggggcgagc | SEQ ID NO:439 |
| | Lu.a.5 CR | aggcgggggcgagctcc | SEQ ID NO:440 |
| Lutheran b-Allel: | | | |
|---|---|---|---|
| | Lu.b.1 | gggagctcgcccccacct | SEQ ID NO:441 |
| | Lu.b.2 | gagctcgcccccacctagc | SEQ ID NO:442 |
| | Lu.b.3 | ctcgcccccacctagcctc | SEQ ID NO:443 |
| | Lu.b.4 | cccccacctagcctcggct | SEQ ID NO:444 |
| | Lu.b.5 | cccacctagcctcggctga | SEQ ID NO:445 |
| | Lu.b.1 CR | acagccgaggctaggtggg | SEQ ID NO:446 |
| | Lu.b.2 CR | agccgaggctggtggggg | SEQ ID NO:447 |
| | Lu.b.3 CR | gaggctggtgggggcgag | SEQ ID NO:448 |
| | Lu.b.4 CR | gctaggtgggggcgagctc | SEQ ID NO:449 |
| | Lu.b.5 CR | aggtgggggcgagctccc | SEQ ID NO:450 |
| MNS M-Allel: | | | |
|---|---|---|---|
| | M.1 | gtgagcatatcagcatcaag | SEQ ID NO:451 |
| | M.2 | atcagcatraagtaccactgg | SEQ ID NO:452 |
| | M.3 | catcaagtaccactggtgtg | SEQ ID NO:453 |
| | M.4 | taccactggtgtggcaatgc | SEQ ID NO:454 |
| | M.5 | ctggtgtggcaatgcaca | SEQ ID NO:455 |
| | M.1.cr | tgtgcattgccacaccagt | SEQ ID NO:456 |
| | M.2.cr | gcattgccacaccagtggta | SBQ ID NO:457 |
| | M.3.cr | ccacaccagtggtacttgatg | SEQ ID NO:458 |
| | M.4.cr | accagtggtacttgatgct | SEQ ID NO:459 |
| | M.5.cr | cttgatgctgatatgctcac | SEQ ID NO:460 |
| MNS N-Allel: | | | |
|---|---|---|---|
| | N.1 | tgtgagcatatcagcattaag | SEQ ID NO:461 |
| | N.2 | atcagcattaagtaccactgagg | SEQ ID NO:462 |
| | N.3 | cattaagtaccactgaggtgg | SEQ ID NO:463 |
| | N.4 | accactgaggtggcaatgc | SEQ ID NO:464 |
| | N.5 | ctgaggtggcaatgcacact | SEQ ID NO:465 |
| | N.1.cr | gtgtgcattgccacctcagt | SEQ ID NO:466 |
| | N.2.cr | gcattgccacctccagtggta | SEQ ID NO:467 |
| | N.3.cr | ccacctcagtggtacttaatgc | SEQ ID NO:468 |
| | N.4.cr | cctcagtggtacttaatgct | SEQ ID NO:469 |
| | N.5.cr | cttaatgctgatatgctcaca | SEQ ID NO:470 |
| MNS S-Allel: | | | |
|---|---|---|---|
| | big.S.1 | tttgctttataggagaaatggga | SEQ ID NO:471 |
| | big.S.2 | ctttataggagaaatgggaca | SEQ ID NO:472 |
| | big.S.3 | ttataggagaaatgggacaacttg | SEQ ID NO:473 |
| | big.S.4 | gagaaatgggacaacttgtcc | SEQ ID NO:474 |
| | big.S.5 | aaatgggacaacttgtccatc | SEQ ID NO:475 |
| | big.S.1.cr | gatggacaagttgtcccattt | SEQ ID NO:476 |
| | big.S.2.cr | gacaagttgtcccatttctcc | SEQ ID NO:477 |
| | big.S.3.cr | aagttgtcccatttcacctata | SEQ ID NO:478 |
| | big.S.4.cr | tgtcccattctcctataaagca | SEQ ID NO:479 |
| | big.S.5.cr | cccatttctcctataaagcaaaa | SEQ ID NO:480 |
| MNS s-Allel: | | | |
|---|---|---|---|
| | little.s.1 | tgctttataggagaaacggga | SEQ ID NO:481 |
| | little.s.2 | tttataggagaaacgggaca | SEQ ID NO:482 |
| | little.s,3 | ggagaaacgggacaacttg | SEQ ID NO:483 |
| | little.s.4 | gagaaacgggacaacttgtc | SEQ ID NO:484 |
| | little.s.5 | aaacgggacaacttgtccat | SEQ ID NO:485 |
| | little.s.1.cr | tggacaagttgtcccgttt | SEQ ID NO:486 |
| | little.s.2.cr | acaagttgtcccgtttctcc | SEQ ID NO:487 |
| | little.s.3.cr | agttgtcccgtttctcctata | SEQ ID NO:488 |
| | little.s.4.cr | tgtcccgttctcctataaagc | SEQ ID NO:489 |
| | little.s.5.cr | cccgtttctcctataaagca | SEQ ID NO:490 |
| MNS U-positiv Allel: | | | |
|---|---|---|---|
| | U.pos.1 | ttgctgctctctttagctcc | SEQ ID NO:491 |
| | U.pos.2 | ctctctttagctcctgtagtgat | SEQ ID NO:492 |
| | U.pos.3 | agctcctgtagtgataatactca | SEQ ID NO:493 |
| | U.pos.4 | gtagtgataatactcattattttg | SEQ ID NO:494 |
| | U.pos.5 | taatactcattatttttggggtg | SEQ ID NO:495 |
| | U.pos.1.cr | caccccaaaaataatgagtatta | SEQ ID NO:496 |
| | U.pos.2.cr | caaaaataatgagtattatcactaca | SEQ ID NO:497 |
| | U.pos.3.cr | agtattatcactacaggaggctaaa | SEQ ID NO:498 |
| | U.pos.4.cr | actactacaggagctaaagag | SEQ ID NO:499 |
| | U.pos.5.cr | gagctaaagagagcagcaaa | SEQ ID NO:500 |
| MNS U-negativ Allel: | | | |
|---|---|---|---|
| | U.neg.1 | ttttgctgctctctttatctcc | SEQ ID NO:501 |
| | U.neg.2 | gctctctttatctcctgtagagat | SEQ ID NO:502 |
| | U.neg.3 | tatctcctgtagagatcccatca | SEQ ID NO:503 |
| | U,neg.4 | gtagagataacactcattattttt | SEQ ID NO:504 |
| | U.neg.5 | taacatcattatttttggggt | SEQ ID NO:505 |
| | U.neg.1.cr | accccaaaaataatgagtgtta | SEQ ID NO:506 |
| | U.neg.2.cr | aaaaataatgagtgttatctctaca | SEQ ID NO:507 |
| | U.neg.3.cr | agtgttatctctacaggagataaa | SEQ ID NO:508 |
| | U.neg.4.cr | atctctacaggagctaaaagagag | SEQ ID NO:509 |
| | U.neg.5.cr | gagataaagagagcagcaaaatta | SEQ ID NO:510 |
| Rhesus C-Allel (307T): | | | |
|---|---|---|---|
| | Rh.big.C.1 | tgagccagttcccttctgg | SEQ ID NO:511 |
| | Rh.big.C.2 | gagccagttcccttctgg | SEQ ID NO:512 |
| | Rh-big.C.3 | ctgagccagttcccttctg | SEQ ID NO:513 |
| | Rh.big.C.4 | ccttctgggaaggtggtc | SEQ ID NO:514 |
| | U.big.C.5 | ccttctgggaaggtggtca | SEQ ID NO:515 |
| | Rh.big.C.1.cr | tgaccaccttcccagaagg | SEQ ID NO:516 |
| | Rh.big.C.2.cr | gaccaccttcccagaaggg | SEQ ID NO:517 |
| | Rh.big.C.3.cr | ccagaagggaactggctc | SEQ ID NO:518 |
| | Rh.big.C.4.cr | ccagaagggaactggctca | SEQ ID NO:519 |
| | Rh.big.C.5.cr | cagaagggaactggctcag | SEQ ID NO:520 |
| Rhesus c-Allel (307C): | | | |
|---|---|---|---|
| | Rh.little.c.1 | gagccagttcootcctgg | SEQ ID NO:521 |
| | Rh.little.c.2 | agccagttocctcctgg | SEQ ID NO:522 |
| | Rh.little.c.3 | tgagccagttccctcctg | SEQ ID NO:523 |
| | Rh.little.c.4 | cctcctgggaaggtggt | SEQ ID NO:524 |
| | Rh.little.c.5 | cctcctgggaaggttggtc | SEQ ID NO:525 |
| | Rh.little.c.1.cr | gaccacctcccaggagg | SEQ ID NO:526 |
| | Rh.little.c.2.cr | accaccttcccaggagg | SEQ ID NO:527 |
| | Rh.little.c.3.cr | ccaggagggaactggct | SEQ ID NO:528 |
| | Rh.little.c.4.cr | ccaggaggaactggctc | SEQ ID NO:529 |
| | Rh.little.c.5.cr | caggaggaactggctca | SEQ ID NO:530 |
| Rhesus BigC-Intron2-spezifisches-Insert Allel: | | | |
|---|---|---|---|
| | RhC.intron.2.1 | agggtgccctttgtcacttc | SEQ ID NO:531 |
| | RhC.intron.2.2 | gcccttgtcacttccagt | SEQ ID NO:532 |
| | RhC.intron.2.3 | cctttgtcacttcccagtgg | SEQ ID NO:533 |
| | RhC.intron.2.4 | ttgtcacttcccagtggtacaa | SEQ ID NO:534 |
| | RhC.intron.2.5 | tcacttcccagtggtacaatca | SEQ ID NO:535 |
| | RhC.intron.2.1.cr | gaagtgacaaaggcaccct | SEQ ID NO:536 |
| | RhC.intron.2.2.cr | actgggaagtgacaaagggc | SEQ ID NO:537 |
| | RhC.intron.2.3.cr | ccactgggaagtgccaaagg | SEQ ID NO:538 |
| | RhC.intron.2.4.cr | tgtaccactgggaagtgacaaa | SEQ ID NO:539 |
| | RhC.intron.2.5.cr | tgattgtaccactgggaagtga | SEQ ID NO:540 |
| Rhesus E-Allel: | | | |
|---|---|---|---|
| | Rh.big.E.1 | gccaagtgtcaactctcctct | SEQ ID NO:541 |
| | Rh.big.E.2 | caagtgtcaactctcctctgct | SEQ ID NO:542 |
| | Rh.big.E.3 | gtgtcaactotcctctgctgag | SEQ ID NO:543 |
| | Rh.big.E.4 | caactctcctctgctgagaagtc | SEQ ID NO:544 |
| | Rb.big.E.5 | tctcctctgctgagaagtcc | SEQ ID NO:545 |
| | Rh.big.E.1.cr | ggacttctcagcagaggagag | SEQ ID NO:546 |
| | Rh.big.E.2.cr | cttctcagcagaggagagttga | SEQ ID NO:547 |
| | Rh.big.E.3.cr | ctcagcagaggagagttgacac | SEQ ID NO:548 |
| | Rh.big.E.4.cr | gcagaggagagttgacacttg | SEQ ID NO:549 |
| | Rh.big.E.5.cr | gaggagagttgacacttgc | SEQ ID NO:550 |
| Rhesus e-Allel: | | | |
|---|---|---|---|
| | Rh.little.e.1 | gccaagtgtcaactctgctct | SEQ ID NO:551 |
| | Rh.little.e.2 | caagtgtcaactctgctctgct | SEQ ID NO:552 |
| | Rh.little.e.3 | tgtcaactctgctctgctgag | SEQ ID NO:553 |
| | Rh.little.e.4 | caactctgctctgctgagaag | SEQ ID NO:554 |
| | Rh.little.e.5 | tctgctctgctgagaagtcc | SEQ ID NO:555 |
| | Rh.little.e.1.cr | ggacttctcagcagagcagag | SEQ ID NO:556 |
| | Rh.little.e.2.cr | ttctcagcagagcagagttga | SEQ ID NO:557 |
| | Rh.little.e.3.cr | tcagcagagcagagttgacac | SEQ ID NO:558 |
| | Rh.little.e.4.cr | gcagagcagagttgacacttg | SEQ ID NO:559 |
| | Rh.little.e.5.cr | gagcagagttgacacttggc | SEQ ID NO:560 |
| Rhesus RHD-Allel: | | | |
|---|---|---|---|
| | RhD 1 | ttccccacagctccatcat | SEQ ID NO:561 |
| | RhD 2 | acagctccatcatgggctac | SEQ ID NO:562 |
| | RhD 3 | tccatcatggctacaacttc | SEQ ID NO:563 |
| | RhD 4 | tcatgggctacaacttcagct | SEQ ID NO:564 |
| | RhD 5 | gggctacaacttcagcttgct | SEQ ID NO:565 |
| | RhD cr 1 | agcaagctgaagttgtagccc | SEQ ID NO:566 |
| | RhD cr 2 | agctgaagttgtagccatga | SEQ ID NO:567 |
| | RhD cr 3 | gaagttgtagcccatgatgg | SEQ ID NO:568 |
| | RhD cr 4 | gcccatgatggagctgt | SEQ ID NO:569 |
| | RhD cr 5 | tgatggagctgtggggaa | SEQ ID NO:510 |
| Rhesus r's-Allel: | | | |
|---|---|---|---|
| | r's.T.1 | ggaaggtcaacttggtgca | SEQ ID NO:571 |
| | r's.T.2 | ggaaggtcaacttggtgcagt | SEQ ID NO:572 |
| | r's.T.3 | caacttggtgcagttggtg | SEQ ID NO:573 |
| | r's.T.4 | acttggtgcagttggtggt | SEQ ID NO:574 |
| | r's.T.5 | ttggtgcagttggtggtgat | SEQ ID NO:575 |
| | r's.T.1.cr | catcaccaccaactgcacca | SEQ ID NO:576 |
| | r's.T.2.cr | caccaccaactgcaccaagt | SEQ ID NO:577 |
| | r's.T.3.cr | accaactgcaccaagttgac | SEQ ID NO:578 |
| | r's.T.4.cr | actgcaccaagttgaccttcc | SEQ ID NO:579 |
| | r's.T.S.cr | tgcaccaagttgaccttcc | SEQ ID NO:580 |
| Rhesus DIV-Allel: | | | |
|---|---|---|---|
| | Rh.DVI.1 | atttcaaccctcttggcctt | SEQ ID NO:581 |
| | Rh.DVI.2 | aaccctcttggcotttgttt | SEQ ID NO:582 |
| | Rh.DVI.3 | tcttggcctttgtttccttg | SEQ ID NO:583 |
| | Rh.DVI.4 | ggtatcagcttgagagctcg | SEQ ID NO:584 |
| | Rh.DVI.5 | atcagcttgagagctcggag | SEQ ID NO:585 |
| | Rh.DVI.1.cr | aaggccaagagggttgaaat | SEQ ID NO:586 |
| | Rh.DVI.2.cr | aaacaaaggccaagagggtt | SEQ ID NO:587 |
| | Rh.DVI.3.cr | caaggaaacaaaggccaaga | SEQ ID NO:588 |
| | Rh.DVI.4.cr | cgagctctcaagctgatacc | SEQ ID NO:589 |
| | Rh.DVI5.cr | ctccgagctctcaagctgat | SEQ ID NO:590 |
| Rhesus RHD-Pseudogen-Mutation-Allel: | | | |
|---|---|---|---|
| | RhD Y 1 | tttctttgcagacttaggtgc | SEQ ID NO:591 |
| | RhD Y 2 | ctttgcagacttaggtgcaca | SEQ ID NO:592 |
| | RHD Y 3 | tttgcagacttaggtgcacgat | SEQ ID NO:593 |
| | RhD Y 4 | acttaggtgcacagtgcgg | SEQ ID NO:594 |
| | RhD Y 5 | cttaggtgcacagtgcggt | SEQ ID NO:595 |
| | RhD Y cr 1 RhD Y cr 1 | caccgcactgtgcacctaa | SEQ ID NO:596 |
| | RhD Y cr 2 | ccgcactgtgcacctaagtc | SEQ ID NO:597 |
| | RhD Y cr 3 | gcactgrgcacctaagtctgc | SEQ ID NO:598 |
| | RhD Y cr 4 | tgcacctaagtctgcaaaga | SEQ ID NO:599 |
| | RhD Y cr 5 | cacctaagtctgcaaagaaatagcg | SEQ ID NO:600 |
| Rhesus RHD-Pseudogen-Norrmal-Allel: | | | |
|---|---|---|---|
| | RhD non Y 1 | cttattcttgcagacttatgtgc | SEQ ID NO:601 |
| | RhD non Y 2 | tcttgcagacttatgtgcaca | SEQ ID NO:602 |
| | RhD non Y 3 | ttgcagacttatgtgcacagtg | SEQ ID NO:603 |
| | RhD non Y 4 | acttatgtgcacagtgcggt | SEQ ID NO:604 |
| | RhD non Y 5 | cttatgtgcacagtgcggtg | SEQ ID NO:605 |
| | RhD non Y cr 1 | acaccgcactgtgcacataa | SEQ ID NO:606 |
| | RhD non Y cr 2 | accgcactgtgcacataagtc | SEQ ID NO:607 |
| | RhD non Y cr 3 | gcactgtgcacataagtctgc | SEQ ID NO:608 |
| | RhD non Y cr 4 | tgtgcacataagtctgcaaag | SEQ ID NO:609 |
| | RhD non Y cr 5 | cacataagtctgcaaagaaatagcg | SEQ ID NO:610 |
| Yt-a-Allel: | | | |
|---|---|---|---|
| | Yt.a.1 | gcgggagacttccacgg | SEQ ID NO:611 |
| | Yt.a.2 | gggagacttccacggcct | SEQ ID NO:612 |
| | Yt.a.3 | gagcttccacggcctgc | SEQ ID NO:613 |
| | Yt.a.4 | gacttccacggactgca | SEQ ID NO:614 |
| | Yt.a.5 | ttccacggctgcaggta | SEQ ID NO:615 |
| | Yt.a1 CR | tacctgcaggccgtggaa | SEQ ID NO:616 |
| | Yt.a.2 CR | tgcaggccgtggaagtc | SEQ ID NO:617 |
| | Yt.a.3 CR | gcaggccgtggaagtctc | SEQ ID NO:618 |
| | Yt.a.4CR | aggccgtggaagtctccc | SEQ ID NO:619 |
| | Yt.a.5CR | ccgtggaagtctcccgc | SEQ ID NO:620 |
| **Yt-b-Allel:** | | | |
|---|---|---|---|
| | Yt.b.1 | gcggagacttcaacggc | SEQ ID NO:621 |
| | Yt.b.2 | gggagacttcaacggcctg | SEQ ID NO:622 |
| | Yt.b.3 | gagacttcaacggcctgca | SEQ ID NO:623 |
| | Yt.b.4 | gacttcaacggcctgcag | SEQ ID NO:624 |
| | Yt.b.5 | ttcaacggcctgcaggtaa | SEQ ID NO:625 |
| | Yt.b.1 CR | ttacctgcaggccgttgaa | SEQ ID NO:626 |
| | Yt.b.2 CR | ctgcaggccgttgaagtc | SEQ ID NO:627 |
| | Yt.b.3 CR | tgcaggccgttgaagtctc | SEQ ID NO:628 |
| | Yt.b.4 CR | caggccgttgaagtctccc | SEQ ID NO:629 |
| | Yt.b.3 CR | gccgttgaagtctcccgc | SEQ ID NO:630 |
enthält.

14. Verfahren gemäß einem der Ansprüche 10 bis 13, wobei die Oligonucleotidsonden an ihrem 5'-Ende einen Linker, vorzugsweise einen -(CH₂)₆--Rest, und eine reaktive Gruppe, vorzugsweise eine Aminogruppe, zur Anheftung an einen Arrayträger, vorzugweise einen Poly-L-Lysin-überschichteten Glasobjektträger, umfassen.

15. Verfahren gemäß einem der Ansprüche 10 bis 14, wobei der Array ein oder mehrere Oligonucleotid(e) mit einer Sequenz, welche nicht in der DNA der Säugetierspezies vorkommt/vorkommen, vorzugsweise ausgewählt aus der Gruppe, bestehend aus
| | | |
|---|---|---|
| a3 | cagaccataagcacaggcgt | SEQ ID NO:631, |
| a9 | gctcgtccacagtgcgttat | SEQ ID NO:632, |
| a17 | cggcgttcaagcaaaccgaa | SEQ ID NO:633, |
| a23 | gacatatagctccactcaga | SEQ ID NO:634, |
| a27 | tagggtactgatgagcactc | SEQ ID NO 635, |
| a33 | tcagcctatcgcaggatgt | SEQ ID NO:169, |
| a3S | gagacacttgacagtagcca | SEQ ID NO:170, |
| a38 | ggcagggcacctcagtttat | SEQ ID NO:171, |
| a42 | tcaccagccagactgtgtag | SEQ ID NO:172, |
| a43 | cttcacgcaagttgtccaga | SEQ ID NO:173 |
beinhaltet, um eine Hintergrundsubtraktion zu ermöglichen.

16. Verfahren gemäß einem der Ansprüche 10 bis 15, wobei der Array eine oder mehrere Hybridisierungspositivkontolle(n), vorzugsweise
| | | |
|---|---|---|
| CS05 | gtcptgacttpctagctcatg | SEQ ID NO:174 |
beinhaltet und sein detektierbar markiertes Kompliment zu den Produkten der multiplexen PCR-Amplifikation zugegeben wird.

17. Verfahren gemäß einem der Ansprüche 10 bis 16, wobei die Sonden vor der Zugabe der Produkte aus der multiplexen PCR-Amplifikation Vorhybridisierungs- und DNA-Denaturierungsbehandlungen unterzogen werden.

18. Verfahren gemäß einem der Ansprüche 10 bis 17, wobei die denaturierten Produkte aus der multiplexen PCR-Amplifikation für den DNA-Array ohne Voraufreinigung verwendet werden.

19. Verfahren gemäß einem der Ansprüche 10 bis 18, wobei für jedes Blutzellen-Antigen das Verhältnis der Signalintensitäten von jedem der Allele zur Zuordnung des Genotyps verwendet wird.

20. Kit zur Genotypisierung von Blutzellen-Antigenen durch ein Verfahren wie in einem der Ansprüche 1 bis 19 beansprucht, welches ein Paar an Blutzellen-Antigen-spezifischen Primern für jedes Blutzellen-Antigen, welches genotypisiert werden soll, und mindestens einen detektierbar markierten Universalprimer, vorzugsweise ein Paar detektierbar markierte Universalprimer wie in einem der Ansprüche 1 bis 7 definiert, umfasst.

21. Kit wie in Anspruch 20 beansprucht, welches weiter einen DNA-Array wie in einem der Ansprüche 10 bis 15 definiert umfasst.

22. Set an Blutzellen-Antigen-spezifischen chimären Primerpaaren, welche nützlich sind bei einer multiplexen PCR, welches mindestens zwei, vorzugsweise im Wesentlichen alle Primerpaare, ausgewählt aus der Gruppe wie in Anspruch 7 definiert, umfasst.

23. Set an Blutzellen-Antigen-Allel-spezifischen Oligonucleotidsonden, welche nützlich sind bei einer Genotypisierung von Blutzellen-Antigenen, welches mindestens 72 verschiedene Sonden, vorzugsweise im Wesentlichen alle Sonden, ausgewählt aus der Gruppe wie in Anspruch 13 definiert, umfasst.

## Revendications

1. Procédé de génotypage d'antigènes de cellules sanguines comprenant l'étape consistant à soumettre de l'ADN prélevé chez un individu d'une espèce mammifère à une réaction en chaîne par polymérase (PCR) multiplexe dans le but d'amplifier et de marquer de manière détectable une région du locus d'au moins deux antigènes différents de cellules sanguines, qui contient le site de polymorphisme nucléotidique dudit antigène de cellules sanguines, et à utiliser les fragments d'ADN marqués et amplifiés de la sorte pour déterminer le génotype de chacun desdits antigènes de cellules sanguines, ladite PCR multiplexe comprenant l'utilisation d'au moins une paire d'amorces chimériques spécifiques aux antigènes de cellules sanguines pour chaque antigène de cellule sanguine à génotyper et d'au moins une amorce universelle marquée de manière détectable, dans lequel ladite au moins une amorce universelle présente une séquence unique n'apparaissant pas dans l'ADN de ladite espèce mammifère et dans lequel chaque paire d'amorces chimériques comprend une amorce chimérique gauche et une amorce chimérique droite, chacune d'elles comprenant une partie spécifique aux antigènes de cellules sanguines au niveau de l'extrémité 3' et une partie universelle au niveau de l'extrémité 5', dans lequel la séquence de bases de la partie universelle des amorces chimériques correspond à la séquence de bases de ladite au moins une amorce universelle, et dans lequel lesdites parties spécifiques aux antigènes de cellules sanguines de la paire d'amorces chimériques comprend une région du locus de l'antigène de cellule sanguine qui contient le site du polymorphisme nucléotidique dudit antigène de cellule sanguine.

2. Procédé selon la revendication 1, dans lequel ladite au moins une amorce universelle marquée de manière détectable est utilisée en quantité molaire représentant au moins 100 fois, de préférence au moins 200 fois la quantité molaire de chaque amorce chimérique.

3. Procédé selon la revendication 1 ou 2, dans lequel on utilise une paire d'amorces universelles marquées de manière détectable contenant une séquence unique n'apparaissant pas dans l'ADN de ladite espèce mammifère et dans lequel, pour chaque paire d'amorces chimériques, la séquence de bases de la partie universelle d'un membre de la paire d'amorces chimériques correspond à la séquence de bases d'un membre de la paire d'amorces universelles et la séquence de bases de la partie universelle de l'autre membre de la paire d'amorces chimériques correspond à la séquence de bases de l'autre membre de la paire d'amorces universelles.

4. Procédé selon la revendication 3, dans lequel l'une desdites amorces universelles présente la séquence de bases gccgcgaattcactagtg (SEQ ID n° 2) et l'autre amorce universelle présente la séquence de bases ggccgcgggaattcgatt (SEQ ID n° 1).

5. Procédé selon l'une quelconque des revendications 1 à 4, dans lequel chaque amorce universelle porte un marqueur fluorescent, de préférence un groupement Cy5, au niveau de son extrémité 5'.

6. Procédé selon l'une quelconque des revendications 1 à 5, dans lequel ladite espèce mammifère est l'être humain, ledit ADN est un ADN génomique et lesdits antigènes de cellules sanguines comprennent au moins deux, de préférence tous les membres choisis dans le groupe constitué des antigènes HPA1, HPA2, HPA3, HPA4, HPA5, Gov, JK1/2, FY1/2, GATAbox, KEL1/2, KEL3/4, RHCEex2/RHex2, RHCEex5/RHex5, RHDyf, RHD, BigC, MN, U et JO.

7. Procédé selon la revendication 6, dans lequel lesdites amorces chimériques comprennent au moins deux, de préférence sensiblement toutes les paires sélectionnées dans le groupe comprenant les séquences suivantes
| | | |
|---|---|---|
| HPA1-gauche | gccgcgaattcatagtgcttcaggtcacagogaggt | SEQ ID n° 3 |
| HPA1-droite | ggccgcgggaattcgattgctccaatgtacggggtaaa | SEQ ID n° 4 |
| | | |
| HPA2-gauche | gccgcgaattcactagtgtgaaaggcaatgagctgaag | SEQ ID n° 5 |
| HPA2-droite | ggccgcgggattcgattagfccagactgagcttctcca | SEQ ID n° 6 |
| | | |
| HPA3-gauche | gccgcgaattcactagtggctgaccactcctttgc | SEQ ID n° 7 |
| HPA3-droite | ggccgcgggaattcgattggaagatctgtctgcgatcc | SEQ ID n° 8 |
| | | |
| HPA4-gauche | gccgcgaattcactagtgatccgcaggttactggtgag | SEQ ID n° 9 |
| HPA4-droite | ggccgcgggaattcgattccatgaaggatgatctgtgg | SEQ ID n° 10 |
| | | |
| HPA5-gauche | gccgcgaattpactagtgtccaaatgcaagttaaattaccag | SEQ ID n° 11 |
| HPA5-droite | ggcogcgggaattcgattacagaegtgotottggtaggt | SEQ ID n° 12 |
| HPA15-gauche | gccgcgaattcactagtgtgtatcagttcttggttttgtgatg | SEQ ID n° 13 |
| HPA15-droite | ggccgcgggaattcgattaaaacpagtagccacccaag | SEQ TD n° 14 |
| | | |
| JK1/2-gauche | gccgcgaattcactagtggtctttcagccccatttgag | SEQ ID n° 15 |
| JK1/2-droite | ggccgcgggaattcgattgttgaaaccccagagtccaa | SEQ ID n° 16 |
| | | |
| FY1/2-gauche | gccgcgaattcactagtggaattcttcctatggtgtgaatga | SEQ ID n° 17 |
| FY1/2-droite | ggccgcgggaattcgattaagaagggcagtgcagatc | SEQ ID n° 18 |
| | | |
| GATAbox-gauche | gccgcgaattcactagtgggccctcattagtccttgg | SEQ ID n° 19 |
| GATAbox-droite | ggccgcgggaattcgattgaaatgaggggcatagggata | SEQ ID n° 20 |
| | | |
| Fyx-gauche | gccgcgaatcactagtgtcatgctttcagacctctcttc | SEQ ID n° 175 |
| Fyx-droite | ggccgcgggaattcgattcaagacgggcaccacaat | SEQ ID n° 176 |
| | | |
| KEL1/2-gauche | gccgcgaattcactagtgaagggaaatggcatactga | SEQ ID n° 21 |
| KEL1/2-droite | ggccgcgggaatcgattagctgtgtaagagccgatcc | SEQ ID n° 22 |
| | | |
| KEL3/4-gauche | gccgcgaatcactagtggcctcagaaaotggaacagc | SEQ ID n° 23 |
| KEL3/4-droite | ggccgcgggaattcgattagcaaggtgcaagaacactct | SEQ ID n° 24 |
| | | |
| KEL6/7-gauche | gccgcgaattcactagtggcagcaccaaccctatgttc | SEQ ID n° 177 |
| KEL6/7-droite | ggccgcgggaattcgatttcaggcacaggtgagcttc | SEQ ID n° 178 |
| | | |
| RHCEex2sens | gccgcgaattcactagtgcgtcgtctgottccccctcc | SEQ ID n° 25 |
| RHex2antisens | ggccgcgggaattcgattctgaacagtgtgatgaccacc | SEQ ID n° 26 |
| | | |
| RHDex3-gauche | gccgcgaattcactagtgtcctggctctccctctct | SEQ ID n° 179 |
| RHCEex3-droite | ggccgcgggaattcgatttttttcaaaaccccggaag | SEQ ID n° 180 |
| | | |
| RHCEex5-gauche | gccgcgattcactagtgggatgttctggcaagtg | SEQ ID n° 27 |
| RHex5-antisens | ggccgcgggaattcgattgctgtcaccacactgactg | SEQ ID n° 28 |
| | | |
| RHDΨ-gauche | ggccgcgggaattcgattgtagtgagctggcccatca | SEQ ID n° 29 |
| RHDΨ-droite | gccgcgaattcactagtgtgtctagtttcttaccggcaagt | SEQ ID n° 30 |
| | | |
| RHD-gaucheB | gccgcgaatcactagtgttataataacacttgtccacaggg | SEQ ID n° 31 |
| RHD-droiteC | ggccgcgggaattcgattcggctccgacggtatc | SEQ ID n° 32 |
| | | |
| BigC-gauche | gccgcgaattcactagtgggccaccaccatttgaa | SEQ ID n° 33 |
| BigC-intron | ggccgcgggaattcgattccagaapatgccaptpac | SEQ ID n° 34 |
| droit 2 | | |
| RhDVI-gauche | ggccgcggattcgattctttgaattaagcacttcacaga | SEQ ID n° 181 |
| (sens) | | |
| RhDVI-droite | gccgcgaattcactagtggccagaatcacactcctgct | SEQ ID n° 182 |
| (antisens) | | |
| | | |
| MN-gauche | gccgcgaattcactagtgtgagggaatttgtcttttgca | SEQ ID n° 35 |
| MN-droite | ggccgcgggaattcgattcagaggcaagaattctcca. | SEQ ID n° 36 |
| | | |
| Ss-gauche | gccgcgaattcactagtgttttctttgcacatgtcttt | SEQ ID n° 183 |
| Ss-droite | ggcogcgggaattcgattctttgtctttacaatttcgtgtg | SEQ ID n° 184 |
| | | |
| U-gauche | gccgcgaattcactagtgcgctgatgttatctgtcttatttttc | SEQ ID n° 37 |
| U-droite | ggccgcgggaattcgattgatcgttccaataataccagcc | SEQ ID n° 38 |
| | | |
| DO-gauche | ggccgcgggaattcgatttgatccctcctatgagctg | SEQ ID n° 185 |
| DO-droite | gccgcgaattcactagtgttatatgtgctcaggttcccagt | SEQ ID n° 186 |
| | | |
| JO-gauche | gccgcgaattcactagtgcctggcttaaccaaggaaaa | SEQ ID n° 39 |
| JO-droite | ggccgcgggaattcgatttcatactgctgtggagtcctg | SEQ ID n° 40 |
| | | |
| Colton-gauche | gccgcgaatcactagtggccacgaccctctttgtct | SEQ ID n° 187 |
| Colton-droite | ggccgcgggaattcgatttacatgagggcacggaagat | SEQ ID n° 188 |
| | | |
| Diego-gauche | gccgogaattcactagtgacttattcacgggcatccag | SEQ ID n° 189 |
| Diego-droite | ggccgcgggaattcgattaagctccacgttcctgaaga | SEQ ID n° 190 |
| | | |
| Wr-gauche | gccgcgaattcactagtgggcttcaaggtgtccaactc | SEQ ID n° 636 |
| Wr-droite | ggccgcgggaattcgattaggatgaagaccagcagagc | SEQ ID n° 637 |
| | | |
| Yt-gauche | ggccgcgggaattcgattccttcgtgcctgtggtagat | SEQ ID n° 638 |
| Yt-droite | gccgcgaattcactagtgttctgggacttctgggaatg | SEQ ID n° 639 |
| | | |
| Lu-gauche | gccgcgaattcactagtgggacccagagagagagagactg | SEQ ID n° 640 |
| Lu-droite | ggccgcgggaattcgattgggagtccagctggtatgg | SEQ ID n° 641 |

8. Procédé selon l'une quelconque des revendications 1 à 7, dans lequel l'ADN polymérase utilisée dans la PCR multiplexe est la polymérase Taq ou une polymérase thermorésistante similaire et chaque cycle de la PCR multiplexe comprend une étape de dénaturation thermique de 15 à 60 s à une température de 90 à 98 °C (de préférence d'environ 30 s à environ 94 °C), une étape d'hybridation de 60 à 120 s à une température de 54 à 60 °C (de préférence d'environ 90 s à environ 57 °C) et une étape d'élongation de l'amorce de 60 à 120 s à une température de 68 à 76 °C (de préférence d'environ 90 s à environ 72 °C).

9. Procédé selon l'une quelconque des revendications 1 à 8, dans lequel ladite PCR multiplexe utilise, sur la base d'un volume réactionnel de 50 µl, environ 100 ng d'ADN génomique dudit individu, environ 5 nM de chaque amorce chimérique et, par paire d'amorces chimériques, environ 0,2 µM de chaque amorce universelle marquée de manière détectable et environ 25 µl de MasterMix 2x contenant un tampon, des dNTP et de l'ADN polymérase.

10. Procédé selon l'une quelconque des revendications 1 à 9, dans lequel le génotype de chacun des antigènes de cellules sanguines est déterminé en hybridant les produits de l'amplification par PCR multiplexe, après dénaturation, à des sondes oligonucléotidiques spécifiques aux allèles d'antigènes de cellules sanguines contenues dans un réseau d'ADN ou présentées sous n'importe quelle autre forme, par exemple une forme supportée sur des billes, et en analysant le profil d'hybridation.

11. Procédé selon la revendication 10, dans lequel les sondes spécifiques aux allèles présentent une taille de 15 à 40 nucléotides, de préférence de 17 à 30 nucléotides.

12. Procédé selon la revendication 10 ou 11, dans lequel le réseau contient, pour chaque allèle d'un antigène de cellule sanguine, au moins deux, de préférence au moins cinq sondes différentes de type sens et au moins deux, de préférence au moins cinq sondes différentes de type antisens, chacune d'elles couvrant le site de polymorphisme nucléotidique, mais dans des positions variables.

13. Procédé selon la revendication 11, dans lequel le réseau contient au moins 72 sondes différentes, de préférence sensiblement toutes les sondes choisies dans le groupe constitué des séquences suivantes :
| HPA1-allèle a : | | | |
|---|---|---|---|
| | HPA-1aa | tacaggccctgcctctggg | SEQ ID n° 41 |
| | HPA-1ab | aggccctgcctctggct | SEQ ID n° 42 |
| | HPA-1ac | ccctgcctctgggctcacc | SEQ ID n° 43 |
| | HPA-1ad | tgcctctgggctcacctcg | SEQ ID n° 44 |
| | HPA-1ae | ctctgggctcacctcgctg | SEQ ID n° 45 |
| | HPA-1aa CR | cagcgggtgagcccagag | SEQ ID n° 46 |
| | HPA-1ab CR | cgaggtgagcccagaggca | SEQ ID n° 47 |
| | HPA-1ac CR | ggtgagcccagaggcaggg | SEQ ID n° 48 |
| | HPA-1ad CR | agcccagaggcagggcct | SEQ ID n° 49 |
| | HPA-1ae CR | cccagaggcagggcctgta | SEQ ID n° 50 |
| HPA1-allèle b: | | | |
|---|---|---|---|
| | HPA-1ba | tacaggccctgcctccggg | SEQ ID n° 51 |
| | HPA-1bb | aggccctgcctccgggct | SEQ ID n° 52 |
| | HPA-1bc | ccctgcctccgggctcac | SEQ ID n° 53 |
| | HPA-1bd | ctgcctccgggctcacct | SEQ ID n° 54 |
| | HPA-1be | ctccgggctcacctcgct | SEQ ID n° 55 |
| | HPA-1ba CR | agcgaggtgagcccggag | SEQ ID n° 56 |
| | HPA-1bb CR | aggtgagcccggaggcag | SEQ ID n° 57 |
| | HPA-1be CR | gtgagcccggaggcaggg | SEQ ID n° 58 |
| | HPA-1bd CR | agcccggaggcagggcct | SEQ ID n° 59 |
| | HPA-1be CR | cccggaggcagggcctgta | SEQ ID n° 60 |
| HPA2-allèle a : | | | |
|---|---|---|---|
| | HPA-2aa | ctgacgcccacacccaag | SEQ ID n° 61 |
| | HPA-2ab | ctcctgacgcccacaccc | SEQ ID n° 62 |
| | HPA-2ac | ggctcctgacgcccacac | SEQ ID n° 63 |
| | HPA-2ad | agggctcctgacgcccac | SEQ ID n° 64 |
| | HPA-2ae | ccagggctcctgacgccc | SEQ ID n° 65 |
| | HPA-2aa CR | cttgggtgtgggcgtcag | SEQ ID n° 66 |
| | HPA-2ab CR | gggtgtgggcgtcaggag | SEQ ID n° 67 |
| | HPA-2ac CR | gtgtgggcgtcaggagcc | SEQ ID n° 68 |
| | HPA-2ad CR | gtgggcgtcaggagccct | SEQ ID n° 69 |
| | HPA-2ae CR | gggcgtcaggagccctgg | SEQ ID n° 70 |
| HPA2-allèle b : | | | |
|---|---|---|---|
| | HPA-2ba | cctgatgcccacacccaag | SEQ ID n° 71 |
| | HPA-2bb | ctcctgatgcccacaccca | SEQ ID n° 72 |
| | HPA-2bc | ggctcctgatgcccacacc | SEQ ID n° 73 |
| | HPA-2bd | agggctcctgatgcccaca | SEQ ID n° 74 |
| | HPA-2be | ccagggctcctgatgccc | SEQ ID n° 75 |
| | HPA-2ba CR | cttgggtgtgggcatcagg | SEQ ID n° 76 |
| | HPA-2bb CR | tgggtgtgggcatcaggag | SEQ ID n° 77 |
| | HPA-2be CR | ggtgtgggcatcaggagcc | SEQ ID n° 78 |
| | HPA-2bd CR | tgtgggcatcaggagccct | SEQ ID n° 79 |
| | HPA-2be CR | gggcatcaggagccctgg | SEQ ID n° 80 |
| HPA3-allèle a : | | | |
|---|---|---|---|
| | HPA-3aa | ccatccccagcccctccc | SEQ ID n° 81 |
| | HPA-3ab | gcccatccccagcccctc | SEQ ID n° 82 |
| | HPA-3ac | ctgcccatccccagcccc | SEQ ID n° 83 |
| | HPA-3ad1 | gctgcccatccccagccc | SEQ ID n° 84 |
| | HPA-3ad | ggctgcccatccccagcc | SEQ ID n° 85 |
| | HPA-3ad2 | gggctgcccatccccagc | SEQ ID n° 86 |
| | HPA-3ae | ggggctgcccatcccca | SEQ ID n° 87 |
| | HPA-3aa CR | gggaggggctggggatgg | SEQ ID n° 88 |
| | HPA-3ab CR | gaggggctggggatgggc | SEQ ID n° 89 |
| | HPA-3ac CR | ggggctggggatgggcag | SEQ ID n° 90 |
| | HPA-3ad1 CR | gggctggggatgggcagc | SEQ ID n° 91 |
| | HPA-3ad CR | ggctggggatgggcagcc | SEQ ID n° 92 |
| | HPA-3ad2 CR | gctggggatgggcagccc | SEQ ID n° 93 |
| | HPA-3ae CR | tggggatgggcagcccc | SEQ ID n° 94 |
| HPA3-allèle b : | | | |
|---|---|---|---|
| | HPA-3ba | ccagccccagccoctcc | SEQ ID n° 95 |
| | HPA-3bb | gcccagccccagcccct | SEQ ID n° 96 |
| | HPA-3bc | ctgcccagccccagccc | SEQ ID n° 97 |
| | HPA-3bd1 | gctgcccagucccagcc | SEQ ID n° 98 |
| | HPA-3bd | ggctgcccagccccagc | SEQ ID n° 99 |
| | HPA-3bd2 | gggctgcccagccccag | SEQ ID n° 100 |
| | HPA-3be | ggggctgcccagcccca | SEQ ID n° 101 |
| | HPA-3ba CR | ggaggggctggggctgg | SEQ ID n° 102 |
| | HPA-3bb CR | aggggctggggctgggc | SEQ ID n° 103 |
| | HPA-3bc CR | gggctggggctgggcag | SEQ ID n° 104 |
| | HPA-3bd1 CR | ggctggggctgggcagc | SEQ ID n° 105 |
| | HPA-3bd CR | gctggggctgggcagcc | SEQ ID n° 106 |
| | HPA-3bd2 CR | ctggggctgggcagccc | SEQ ID n° 107 |
| | HPA-3bc CR | tggggctgggcagcccc | SEQ ID n° 108 |
| HPA4-allèle a : | | | |
|---|---|---|---|
| | HPA-4aa | gccacccagatgcgaaag | SEQ ID n° 109 |
| | HPA-4ab | cacccagatgcgaaagct | SEQ ID n° 110 |
| | HPA-4ac | cccagatgcgaaagctca | SEQ ID n° 111 |
| | HPA-4ad | cagatgcgaaagctcacc | SEQ ID n° 112 |
| | HPA-4ae | gatgcgaaagctcaccag | SEQ ID n° 113 |
| | HPA-4aa CR | ctggtgagctttcgcatc | SEQ ID n° 114 |
| | HPA-4ab CR | ggtgagctttcgcatctg | SEQ ID n° 115 |
| | HPA-4ac CR | tgagctttcgcatctggg | SEQ ID n° 116 |
| | HPA-4ad CR | agctttcgcatctgggtg | SEQ ID n° 117 |
| | HPA-4ae CR | ctttcgcatctgggtggc | SEQ ID n° 118 |
| HPA4-allèle b : | | | |
|---|---|---|---|
| | HPA-4ba | gccacccagatgcaaaag | SEQ ID n° 119 |
| | HPA-4bb | ccacccagatgcaaaagct | SEQ ID n° 120 |
| | HPA-4bc | acccagatgcaaaagctcac | SEQ ID n° 121 |
| | HPA-4bd | cagatgcaaaagctcacca | SEQ ID n° 122 |
| | HPA-4be | gatgcaaaagctcacoagtaa | SEQ ID n° 123 |
| | HPA-4ba CR | ttactggtgagcttttgcatc | SEQ ID n° 124 |
| | HPA-4bb CR | tggtgagcttttgcatctg | SEQ ID n° 125 |
| | HPA-4bc CR | gtgacttttgcatctgggt | SEQ ID n° 126 |
| | HPA-4bd CR | agcttttgcatctgggtgg | SEQ ID n° 127 |
| | HPA-4be CR | cttttgcatctgggtggc | SEQ ID n° 128 |
| HPA5-allèle a : | | | |
|---|---|---|---|
| | HPA-5aa | gagctacctgtttactatcaaagagg | SEQ ID n° 129 |
| | HPA-5ab | agtctacctgtttactatcaaagaggta | SEQ ID n° 130 |
| | HPA-5ac | gtctacctgtttactatcaaagaggtaa | SEQ ID n° 131 |
| | HPA-5ad | ctacctgtttactatcaaagaggtaaa | SEQ ID n° 132 |
| | HPA-5ae | acctgtttactatcaaagaggtaaaaa | SEQ ID n° 133 |
| | HPA-5aa CR | tttttacctctttgatagtaaacaggt | SEQ ID n° 134 |
| | HPA-5ab CR | ttttacctctttgatagtaaacaggtag | SEQ ID n° 135 |
| | HPA-5ac CR | ttacctctttgatagtaaacaggtagac | SEQ ID n° 136 |
| | HPA-5ad CR | tacctctttgatagtaaacaggtagact | SEQ ID n° 137 |
| | HPA-5ae CR | cctctttgatagtaaacaggtagactc | SEQ ID n° 138 |
| HPA5-allèle b : | | | |
|---|---|---|---|
| | HPA-5ba | gagtctacctgtttactatcaaaaagg | SEQ ID n° 139 |
| | HPA-5bb | agtctacctgtttactatcaaaaaggta | SEQ ID n° 140 |
| | HPA-5bc | gtctacctgtttactatcaaaaaggtaa | SEQ ID n° 141 |
| | HPA-5ad | ctacctgtttactatcaaaaaggtaaaa | SEQ ID n° 142 |
| | HPA-5be | acctgtttactatcaaaaaggtaaaaa | SEQ ID n° 143 |
| | HPA-5ba CR | tttttacctttttgatagtaaacaggt | SEQ ID n° 144 |
| | HPA-5bb CR | ttttacctttttgatagtaaacaggtag | SEQ ID n° 145 |
| | HPA-5bc CR | ttacctttttgatagtaaacaggtagac | SEQ ID n° 146 |
| | HPA-5bd CR | tacctttttgatagtaaacaggtagact | SEQ ID n° 147 |
| | HPA-5be CR | cctttttgatagtaaacaggtagactc | SEQ ID n° 148 |
| HPA15-allèle a : | | | |
|---|---|---|---|
| | Gov-aa | ttattatcttgacttcagttacaggattt | SEQ IDn° 149 |
| | Gov-ab | tcttgacttcagttacaggatttacc | SEQ ID n° 150 |
| | Gov-ac | tgacttcagttacaggatttaccaa | SEQ TD n° 151 |
| | Gov-ad | cttcagttacaggatttaccaagaat | SEQ ID n° 152 |
| | Gov-ae | cagttacaggatttaccaagaatttg | SEQ ID n° 153 |
| | Gov-aa CR | caaattcttggtaaatcctgtaactg | SEQ ID n° 154 |
| | Gov-ab CR | attcttggtaaatcctgtaactgaag | SEQ ID n° 155 |
| | Gov-ac CR | tggtaaatcctgtaactgaagtcaa | SEQ ID n° 156 |
| | Gov-ad CR | ggtaaatcctgtaactgaagtcaaga | SEQ ID n° 157 |
| | Gov-ae CR | aaatcctgtaactgaagtcaagataataa | SEQ ID n° 158 |
| HPA15-allèle b : | | | |
|---|---|---|---|
| | Gov-ba | tatcttgacttcagttccaggatt | SEQ ID n° 159 |
| | Gov-bb | cttgacttcagttccaggatttac | SEQ ID n° 160 |
| | Gov-bc | gacttcagttccaggatttacca | SEQ ID n° 161 |
| | Gov-bd | ttcagttccaggatttaccaag | SEQ ID n° 162 |
| | Gov-be | cagttccaggatttaccaagaatt | SEQ ID n° 163 |
| | Gov-ba CR | aattcttggtaaatcctggaactg | SEQ ID n° 164 |
| | Gov-bb CR | cttggtaaatcctggaactgaa | SEQ ID n° 165 |
| | Gov-bc CR | ggtaaatcctggaactgaagtca | SEQ ID n° 166 |
| | Gov-bd CR | gtaaatcctggaactgaagtcaag | SEQ ID n° 167 |
| | Gov-be CR | aatcctggaactgaagtcaagata | SEQ ID n° 168 |
| Colton-allèle a : | | | |
|---|---|---|---|
| | Co.a,1 | aacaaccagacggcggt | SEQ ID n° 191 |
| | Co.a.2 | accagacggcggtccag | SEQ ID n° 192 |
| | Co.a.3 | cagacggcggtccagga | SEQ ID n° 193 |
| | Co.a.4 | gacggcggtccaggacaa | SEQ ID n° 194 |
| | Co.a.5 | cggcggtccaggacaac | SEQ ID n° 195 |
| | Co.e.1.cr | gttgtcctggaccgccgt | SEQ ID n° 196 |
| | Co.a.2.cr | tcctggaccgccgtctg | SEQ ID n° 197 |
| | Co.a.3.cr | ctggaccgccgtctggt | SEQ ID n° 198 |
| | Co.a.4.cr | ggaccgccgtctggttg | SEQ ID n° 199 |
| | Co.a.5.cr | accgccgtctggttgtt | SEQ ID n° 200 |
| Colton-allèle b : | | | |
|---|---|---|---|
| | Co.b,1 | ggaacaaccagacggtggt | SEQ ID n° 201 |
| | Co.b.2 | acaaccagacggtggtccag | SEQ ID n° 202 |
| | Co.b.3 | accagacggtggtccagga | SEQ ID n° 203 |
| | Co.b.4 | gacggtggtccaggacaacg | SEQ ID n° 204 |
| | Co.b,5 | cggtggtccaggacaacg | SEQ ID n° 205 |
| | Co.b.1.cr | cgttgtcctggaccaccgt | SEQ ID n° 206 |
| | Co.b.2.cr | ttgtcctggaccaccgtctg | SEQ ID n° 207 |
| | Co.b.3.cr | ctggaccaccgtctggttgt | SEQ ID n° 208 |
| | Co.b.4.cr | ggaccaccgtctggttgttc | SEQ ID n° 209 |
| | Co.b.5.cr | accaccgtctggttgttcc | SEQ ID n° 210 |
| Diego-allèle a : | | | |
|---|---|---|---|
| | Di.a.1 | gtgaagtccacgccggc | SEQ ID n° 211 |
| | Di.a.2 | gaagtccacgccggcct | SEQ ID n° 212 |
| | Di.a.3 | agtccacgccggcctcc | SEQ ID n° 213 |
| | Di.a.4 | tccacgccggcctccct | SEQ ID n° 214 |
| | Di.a.5 | acgccggcctccotggcc | SEQ ID n° 215 |
| | Di.a.1.cr | gccagggaggccggcgt | SEQ ID n° 216 |
| | Di.a.2.cr | agggaggccggcgtgga | SEQ ID n° 217 |
| | Di.a.3.cr | ggaggccggcgtggact | SEQ ID n° 218 |
| | Di.a.4.cr | aggccggcgtggacttc | SEQ ID n° 219 |
| | Di.a.5.cr | ggccggcgtggacttca | SEQ ID n° 220 |
| Diego-allèle b : | | | |
|---|---|---|---|
| | Di.b.1 | ggtgaagtccacgctggc | SEQ ID n° 221 |
| | Di.b.2 | gtgaagtccacgctggcct | SEQ ID n° 222 |
| | Di.b.3 | tgaagtccacgctggcctcc | SEQ ID n° 223 |
| | Di.b.4 | gaagtccacgctggcatccct | SEQ ID n° 224 |
| | Di.b.5 | acgctggcctccctggccc | SEQ ID n° 225 |
| | Di.b.1.cr | ggccagggaggccagcgt | SEQ ID n° 226 |
| | Di.b.2.cr | cagggaggccagcgtgga | SEQ ID n° 227 |
| | Di.b.3.cr | agggaggccagcgtggact | SEQ ID n° 228 |
| | Di.b.4.cr | ggaggccagcgtggacttc | SEQ ID n° 229 |
| | Di.b.5.cr | ggccagcgtggacttcacc | SEQ ID n° 230 |
| Diego Wr-allèle a : | | | |
|---|---|---|---|
| | Wr.a.1 | tgggcttgcgttccaagt | SEQ ID n° 231 |
| | Wr.a.2 | ggcttgcgttccaagtttc | SEQ ID n° 232 |
| | Wr.a.3 | ttgcgttccaagtttccca | SEQ ID n° 233 |
| | Wr.a.4 | cgttccaagtttcccatct | SEQ ID n° 234 |
| | Wr.a.5 | tccaagtttcccatctgga | SEQ ID n° 235 |
| | Wr.a.1 CR | tccagatgggaaacttgga | SEQ ID n° 236 |
| | Wr.a.2 CR | agatgggaaacttggaacg | SEQ ID n° 237 |
| | Wr.a.3 CR | tgggaaacttggaacgcaa | SEQ ID n° 238 |
| | Wr.a.4 CR | gaaacttggaacgcaagcc | SEQ ID n° 239 |
| | Wr.a.5 CR | acttggaacgcaagccca | SEQ ID n° 240 |
| Diego Wr-allèle b : | | | |
|---|---|---|---|
| | Wr.b.1 | gggcttgcgttccgagtt | SEQ ID n° 241 |
| | Wr.b.2 | gcttgcgttccgagtttc | SEQ ID n° 242 |
| | Wr.b.3 | ttgcgttccgagtttccc | SEQ ID n° 243 |
| | Wr.b.4 | cgttccgagtttcccatc | SEQ ID n° 244 |
| | Wr.b.5 | tccgagtttcccatctgg | SEQ ID n° 245 |
| | Wr.b.1 CR | ccagatgggaaactcgga | SEQ ID n° 246 |
| | Wr.b.2 CR | gatgggaaactcggaacg | SEQ ID n° 247 |
| | Wr.b.3 CR | gggaaactcggaacgcaa | SEQ ID n° 248 |
| | Wr.b.4 CR | gaaactcggaacgcaagc | SEQ ID n° 249 |
| | Wr.b.5 CR | aactcggaacgcaagccc | SEQ ID n° 250 |
| Dombrock-allèle a : | | | |
|---|---|---|---|
| | Do.a.1 | taccacccaagaggaaact | SEQ ID n° 251 |
| | Do.a.2 | ccacccaagaggaaactgg | SEQ ID n° 252 |
| | Do.a.3 | acccaagaggaaactggttg | SEQ ID n° 253 |
| | Do.a.4 | caagaggaaactggttgca | SEQ ID n° 254 |
| | Do.a.5 | aggaaactggttgcagttga | SEQ ID n° 255 |
| | Do a 6 cr | ctcaactgcaaccagtttcc | SEQ ID n° 256 |
| | Do a 7 cr | caactgcaaccagtttcctc | SEQ ID n° 257 |
| | Do a 8 cr | tgcaaccagtttcctcttgg | SEQ ID n° 258 |
| | Do a 9 cr | accagtttcctcttgggtgg | SEQ ID n° 259 |
| | Do a 10 cr | cagtttcctcttgggtggta | SEQ ID n° 260 |
| Dombrock-allèle b : | | | |
|---|---|---|---|
| | Do.b.1 | taccacccaagaggagact | SEQ ID n° 261 |
| | Do.b.2 | ccacccaagaggagactgg | SEQ ID n° 262 |
| | Do.b.3 | acccaagaggagactggttg | SEQ ID n° 263 |
| | Do.b.4 | caagaggagactggttgca | SEQ ID n° 264 |
| | Do.b.5 | aggagactggttgcagttga | SEQ ID n° 265 |
| | Do b 6 cr | ctcaactgcaaccagtctcc | SEQ ID n° 266 |
| | Do b 7 cr | caactgcaaccagtctcctc | SEQ ID n° 267 |
| | Do b 8 cr | tgcaaccagtctcctcttgg | SEQ ID n° 268 |
| | Do b 9 cr | accagtctcctcttgggtgg | SEQ ID n° 269 |
| | Do b 10 cr | cagtctcctcttgggtggt | SEQ ID n° 270 |
| Dombrock-Joseph(a) allèle positif : | | | |
|---|---|---|---|
| | Jo.a.pos.1 | ccccagaacatgactaccac | SEQ ID n° 271 |
| | Jo.a.pos.2 | ccagaacatgactaccacaca | SEQ ID n° 272 |
| | Jo.a.pos.3 | agaacatgactaccacacacgc | SEQ ID n° 273 |
| | Jo.a.pos.4 | catgactaccacacacgctgt | SEQ ID n° 274 |
| | Jo.a.pos.5 | actaccacacacgctgtgg | SEQ ID n° 275 |
| | Jo.a.pos.1.cr | gccacagcgtgtgtggtagt | SEQ ID n° 276 |
| | Jo.a.pos.2.cr | acagcgtgtgtggtagtcatg | SEQ ID n° 277 |
| | Jo.a.pos.3.cr | agcgtgtgtggtagtcatgtt | SEQ ID n° 278 |
| | Jo.a.pos.4.cr | cgtgtgtggtagtcatgttctg | SEQ ID n° 279 |
| | Jo.a.pos.5.cr | gtggtagtcatgttctgggg | SEQ ID n° 280 |
| Dombrock-Joseph(a) allèle négatif : | | | |
|---|---|---|---|
| | Jo.a.neg.1 | ctaccccagaacatgactgtcac | SEQ ID n° 281 |
| | Jo.a.neg.2 | cccagaacatgactatcacaca | SEQ ID n° 282 |
| | Jo.a.neg.3 | cagaacatgactatcacacacgc | SEQ ID n° 283 |
| | Jo.a.neg.4 | catgactatcacaeacgctgtg | SEQ ID n° 284 |
| | Jo.a.n3g.5 | actatcacacacgctgtggc | SEQ ID n° 285 |
| | Jo.a.n3g.1.cr | aatagccacagcgtgtgtgatagt | SEQ ID n° 286 |
| | Jo.a.neg.2.cr | cacagcgtgtgtgatagtcatg | SEQ ID n° 287 |
| | Jo.a.neg.3.cr | cagcgtgtgtgatagtcatgtt | SEQ ID n° 288 |
| | Jo.a.neg.4.cr | cgtgtgtgatagtcatgttctgg | SEQ ID n° 289 |
| | Jo.a.neg.5.cr | gtgatagtcatgttctggggtag | SEQ ID n° 290 |
| Duffy-allèle a : | | | |
|---|---|---|---|
| | Fy.A.1 | ccagatggagactatggtgcc | SEQ ID n° 291 |
| | Fy.A.2 | atggagactatggtgccaac | SEQ ID n° 292 |
| | Fy.A.3 | ggagactatggtgccaacctg | SEQ ID n° 293 |
| | Fy.A.4 | gactatggtgccaacctgga | SEQ ID n° 294 |
| | Fy.A.5 | tatggtgccaacctggaag | SEQ ID n° 295 |
| | Fy.A.1.cr | cttccaggttggoacaata | SEQ ID n° 296 |
| | Fy.A.2.cr | tccaggttggcaccatagtc | SEQ ID n° 297 |
| | Fy.A.3.cr | aggttggcaccatagtctcc | SEQ ID n° 298 |
| | Fy.A.4.cr | gttggcaccntagtctccat | SEQ ID n° 299 |
| | Fy.A.5.cr | gcaccatagtctccatctgg | SEQ ID n° 300 |
| Duffy-allèle b : | | | |
|---|---|---|---|
| | Fy.B.1 | cccagatggagactatgatgcc | SEQ ID n° 301 |
| | Fy.B.2 | gatggagactatgatgccaac | SEQ ID n° 302 |
| | Fy.B.3 | tggagactatgatgccaacctg | SEQ ID n° 303 |
| | Fy.B.4 | gactatgatgccaacctggaa | SEQ ID n° 304 |
| | Fy.B.5 | tatgatgccaacctggaagc | SEQ ID n° 305 |
| | Fy.B.1.cr | gcttccaggttggcatcata | SEQ ID n° 306 |
| | Fy.B.2.cr | ttccaggttggcatcatagtc | SEQ ID n° 307 |
| | Fy.B.3.cr | caggttggcatcatagtctcc | SEQ ID n° 308 |
| | Fy.B.4.cr | gttggcatcatagtctccatc | SEQ ID n° 309 |
| | Fy.B.5.cr | gcatcatagtctccatctggg | SEQ ID n° 310 |
| Duffy GATAbox-allèle normal : | | | |
|---|---|---|---|
| | Fy.GATA.normal.1 | agtccttggctcttatcttg | SEQ ID n° 311 |
| | Fy.GATA.normal.2 | agtccttggctcttatcttgga | SEQ ID n° 312 |
| | Fy.CATA.normal.3 | cttggctcttatcttggaagc | SEQ ID n° 313 |
| | Fy.GATA.normal.4 | gctcttatcttggaagcacagg | SEQ ID n° 314 |
| | Fy.GATA.normal.5 | cttatcttggaagcacaggcgc | SEQ ID n° 315 |
| | Fy.GATA.normal.1.cr | gcctgtgcttccaagataag | SEQ ID n° 316 |
| | Fy.GATA.normal.2.cr | tgtgcttccaagataagagc | SEQ ID n° 317 |
| | Fy.GATA.normal.3.cr | tgcttccaagataagagcca | SEQ ID n° 318 |
| | Fy.GATA.normal.4.cr | ttccaagataagagccaagga | SEQ ID n° 319 |
| | Fy.GATA.normal.5.cr | caagataagagccaaggact | SEQ ID n° 320 |
| Duffy GATAbOX-allèle muté : | | | |
|---|---|---|---|
| | Fy.GATA.mut.1 | tccttggctcttaccttg | SEQ ID n° 321 |
| | Fy.GATA.mut.2 | tccttggctcttaccttgga | SEQ ID n° 322 |
| | Fy.GATA.mut.3 | tgctcttaccttggaagc | SEQ ID n° 323 |
| | Fy.CATA.mut.4 | gctcttaccttggaagcacag | SEQ ID n° 324 |
| | Fy.GATA.mut.5 | cttaccttggaagcacaggcg | SEQ ID n° 325 |
| | Fy.GATA.mut.1.cr | cctgtgcttccaaggttaag | SEQ ID n° 326 |
| | Fy.GATA.mut.2.cr | gtgcttccaaggtaagagc | SEQ ID n° 327 |
| | Fy.GATA.mut.3.cr | gcttccaaggtaagagcca | SEQ ID n° 328 |
| | Fy.GATA.mut.4.cr | ttccaaggtaagagccaagg | SEQ ID n° 329 |
| | Fy.GATA.mut.5.cr | caaggtaagagccaagga | SEQ ID n° 330 |
| Duffy Fyx-allèle normal : | | | |
|---|---|---|---|
| | Fy.X.(b).1 | ttttaagacctctcttccgct | SEQ ID n° 331 |
| | Fy.X.(b).2 | agacctctcttccgctggc | SEQ ID n° 332 |
| | Fy.X.(b).3 | ctctcttccgstggcagc | SEQ ID n° 333 |
| | Py.X.(b).4 | ctcttccgctggcagctc | SEQ ID n° 334 |
| | Fy.X.(b).5 | cttccgctggcagctctg | SEQ ID n° 335 |
| | Fy.X.(b).1.cr | cagagctgccagcggaa | SEQ ID n° 336 |
| | Fy.X.(b).2.cr | agctgccagcggaagag | SEQ ID n° 337 |
| | Fy.X.(b).3.cr | ctgccagcggaagagagg | SEQ ID n° 338 |
| | Fy.X.(b).4.cr | gccagcggaagagaggtc | SEQ ID n° 339 |
| | Fy.X.(b).5.cr | cagcggaagagaggtctg | SEQ ID n° 340 |
| Duffy Fyx-allèle muté : | | | |
|---|---|---|---|
| | Fy.X.1 | gcttttcagacctctcttctgct | SEQ ID n° 341 |
| | Fy.X.2 | tcagecctstcttctgctggc | SEQ ID n° 342 |
| | Fy.X.3 | acctctcttcrgctggcagc | SEQ ID n° 343 |
| | Fy.X.4 | ctcttctgctggcagctctg | SEQ ID n° 344 |
| | Fy.X.5 | cttctgctggcagctctgc | SEQ ID n° 345 |
| | Fy.X.1.cr | gcagagctgccagcagaa | SEQ ID n° 346 |
| | Fy.X.2.cr | gagctgccagcagaagagag | SEQ ID n° 347 |
| | Fy.X.3.cr | agctgccagcagaagagagg | SEQ ID n° 348 |
| | Py.X.4.cr | gccagcagaagagaaggtctg | SEQ ID n° 349 |
| | Fy.X.5.cr | cagcagaagagaggtctgaaa | SEQ ID n° 350 |
| Kidd-allèle-a : | | | |
|---|---|---|---|
| | Jk.a.1 | cagccccatttgaggaca | SEQ ID n° 351 |
| | Jk.a.2 | gccccatttgaggacatcta | SEQ ID n° 352 |
| | Jk.a.3 | ccatttgaggacatctactttg | SEQ ID n° 353 |
| | Jk.a.4 | atttgaggacatctactttgga | SEQ ID n° 354 |
| | Jk.a.5 | gaggacatctactttggactct | SEQ ID n° 355 |
| | Jk.a.1.cr | cagagtccaaagtagatgtcctc | SEQ ID n° 356 |
| | Jk.a.2.cr | agtccaaagtagatgtcotcaaa | SEQ ID n° 357 |
| | Jk.a.3.cr | aaagtagatgtcctcaaatggg | SEQ ID n° 358 |
| | Jk.a.4.cr | tagatgtcctcaaatggggc | SEQ ID n° 359 |
| | Jk.a.5.cr | atgtcctcaaatggggctg | SEQ ID n° 360 |
| Kidd-allèle-b : | | | |
|---|---|---|---|
| | Jk.b.1 | tcagccccatttgagaaca | SEQ ID n° 361 |
| | Jk.b.2 | gccccatttgagaacatcta | SEQ ID n° 362 |
| | Jk.b.3 | cccatttgagaacatctactttg | SEQ ID n° 363 |
| | Jk.b.4 | atttgagaacatctactttggac | SEQ ID n° 364 |
| | Jk.b.5 | gagaacatctactttggactctg | SEQ ID n° 365 |
| | Jk.b.1.cr | ccagagtcoaaagtagatgttctc | SEQ ID n° 366 |
| | Jk.b.2.cr | agagtccaaagtagatgttctcaaa | SEQ ID n° 367 |
| | Jk.b.3.cr | ccaaagtagatgttctcaaatgg | SEQ ID n° 368 |
| | Jk.b.4.cr | agtagatgttctcaaatggggc | SEQ ID n° 369 |
| | Jk.b.5.cr | atgttctcaaatggggctga | SEQ ID n° 370 |
| Kell-allèle K1 : | | | |
|---|---|---|---|
| | KEL.1.1 | tcctaaactttaaccgaatgct | SEQ ID n° 371 |
| | KEL,1,2 | ttaaactttaaccgaatgctgaga | SEQ ID n° 372 |
| | KEL.1.3 | aactttaaccgaatgctgagactt | SEQ ID n° 373 |
| | KEL.1.4 | aaccgaatgctgagacttctg | SEQ ID n° 374 |
| | KEL.1.5 | cgaatgctgagacttctgatgag | SEQ ID n° 375 |
| | JEL 1.6 CR | actcatcgaagtctcagcattc | SEQ ID n° 376 |
| | KEL 1.7 CR | tcagaagtctcagcattcggt | SEQ ID n° 377 |
| | KEL 1.8 CR | aagtctcagcattcggttaaag | SEQ ID n° 378 |
| | KEL 1.9 CR | tctcagcattcggttaaagtttaa | SEQ ID n° 379 |
| | KEL 1.10 CR | agcattcggttaaagtttaagga | SEQ ID n° 380 |
| Kell-allèle K2 : | | | |
|---|---|---|---|
| | KEL.2.1 | ccttaaactttaaccgaacgct | SEQ ID n° 381 |
| | KEL.2.2 | aactttaaccgaacgctgaga | SEQ ID n° 382 |
| | KEL.2.3 | ctttaaccgaacgctgagactt | SEQ ID n° 383 |
| | KEL.2.4 | aaccgaacgctgagacttct | SEQ ID n° 384 |
| | KEL.2.5 | cgaacgctgagacttctgatg | SEQ ID n° 385 |
| | KEL 2.6 CR | tcatcagaagtctcagcgttc | SEQ ID n° 386 |
| | KEL 2.7 CR | cagaagtctcagcgttcggt | SEQ ID n° 387 |
| | KEL 2.8 CR | agtctcagcgttcggttaaag | SEQ ID n° 388 |
| | KEL 2.9 CR | tctcagcgttcggttaaagtt | SEQ ID n° 389 |
| | KEL 2.10 CR | agcgttcggttaaagtttaagg | SEQ ID n° 390 |
| Kell-allèle K3 : | | | |
|---|---|---|---|
| | KEL.3.1 | aatctccatcacttcatggct | SEQ ID n° 391 |
| | KEL.3.2 | tccatcacttcatggctgtt | SEQ ID n° 392 |
| | KEL.3.3 | atcacttcatggctgttcca | SEQ ID n° 393 |
| | KBL.3.4 | acttcatggctgttccagtt | SEQ ID n° 394 |
| | KEL.3.5 | tcatggctgttccagtttc | SEQ ID n° 395 |
| | KEL.3.cr | agaaactggaacagccatgaa | SEQ ID n° 396 |
| | KEL.3.2.cr | aactggaacagccatgaagtg | SEQ ID n° 397 |
| | KEL.3.3.cr | tggaacagccatgaagtgatg | SEQ ID n° 398 |
| | KEL,3.4.cr | acagccatgaagtgatggag | SEQ ID n° 399 |
| | KEL.3.5.cr | gccatgaagtgatggagatt | SEQ ID n° 400 |
| Kell-allèle K4 : | | | |
|---|---|---|---|
| | KEL.4.1 | tctccatcactcacggct | SEQ ID n° 401 |
| | KEL.4.2 | ccatcacttcacggctgtt | SEQ ID n° 402 |
| | KEL.4.3 | cacttcacggctgttcca | SEQ ID n° 403 |
| | KEL.4.4 | acttcacggctgttccag | SEQ ID n° 404 |
| | KEL.4.5 | tcacggotgttccagttt | SEQ ID n° 405 |
| | KEL.4.1.cr | aaactggaacagccgtgaa | SEQ ID n° 406 |
| | KEL.4.2,or | ctggaacagccgtgaagtg | SEQ ID n° 407 |
| | KEL.4.3.cr | ggaacagccgtgaagtgatg | SEQ ID n° 408 |
| | KEL.4.4.cr | acagccgtgaagtgatgg | SEQ ID n° 409 |
| | KEL.4.5.cr | gccgtgaatggatggaga | SEQ ID n° 410 |
| Kell-allèle K6 : | | | |
|---|---|---|---|
| | KEL.6.1 | tactgcctgggggctgccccgcc | SEQ ID n° 411 |
| | KEL.6.2 | tgggggctgcccgcctgt | SEQ ID n° 412 |
| | KEL.6.3 | gctgccccgcctgtgac | SEQ ID n° 413 |
| | KEL.6.4 | ctgocccgcctgtgacaa | SEQ ID n° 414 |
| | KEL.6.5 | gccccgcctgtgaaac | SEQ ID n° 415 |
| | KEL.6.1.cr | gttgtcacaggcggggc | SEQ ID n° 416 |
| | KEL.6.2.cr | ttgtcacaggcggggcag | SEQ ID n° 417 |
| | KEL.6.3.cr | tcacaggcggggcagccc | SEQ ID n° 418 |
| | KEL.6.4.cr | acaggcggggcagccccca | SEQ ID n° 419 |
| | KEL.6.5.cr | aggcggggcagccccca | SEQ ID n° 420 |
| Kell-allèle K7 : | | | |
|---|---|---|---|
| | KEL.7.1 | actgcctgggggctgcctcgcc | SEQ ID n° 421 |
| | KEL.7.2 | cctgggggctgcctcgcctgt | SEQ ID n° 422 |
| | KEL.7.3 | ggctgcctcgcctgtgac | SEQ ID n° 423 |
| | KEL.7,4 | ctgcctogcttgtgacaacc | SEQ ID n° 424 |
| | KEL.7.5 | gcctcgcctgtgacaacc | SEQ ID n° 425 |
| | KEL.7.1.cr | ggttgtcacaggcgaggc | SEQ ID n° 426 |
| | KEL.7.2.cr | ggttgtcacaggcgaggcag | SEQ ID n° 427 |
| | KEL.7.3.cr | ttgtcacaggcgaggcagccc | SEQ ID n° 428 |
| | KEL.7.4.cr | acaggcgaggcagcccccagg | SEQ ID n° 429 |
| | KEL.7.5.cr | aggcgaggcagcccccagg | SEQ ID n° 430 |
| Luthéran allèle a : | | | |
|---|---|---|---|
| | Lu.a.1 | ggagctcgoaacgcct | SEQ ID n° 431 |
| | Lu.a.2 | gctcgcccccgcctagc | SEQ ID n° 432 |
| | Lu.a.3 | cgcccccgcctagcctc | SEQ ID n° 433 |
| | Lu.a.4 | cccccgcctagcctcgg | SEQ ID n° 434 |
| | Lu.a.5 | cccgcctagcctcggct | SEQ ID n° 435 |
| | Lu.a.1 CR | agccgaggctaggcggg | SEQ ID n° 436 |
| | Lu.a.2 CR | ccgaggctaggcggggg | SEQ ID n° 437 |
| | Lu.a.3 CR | gaggctaggcgggggcg | SEQ ID n° 438 |
| | Lu.a.4 CR | gctaggcgggggcgagc | SEQ ID n° 439 |
| | Lu.a.5 CR | aggcgggggcgagctcc | SEQ ID n° 440 |
| Luthéran allèle b : | | | |
|---|---|---|---|
| | Lu.b.1 | gggagctcgcccccacct | SEQ ID n° 441 |
| | Lu.b.2 | gagctcgcccccacctagc | SEQ ID n° 442 |
| | Lu.b.3 | ctcgcccccacctagcctc | SEQ ID n° 443 |
| | Lu.b.4 | cccccacctagcctcggct | SEQ ID n° 444 |
| | Lu.b.5 | cccacctagoctcggctga | SEQ ID n° 445 |
| | Lu.b,1 CR | tcagccgaggctaggtggg | SEQ ID n° 446 |
| | Lu.b.2 CR | agccgaggctaggtggggg | SEQ ID n° 447 |
| | Lu.b.3 CR | gaggctaggtgggggcgag | SEQ ID n° 448 |
| | Lu.b.4 CR | gctaggtgggggcgagctc | SEQ ID n° 449 |
| | Lu.b.5 CR | aggtgggggcgagctccc | SEQ ID n° 450 |
| MNS allèle M : | | | |
|---|---|---|---|
| | M.1 | gtgagcatatcagcatcaag | SEQ ID n° 451 |
| | M.2 | atcagcatcaagtaccactgg | SEQ ID n° 452 |
| | M.3 | cataagtaccactggtgtg | SEQ ID n° 453 |
| | M.4 | taccactggtgtggcaatgc | SEQ ID n° 454 |
| | M.5 | ctggtgtggcaatgcaca | SEQ ID n° 455 |
| | M.1.cr | tgtgcattgccacaccagt | SEQ ID n° 456 |
| | M.2.cr | gcattgccacaccagtggta | SEQ ID n° 457 |
| | M.3.cr | ccacaccagtggtacttgatg | SEQ ID n° 458 |
| | M.4.cr | accagtggtacttgatgct | SEQ ID n° 459 |
| | M.5.cr | cttgatgctgatatgctcac | SEQ ID n° 460 |
| MNS allèle N : | | | |
|---|---|---|---|
| | N.1 | tgtgagcatatcagcattaag | SEQ ID n° 461 |
| | N.2 | atcagcattaagtaccactgagg | SEQ ID n° 462 |
| | N.3 | cattaagtaccactgaggtgg | SEQ ID n° 463 |
| | N.4 | acoactgaggtggcaatgc | SEQ ID n° 464 |
| | N.5 | ctgaggtggcaatgcacact | SEQ ID n° 465 |
| | N.1.cr | gtgtcattgccacctcagt | SEQ ID n° 466 |
| | N.2.cr | goattgccacctcagtggta | SEQ ID n° 467 |
| | N.3.cr | ccacctcagtggtactaatgc | SEQ ID n° 468 |
| | N.4.cr | cctcagtggtacttaatgct | SEQ ID n° 469 |
| | N.5.cr | cttaatgctgatatgctcaca | SEQ ID n° 470 |
| MNS allèle S : | | | |
|---|---|---|---|
| | big.5.1 | tttgctttataggagaaatgga | SEQ ID n° 471 |
| | big.S.2 | ctttataggagaaatgggaca | SEQ ID n° 472 |
| | big.S.3 | ttataggagaaatgggacaacttg | SEQ ID n° 473 |
| | Mg.S.4 | gagaaatgggacaacttgtcc | SEQ ID n° 474 |
| | big.S.5 | aaatgggacaacttgtccatc | SEQ ID n° 475 |
| | big.S.1.cr | gatggacaagttgtccoattt | SEQ ID n° 476 |
| | big.S.2.cr | gacaagttgtçcoatttotcc | SEQ ID n° 477 |
| | big.S.3.cr | aagttgtcccatttctcotata | SEQ ID n° 478 |
| | big S.4.cr | tgtcccatttctcctataaagca | SEQ ID n° 479 |
| | big.S.5.cr | cccatttctcctataaagcaaaa | SEQ ID n° 480 |
| MNS allèle s : | | | |
|---|---|---|---|
| | little.s.1 | tgctttataggagaaacggga | SEQ ID n° 481 |
| | little.s.2 | tttataggagaaacgggaca | SEQ ID n° 482 |
| | little.s.3 | ggagaaacgggacaacttg | SEQ ID n° 483 |
| | little.s.4 | gagaaacgggacaacttgtc | SEQ ID n° 484 |
| | little.s.5 | aaacgggacaacttgtccat | SEQ ID n° 485 |
| | little.s.1.or | tggacaagttgtoccgttt | SEQ ID n° 486 |
| | little.s.2.cr | acaagttgtcccgtttctcc | SEQ ID n° 487 |
| | little.s.3.cr | agttgtcccgtttctcctata | SEQ ID n° 488 |
| | little.s.4.cr | tgtcccgtttotcctataaagc | SEQ ID n° 489 |
| | little.s.5.cr | cccgtttctcctataaagca | SEQ ID n° 490 |
| MNS allèle positif U : | | | |
|---|---|---|---|
| | U-pos.1 | ttgctgctctctttagctcc | SEQ ID n° 491 |
| | U.pos.2 | ctctctttagctcctgtagtgat | SEQ ID n° 492 |
| | U.pos.3 | agctcctgtagtgataatactca | SEQ IDn° 493 |
| | U.pos.4 | gtagtgataatactcattatttttg | SEQ ID n° 494 |
| | U.pos.5 | taatactcattatttttggggtg | SEQ ID n° 495 |
| | U.poa.1.cr | caccccaaaaataatgagtatta | SEQ ID n° 496 |
| | U.pos.2.cr | caaaaataatgagtaatcactaca | SEQ ID n° 497 |
| | U.pos.3.cr | agtattatcatacaggagctaaa | SEQ ID n° 498 |
| | U.pos.4.cr | atcactacaggagctaaagag | SEQ ID n° 499 |
| | U.pos.5.cr | gagctaaagagagcagcaaa | SEQ ID n° 500 |
| MNS allèle négatif U : | | | |
|---|---|---|---|
| | U.neg.1 | ttttgctgctctctttatctcc | SEQ ID n° 501 |
| | U.neg.2 | gctctctttatctcctgtagagat | SEQ ID n° 502 |
| | U.neg.3 | tatctcctgtagagataacactca | SEQ ID n° 503 |
| | U.neg.4 | gatagagataacactcattattttt | SEQ ID n° 504 |
| | U.neg.5 | taacactcattatttttggggt | SEQ ID n° 505 |
| | U.neg.1.cr | accccaaaaataatgagtgtta | SEQ ID n° 506 |
| | U.neg.2.cr | aaaaataatgagtgttatctctaca | SEQ ID n° 507 |
| | U.neg.3.cr | agtgttatctctacaggagataaa | SEQ ID n° 508 |
| | U.neg.4.cr | atctctacaggagataaagagag | SEQ ID n° 509 |
| | U.neg.5.cr | gagataaagagagcagcaaaatta | SEQ ID n° 510 |
| Rhésus allèle C (307T) : | | | |
|---|---|---|---|
| | Rh.big.C.1 | tgagccagttcccttctgg | SEQ ID n° 511 |
| | Rh.big.C.2 | gagccagttcccttctgg | SEQ ID n° 512 |
| | Rh.big.C.3 | ctgagccagttcccttctg | SEQ ID n° 513 |
| | Rh.big.C.4 | ccttctgggaaggtggtc | SEQ ID n° 514 |
| | Rh.big.C.5 | ccttctgggaaggtggtca | SEQ ID n° 515 |
| | Rh.big.C.1.cr | tgaccaccttcccagaagg | SEQ ID n° 516 |
| | Rh.big.C.2.cr | gaccaccttcccagaagg | SEQ ID n° 517 |
| | Rh.big.C.3.cr | ccagaagggaactggctc | SEQ ID n° 518 |
| | Rh.big.C.4.cr | ccagaagggaactggctca | SEQ ID n° 519 |
| | Rh.big.C.5.cr | cagaagggaactggctcag | SEQ ID n° 520 |
| Rhésus allèle c (307C) : | | | |
|---|---|---|---|
| | Rh.little.c.1 | gagccagttccctcctgg | SEQ ID n° 521 |
| | Rh.little.c.2 | agccagttccctcctgg | SEQ ID n° 522 |
| | Rh.lttle.c.3 | tgagccagttccctcctg | SEQ ID n° 523 |
| | Rh.little.c.4 | cctcctgggaaggtggt | SEQ ID n° 524 |
| | Rh.littie.c.5 | cctcctgggaaggtggtc | SEQ ID n° 525 |
| | Rh.little.c.1.cr | gaccaccttcccaggagg | SEQ ID n° 526 |
| | Rh.little.c.2.cr | accacctcccaggagg | SEQ ID n° 527 |
| | Rh.little.c.3.cr | ccaggagggaactggct | SEQ ID n° 528 |
| | Rh.little.c.4.cr | ccaggagggaactggctc | SEQ ID n° 529 |
| | Rh.little.c.5.cr | caggagggaactggctca | SEQ ID n° 530 |
| Rhésus BigC-intron2-allèle d'insertion spécifique : | | | |
|---|---|---|---|
| | RhC.intron.2.1 | agggtgccctttgtcacttc | SEQ ID n° 531 |
| | RhC.intron.2.2 | gccctttgtcacttcccagt | SEQ ID n° 532 |
| | RhC,intron.2.4 | cctttgtcacttccoagtgg | SEQ ID n° 533 |
| | RhC,intron.2.4 | ttgtcaotocaagtggtcaa | SEQ ID n° 534 |
| | RhC.intron.2.5 | tcacttcccagtggtacaatca | SEQ ID n° 535 |
| | RhC.intron.2.1.cr | gaagtgacaaagggcaccct | SEQ ID n° 536 |
| | RhC.intron.2.2.cr | actgggaagtgacaaagggc | SEQ ID n° 537 |
| | RhC.intron.2.3.cr | ccactgggaagtgacaaagg | SEQ ID n° 538 |
| | RhC.intron.2.4.cr | tgtaccactgggaagtgacaaa | SEQ ID n° 539 |
| | RhC.intron.2.5.cr | tgattgtaccactgggaagtga | SEQ ID n° 540 |
| Rhésus allèle E : | | | |
|---|---|---|---|
| | Rh.big.E.1 | gccaagtgtcaactctcctct | SEQ ID n° 541 |
| | Rh.big.E.2 | caagtgtcaactctcctgct | SEQ ID n° 542 |
| | Rh.big.E.3 | gtgtcaactctcctctgctgag | SEQ ID n° 543 |
| | Rh.big.E.4 | caactctcctctgctgagaagtc | SEQ ID n° 544 |
| | Rh.big.E.5 | tctcctctgctgagaatcc | SEQ ID n° 545 |
| | Rh.big.E.1.cr | ggacttctcagcagaggagag | SEQ ID n° 546 |
| | Rh.big.E.2.cr | cttctcagcagaggagattga | SEQ ID n° 547 |
| | Rh.big.E.3.cr | ctcagcagaggagagttgacac | SEQ ID n° 548 |
| | Rh.big.E.4.cr | gcagaggagagttgacacttg | SEQ ID n° 549 |
| | Rh.big.E.5.cr | gaggagagttgaeaottggc | SEQ ID n° 550 |
| Rhésus allèle e : | | | |
|---|---|---|---|
| | Rh.little.e.1 | gccaagtgtcaactctgctct | SEQ ID n° 551 |
| | Rh.little.e.2 | caagtgtcaactctgctctgct | SEQ ID n° 552 |
| | Rh.little.e.3 | tgtcaactctgctctgctgag | SEQ ID n° 553 |
| | Rh.littte.e.4 | caactctgctctgctgagaag | SEQ ID n° 554 |
| | Rh.little.e.5 | tctgctctgctgagaagtcc | SEQ ID n° 555 |
| | Rh,little.e.1.cr | ggacttctcagcagagcagag | SEQ ID n° 556 |
| | Rh.little.e.2.cr | ttctcagcagagcagagttga | SEQ ID n° 557 |
| | Rh.little.e.3.cr | tcagcagagcagagttgacac | SEQ ID n° 538 |
| | Rh.little.e.4.cr | gcagagcagagttgacacttg | SEQ ID n° 559 |
| | Rh.little.e.5.cr | gagcagagttgacacttggc | SEQ ID n° 560 |
| Rhésus allèle RHD : | | | |
|---|---|---|---|
| | RhD 1 | ttccccacagctccatcat | SEQ ID n° 561 |
| | RhD 2 | acagctccatcatgggctac | SEQ ID n° 562 |
| | RbD 3 | tccatcatgggctacaacttc | SEQ ID n° 563 |
| | RhD 4 | tcatgggctacaacttcagct | SEQ ID n° 564 |
| | RMD 5 | gggctacaacttcagcttgct | SEQ ID n° 565 |
| | RhD cr 1 | agcaagctgaagttgtagccc | SEQ ID n° 566 |
| | RhD cr 2 | agctgaagttgtascccatga | SEQ ID n° 567 |
| | RhD cr 3 | gaagttgtagcccatgatgg | SEQ ID n° 568 |
| | RhD cr 4 | gcccatgatggagctgt | SEQ ID n° 569 |
| | RhD cr 5 | tgatggagctgctgtggggaa | SEQ ID n° 570 |
| Rhésus allèle r's : | | | |
|---|---|---|---|
| | r's.T.1 | ggaaggtcaacttggtgca | SEQ ID n° 571 |
| | r's.T.2 | ggaaggtcaacttggtgcagt | SEQ ID n° 572 |
| | r's.T.3 | caacttggtgcagttggtg | SEQ ID n° 573 |
| | r's.T.4 | acttggtgcagttggtgt | SEQ ID n° 574 |
| | r's.T.5 | ttggtgcagttggtggtgat | SEQ ID n° 575 |
| | r's.T.1.cr | catcaccaccaactgcacca | SEQ ID n° 576 |
| | r's.T.2.cr | caccaccaactgcaccaagt | SEQ ID n° 577 |
| | r's,T.3.cr | accaactgcaccaagttgac | SEQ ID n° 578 |
| | r's.T.4.cr | actgcaccaagttgaccttcc | SEQ ID n° 579 |
| | r's:T.5.cr | tgcaccaagttgaccttcc | SEQ ID n° 580 |
| Rhésus allèle DVI : | | | |
|---|---|---|---|
| | Rh.DVI.1 | attccattccctcttggcctt | SEQ ID n° 581 |
| | Rh.DVI.2 | aaccctcttggcctttgttt | SEQ ID n° 582 |
| | Rh.DVI.3 | tcttggcctttgtttccttg | SEQ ID n° 583 |
| | Rh.DVI.4 | ggtatcagcttgagagctg | SEQ ID n° 584 |
| | Rh.DVI.5 | atcagcttgagagctcggag | SEQ ID n° 585 |
| | Rh.DVI.1.cr | aaggccaagagggttgaaat | SEQ ID n° 586 |
| | Rh.DVI.2.cr | aaacaaaggccaagagggtt | SEQ ID n° 587 |
| | Rh.DVI.3.cr | caaggaaacaaaggccaaga | SEQ ID n° 588 |
| | Rh.DVI.4.cr | cgagctctcaagctgatacc | SEQ ID n° 589 |
| | Rh.DVI.5.cr | ctccgagctctcaagctgat | SEQ ID n° 590 |
| Rhésus allèle RHD-pseudogène muté : | | | |
|---|---|---|---|
| | RHD Y 1 | tttctttgcagacttaggtgc | SEQ ID n° 591 |
| | RhD Y 2 | ctttgcagacttaggtgcaca | SEQ ID n° 592 |
| | RhD Y 3 | tttgcagacttaggtgcacagt | SEQ ID n° 593 |
| | RhD Y 4 | acttaggtgcacagtgcgg | SEQ ID n° 594 |
| | RhD Y 5 | cttaggtgcacagtgcggt | SEQ ID n° 595 |
| | RhD Y cr 1 | caccgcactgtgcacctaa | SEQ ID n° 596 |
| | RhD Y cr 2 | ccgcactgtgcacctaagtc | SEQ ID n° 597 |
| | RhD Y cr 3 | gcactgtgcacctaagtctgc | SEQ ID n° 598 |
| | RhD Y cr 4 | tgcacctaagtctgcaaaga | SEQ ID n° 599 |
| | RhD Y cr 5 | cacctaagtctgcaaagaaatagcg | SEQ ID n° 600 |
| Rhésus allèle RHD-pseudogène normal : | | | |
|---|---|---|---|
| | RhD non Y 1 | ctatttctttgcagacttagttgc | SEQ ID n° 601 |
| | RhD non Y 2 | tctttgcagacttatgtgcaca | SEQ ID n° 602 |
| | RhD non Y 3 | ttgcagacttatgtgcacagtg | SEQ ID n° 603 |
| | RhD non Y 4 | acttatgtgcacagtgcggt | SEQ ID n° 604 |
| | RhD non Y 5 | cttatgtgcacagtgcggtg | SEQ ID n° 605 |
| | RhD non Y cr 1 | acaccgcactgtgcacataa | SEQ ID n° 606 |
| | RhD non Y cr 2 | accgcactgtgcacataagtc | SEQ ID n° 607 |
| | RhD non Y cr 3 | gcactgtgcacataagtctgc | SEQ ID n° 608 |
| | RhD non Y cr 4 | tgtgcacataagtctgcaaag | SEQ ID n° 609 |
| | RhD non Y cr 5 | cacataagtctgcaaagaaatgacg | SEQ ID n° 610 |
| Yt-allèle a : | | | |
|---|---|---|---|
| | Yt.a.1 | gcgggagacttccacgg | SEQ ID n° 611 |
| | Yt.a.2 | gggagacttccacggcct | SEQ ID n° 612 |
| | Yt.a.3 | gagacttccacggcctgc | SEQ ID n° 613 |
| | Yt.a.4 | gacttccacggcctgca | SEQ ID n° 614 |
| | Yt.a.5 | ttccacggcctgcaggta | SEQ ID n° 615 |
| | Yt.a.1 CR | tacctgcaggccgtggaa | SEQ ID n° 616 |
| | Yt.a.2 CR | tgcaggccgtggaagtc | SEQ ID n° 617 |
| | Yt.a.3 CR | gcaggcctggaagtctc | SEQ ID n° 618 |
| | Yt.a.4 CR | aggccgtggaagtctccc | SEQ ID n° 619 |
| | Yt.a.5 CR | ccgtggaagtctcccgc | SEQ ID n° 620 |
| Yt-allèle b : | | | |
|---|---|---|---|
| | Yt.b:1 | gcgggagacttcaacggc | SEQ ID n° 621 |
| | Yt.b.2 | gggagacttcaacggcctg | SEQ ID n° 622 |
| | Yt.b.3 | gagacttcaacggcctgca | SEQ ID n° 623 |
| | Yt.b.4 | gacttcaacggcctgcag | SEQ ID n° 624 |
| | Yt.b.5 | ttcaacggcctgcaggtaa | SEQ ID n° 625 |
| | Yt.b.1 CR | ttacctgcaggccgttgaa | SEQ ID n° 626 |
| | Yt.b.2 CR | ctgcaggccgttgaagtc | SEQ ID n° 627 |
| | Yt.b.3 CR | tgcaggccgttgaagtctc | SEQ ID n° 628 |
| | Yt.b.4 CR | caggccgttgaagtctccc | SEQ ID n° 629 |
| | Yt.b.5 CR | gccgttgaagtctcccgc | SEQ ID n° 630 |

14. Procédé selon l'une quelconque des revendications 10 à 13, dans lequel les sondes oligonucléotidiques comprennent au niveau de leur extrémité 5' un lieur, de préférence un résidu -(CH₂)₆- et un groupement réactionnel, de préférence un groupement amino, pour fixation sur un support de réseau, de préférence une lame de verre revêtue de poly-L-lysine.

15. Procédé selon l'une quelconque des revendications 10 à 14, dans lequel le réseau comprend un ou plusieurs oligonucléotides présentant une séquence n'apparaissant pas dans l'ADN de ladite espèce mammifère pour pouvoir soustraire le fond, de préférence choisis dans le groupe comprenant les séquences suivantes :
| | | |
|---|---|---|
| a3 | cagaccataagcacaggcgt | SEQ ID n° 631 |
| a9 | gctcgtccacagtgcgttat | SEQ ID n° 632 |
| a17 | cggcgttcaagcaaaccgaa | SEQ ID n° 633 |
| a23 | gacatatagctccactctcaga | SEQ ID n° 634 |
| a27 | tagggtactgatgagcactc | SEQ ID n° 635 |
| a33 | tcagccctatcgcaggatgt | SEQ ID n° 169 |
| a35 | gagacacttgacagtagcca | SEQ ID n° 170 |
| a38 | ggcagggcacctcagtttat | SEQ ID n° 171 |
| a42 | tcaccagccagactgtgtag | SEQ ID n° 172 |
| a43 | cttcacgcaagttgtccaga | SEQ ID n° 173 |

16. Procédé selon l'une quelconque des revendications 10 à 15, dans lequel le réseau comprend un ou plusieurs témoins d'hybridation positifs, de préférence la séquence suivante :
| | | |
|---|---|---|
| CS05 | gtcctgacttctagctcatg | SEQ ID n° 174 |
et sa séquence complémentaire marquée de manière détectable est ajoutée aux produits issus de l'amplification par PCR multiplexe.

17. Procédé selon l'une quelconque des revendications 10 à 16, dans lequel on soumet les sondes, avant d'ajouter les produits issus de l'amplification par PCR multiplexe, à des traitements de pré-hybridation et de dénaturation de l'ADN.

18. Procédé selon l'une quelconque des revendications 10 à 17, dans lequel les produits dénaturés issus de l'amplification par PCR multiplexe sont appliqués au réseau d'ADN sans pré-purification.

19. Procédé selon l'une quelconque des revendications 10 à 18, dans lequel, pour chaque antigène de cellule sanguine, on utilise le rapport des intensités de signal pour chacun des allèles afin d'attribuer le génotype.

20. Trousse pour le génotypage d'antigènes de cellules sanguines selon un procédé tel que revendiqué dans l'une quelconque des revendications 1 à 19, comprenant une paire d'amorces chimériques spécifiques aux antigènes de cellules sanguines pour chaque antigène de cellule sanguine à génotyper et au moins une amorce universelle marquée de manière détectable, de préférence une paire d'amorces universelles marquées de manière détectable, telles que définies dans l'une quelconque des revendications 1 à 7.

21. Trousse selon la revendication 20, comprenant en outre un réseau d'ADN, tel que défini dans l'une quelconque des revendications 10 à 15.

22. Jeu de paires d'amorces chimériques spécifiques aux antigènes de cellules sanguines utile dans une PCR multiplexe, comprenant au moins deux, de préférence sensiblement toutes les paires d'amorces sélectionnées dans le groupe tel que défini dans la revendication 7.

23. Jeu de sondes oligonucléotidiques spécifiques aux allèles d'antigènes de cellules sanguines utile pour le génotypage d'antigènes de cellules sanguines, comprenant au moins 72 sondes différentes, de préférence sensiblement toutes les sondes sélectionnées dans le groupe tel que défini dans la revendication 13.
